# EUROPÄISCHE PATENTSCHRIFT

(11) **EP 2 513 071 B1**
(45) Veröffentlichungstag und Bekanntmachung des Hinweises auf die Patenterteilung: **17.06.2015**
(21) Anmeldenummer: 10779706.0
(22) Anmeldetag: 09.11.2010
(51) Int. Cl.: C07D 277/48, C07D 417/04, C07D 513/04, C07D 519/00, A61K 31/425, A61K 31/429, A61P 35/00

(54) **THIAZOLDERIVATE ZUR BEHANDLUNG VON KRANKHEITEN WIE KREBS**
THIAZOLEDERIVATES FOR THE TREATMENT OF DISEASES SUCH AS CANCER
DERIVES DE THIAZOLE POUR LE TRAITEMENT DES MALADIES COMME LE CANCER

(30) Priorität: 14.12.2009 DE 102009058280
(43) Veröffentlichungstag der Anmeldung: 24.10.2012
(73) Patentinhaber: Merck Patent GmbH, 64293 Darmstadt (DE)
(72) Erfinder: HEINRICH, Timo, 64823 Gross-Umstadt (DE); KOOLMAN, Hannes, 64285 Darmstadt (DE)
(86) Internationale Anmeldenummer: PCT/EP2010/006822
(87) Internationale Veröffentlichungsnummer: WO 2011/072779

(56) Entgegenhaltungen:
- EP-A1- 0 321 115
- EP-A2- 0 097 323
- WO-A1-2004/041813
- WO-A1-2005/077324
- WO-A1-2006/119146
- WO-A1-2010/079443
- WO-A2-03/027085
- WO-A2-2009/011880
- WO-A2-2010/003133
- US-A1- 2002 049 215
- US-A1- 2006 003 968
- ADEL M. ET AL: "Substituent effect on the amino-imino tautomerism of aminothiazoles", JOURNAL OF MOLECULAR STRUCTURE: THEOCHEM, Bd. 849, 12. Oktober 2007 (2007-10-12), Seiten 52-61, XP002614787,
- DIENER H ET AL: "MECHANISM OF AZO COUPLING REACTIONS.PART 34.REACTIVITY OF FIVE MEMBERED RING HETEROAROMATIC DIAZONIUM IONS", CAN.J.CHEM, Bd. 64, Nr. 6, 1. Januar 1986 (1986-01-01) , Seiten 1102-1107, XP002614788,
- SHULKRI J ET AL: "NEW SULFATHIAZOLES", WISSENSCHAFTLICHE ZEITSCHRIFT.MARTIN-LUTHER UNIVERSITÄT HALLE-WITTENBERG,MATHEMATISCH.NATURWISSENS CHAFTLICHE REIHE, Bd. 33, Nr. 3, 1. Januar 1984 (1984-01-01) , Seiten 91-93, XP002614789,
- BUOLAMWINI J K: "CELL CYCLE MOLECULAR TARGETS IN NOVEL ANTICANCER DRUG DISCOVERY", CURRENT PHARMACEUTICAL DESIGN, BENTHAM SCIENCE PUBLISHERS, NL, Bd. 6, Nr. 4, 1. Januar 2000 (2000-01-01), Seiten 379-392, XP009014400, ISSN: 1381-6128, DOI: DOI:10.2174/1381612003400948 in der Anmeldung erwähnt

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap. 2000; 88: 229 - 279).

Die vorliegende Erfindung betrifft Verbindungen und die Verwendung von Verbindungen, bei denen die Hemmung, Regulierung und/oder Modulation der Signaltransduktion von Proteinkinasen, insbesondere der Tyrosinkinasen und/oder Serin/Threonin-Kinasen eine Rolle spielt, ferner pharmazeutische Zusammensetzungen, die diese Verbindungen enthalten, sowie die Verwendung der Verbindungen zur Behandlung kinasebedingter Krankheiten. Die vorliegende Erfindung betrifft insbesondere Verbindungen und die Verwendung von Verbindungen, bei denen die Hemmung, Regulierung und/oder Modulation der Signaltransduktion von Kinasen eine Rolle spielt, insbesondere der Proteinkinasen NUAK1, auch ARK5 genannt, MAPK (MAP2K1, MAP4K2, MAP3K11), MARK3, ROCK2, auch Rho-Kinase 2 genannt, CHEK1, auch CHK1 genannt, CDK2, PKN2, KDR, auch VEGFR genannt, HIPK1 und AURORA.

### MAPxKy (mitogen-activated protein (MAP) Kinase

Das Protein, das durch dieses Gen kodiert wird, ist ein Mitglied der dual spezifischen Proteinkinase Familie, die als eine Mitogen-aktivierte Protein (MAP) Kinase fungiert. MAP Kinasen, auch als extrazelluläre signalregulierte Kinasen (ERK) beschrieben, fungieren als Integrationspunkt für unterschiedliche biochemische Signale. MAP2K1 liegt in der Signalkaskade vor anderen MAP Kinasen und stimuliert deren enzymatische Aktivität in Abhängigkeit von vielen extra- und intrazellulären Signalen. Als eine essentielle Komponente des MAP Kinase -Signaltransduktionsweges ist MAP2K1 in viele zelluläre Pozesse wie Proliferation, Differenzierung, Transkriptionsregulation und Zellentwicklung involviert.

Ähnliche Betrachtungen gelten für die anderen MAPKs.

Die Verbindung RO-4927350, ein Thiazolderivat, ist von K. Kolinsky et al. als MEK-Inhibitor zur spezifischen Inhibierung der MAPK-Signaltransduktionskaskade in Cancer Res. 2009; 69: 1924 ff. beschrieben, wobei die Verbindung in vivo eine Antitumor-Aktivität aufweist.

T. Kato et al. beschreiben in Neoplasia 2001; 3 (1): 4-9 MARK3 (homolog zu MARKL1) als potentielles Target zur Behandlung hepatozellulärer Carcinogenese.

### CHEK1

Studien belegen, das CHEK1 Inhibierung die Cytotoxicität von DNA schädigenden Agenzien verstärkt (Xiao Z, Chen Z, Gunasekera AH, et al. Chk1 mediates S and G2 arrests through Cdc25A degradation in response to DNA-damaging agents, J. Biol. Chem. 2003; 278: 21767 - 73;
Xiao D, Herman-Antosiewicz A, Antosiewicz J, et al. Diallyl trisulfideinduced G(2)-M phase cell cycle arrest in human prostate cancer cells is caused by reactive oxygen species-dependent destruction and hyperphosphorylation of Cdc 25 C, Oncogene 2005; 24: 6256 - 68;
Zhao B, Bower MJ, McDevitt PJ, et al. Structural basis for Chk1 inhibition by UCN-01, J. Biol. Chem. 2002; 277: 46609 - 15;
Maude SL, Enders GH. Cdk inhibition in human cells compromises chk1 function and activates a DNA damage response. Cancer Res. 2005; 65: 780 - 6.)
bzw. die Expression von CHEK1 gehört zum Verteidigungsmechanismus der Zelle, um die Toxizität von DNA Schäden zum umgehen.

### ROCK2

ROCK2 ist eine Serin/Threonin Kinase, die durch die Assoziation mit RhoGTP aktiviert wird, was zu einer Phosphorilierung einer Vielzahl von Substraten führt und schließlich die Stabilisation des filamentösen Actins und eine Erhöhung der Aktivität der Myosin ATPase zur Folge hat. Das wiederum bedingt die Bildung kontraktiler Actin-Myosin Einheiten (stressfibres) und Integrin beinhaltender fokaler Adhesionen. Durch die Modulation der Actin-Myosin Kontraktilität hat ROCK2 einen signifikanten Einfluß auf die Regulation der Zellmophologie, Zellmobilität und Zelladhesion. C. Chak-Lui Wong et al. beschreiben in Hepatology 2009; 49 (5): 1583 - 94, dass die Inhibierung der Rho-Kinasen 1 und 2 (ROCK1 und ROCK2) zur Behandlung von Krebserkrankungen genutzt werden kann. So spielt ROCK2 eine signifikante Rolle beim Wachstum von hepatozellulären Carcinomen.

X.Q. Wang et al. beschreiben in Radiation Res. 2007; 168 (6): 706 - 15 die Checkpointkinase CHK1 als potentielles Target zur Behandlung von Krebs.

### NUAK1

Das Nuak1 Gen kodiert für die "NUAK family SNF1-like kinase 1". NUAK1 interagiert mit USPX9 und Ubiquitin C.

Die Rolle von NUAK1 (ARK5) als Wachstums- oder Nahrungsfaktor von Tumorzellen ist von A. Suzuki et al. in J. Biol. Chem. 2003; 278 (1): 48 - 53 beschrieben, sowie in Oncogene 2003; 22: 6177 - 82.

Die Inhibierung von NUAK1 (ARK5) stellt somit eine potentielle Möglichkeit zur Krebsbekämpfung

### CDK2

Die Inaktivierung von CDK2 verhindert die Phosphorylierung des Proteins RB1. Somit kann die Zelle die G1-Phase des Zellzyklus nicht verlassen was zum Stopp jeglicher Zellteilungen führt.

J.K.Buolamwini beschreibt in Current Pharmaceutical Design 2009; 6, (4): 379 - 92 das therapeutische Potential von CDK (cyclin dependent kinase) - Inhibitoren bei der Krebsbekämpfung.

A. Schmidt et al. beschreiben in EMBO J. 2007; 26: 1624 - 36 PRK2/PKN2 als potentielles Target zur Behandlung von Tumorerkrankungen.

N. Ferrara beschreibt in Endocrine Rev. 2004; 25 (4): 581 - 611 die Verwendung VEGF-Inhibitoren zur Krebsbekämpfung.

VEGF und KDR stellen ein Ligand-Rezeptor-Paar dar, das eine wesentliche Rolle bei der Proliferation der Gefäßendothelzellen und der Bildung und Sprossung der Blutgefäße, die als Vaskulogenese bzw. Angiogenese bezeichnet werden, spielt.

Die Angiogenese ist durch eine übermäßig starke Aktivität des Gefäßendothelwachstumsfaktors (VEGF) gekennzeichnet. Der VEGF besteht eigentlich aus einer Familie von Liganden (Klagsburn und D'Amore, Cytokine & Growth Factor Rev. 1996; 7: 259 - 70,). Der VEGF bindet den hochaffinen transmembranösen Tyrosinkinaserezepzor KDR und die verwandte fms-Tyrosinkinase-1, auch unter der Bezeichnung Flt-1 oder Gefäßendothelzellenwachstumsfaktorrezeptor 1 (VEGFR-1) bekannt. Aus Zellkultur- und Gen-Knockout-Versuchen geht hervor, dass jeder Rezeptor zu unterschiedlichen Aspekten der Angiogenese beiträgt. Der KDR führt die mitogene Funktion des VEGF herbei, während Flt-1 nichtmitogene Funktionen, wie diejenigen, die mit der Zelladhäsion in Zusammenhang stehen, zu modulieren scheint. Eine Hemmung des KDR moduliert daher das Niveau der mitogenen VEGF-Aktivität. Tatsächlich wurde gezeigt, dass das Tumorwachstum von der antiangiogenen Wirkung der VEGF-Rezeptor-Antagonisten beeinflusst wird (Kim et al., Nature 1993; 362: 841 - 4).

Drei PTK (Protein-Tyrosinkinase)-Rezeptoren für VEGFR sind identifiziert worden: VEGFR-1 (Flt-1); VEGRF-2 (Flk-1 oder KDR) und VEGFR-3 (Flt-4). Das die Hemmung von VEGFR (Vascular Endothelial Growth Factor Receptor 2 - KDR) für die Tumortherapie von Bedeutung ist, konnte mit den eingeführten Medikamenten Sunitinib, Sorafenib und Vatalanib gezeigt werden, die auch VEGFR inhibieren.

### HIPK1

HIPK1, eine Nuklearproteinkinase, die in Brustkrebszellen angereichert vorliegt, dient dazu, verschiedene Transkriptionsfaktoren, einschließlich p53, zu phosphorylieren.

Es kann angenommen warden, daß HIPK1 bei Krebs und der Tumorgenese eine Rolle spielt, und zwar durch Regulierung von p53 und/oder der Mdm2-Funktion.

Y. Aikawa et al. beschreiben in EMBO J. 2006; 25: 3955 - 65 die homeodomain-interacting Proteinkinasen HIPK1, HIPK2 und HIPK3 als potentielle Targets zur Behandlung von Tumorerkrankungen.

R. Copeland et al. al beschreiben in FASEB J. 2008; 22: 1050 ff. HIPK1 als Target zur Behandlung von Krebserkrankungen.

### HIPK2 (Homeodomain interacting protein kinase)

Die deutlich verstärkte Expression von HIPK2 bei Gebärmutterhalskrebs korreliert offensichtlich mit der Progression der Erkrankung (Eva Krieghoff-Henning javascript:popRef('a1') & Thomas G Hofmann Future Oncology 2008; 4 (6): 751 - 54).

Die Rolle von Aurora-Kinase-Inhibitoren bei der Behandlung von Tumoren werden von einer Reihe von Autoren veschrieben:
D.S. Boss et al., The Oncologist 2009; 14: 780 - 93;
S. Lapenna et al., Nature Rev. Drug Discov. 2009; 8: 547 - 66;
L. Garuti et al., Cur. Med. Chem. 2009; 16, 1949 - 63;
J. R. Pollard et al., J. Med. Chem. 2009; 52 (9): 2629 - 51;
C.H.A. Cheung et al., Expert Opin. Investig. Drugs 2009; 18 (4): 379 - 98.

Die vorliegende Erfindung betrifft daher die Verwendung der Verbindungen der Formel I zur Behandlung von Krankheiten oder Zuständen, bei denen eine Hemmung der Aktivität von Poteinkinasen, insbesondere der Proteinkinasen NUAK1, auch ARK5 genannt, MAPK (MAP2K1, MAP4K2, MAP3K11), MARK3, ROCK2, auch Rho-Kinase 2 genannt, CHEK1, auch CHK1 genannt, CDK2, PKN2, KDR, auch VEGFR genannt, HIPK1 und AURORA, vorteilhaft ist.

Die Synthese von kleinen Verbindungen, die die Signaltransduktion der Tyrosinkinasen und/oder Serin/Threonin-Kinasen, insbesondere der oben genannten Proteinkinasen spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Im einzelnen betrifft die vorliegende Erfindung Verbindungen der Formel I, die die Signaltransduktion von Proteinkinasen hemmen, regulieren und/oder modulieren, Zusammensetzungen, die diese Verbindungen enthalten, sowie Verfahren zu ihrer Verwendung zur Behandlung von kinasebedingten Krankheiten und Leiden wie Angiogenese, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Feste Tumore, insbesondere schnell wachsende Tumore, können mit Kinasehemmern behandelt werden. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom.

Weiterhin können die Verbindungen der Formel la und Ib verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO J. 1997; 16: 2783 - 93) und Modelle transgener Tiere (z.B. White et al., Oncogene 2001; 20: 7064 - 72). Zur Bestimmung bestimmter Stufen in der Signalübertragungskaskade können wechselwirkende Verbindungen genutzt werden, um das Signal zu modulieren (z.B. Stephens et al., Biochemical J. 2000; 351: 95 - 105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996; 399 (3): 333 - 8) oder dem basischen Myelinprotein sind in der Literatur beschrieben (z.B. Campos-González, R. und Glenney, Jr., J.R. J. Biol. Chem. 1992; 267: 14535).

Zur Identifikation von Kinase-Inhibitoren stehen verschiedene Assay-Systeme zur Verfügung. Beim Scintillation-Proximity-Assay (Sorg et al., J. Biomol. Screen. 2002; 7: 11 - 19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. Biomol. Screen. 2002; 7 (3): 191 - 214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al. Biochem. J. 2002, 366: 977 - 981).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK

Andere heterocyclische Verbindungen sind als Aurora-Kinase-Inhibitoren zur Krebsbekämpfung von verschiedenen Autoren beschrieben:
L. Garuti et al., Curr. Med. Chem. 2009 ; 16 : 1949 - 63;
J. R. Pollard et al., J. Med. Chem. 2009; 52 (9): 2629 - 51;
C.H.A. Cheung et al., Expert Opin. Investig. Drugs 2009; 18 (4): 379 - 98.
J.K.Buolamwini beschreibt in Current Pharmaceutical Design 2009; 6, (4): 379 - 92 andere heterocyclische Verbindungen, u.a. ein Diaminothiazolderivat als CDK-Inhibitor.
Thiazolopyrazole sind als Arzneimittel in WO 2005/095420 beschrieben. Die Synthese von Pyrrolo[2,1-b]thiazolen wird in Heterocycles 2007; 71: 761 - 98 von A.V. Tverkhlebov beschrieben.
L. Grehn beschreibt in Chemica Scripta 1978; 13: 78 - 95 Verfahren zur Herstellung von Pyrrolothiazolen.
S. Athmani et al. beschreiben in J. Chem. Soc. Perkin Trans. 1992; 973-77 die Synthese von Pyrrolo-[2,3-d]thiazolen.
Eine andere Synthese von Pyrrolo-[3,2-d]thiazolen wird von A. Shafiee et al. in J. Heterocyclic Chem. 1979; 16: 1563 - 66 beschrieben.
Andere heterocyclische Verbindungen werden vom M.L.Dekhtyar in Dyes and Pigments 2007; 74: 744 - 48 als Farbstoffe beschrieben.
Andere Pyrrolothiazole werden in Keratinfarbstoff-formulierungen in WO 2005/077324 offenbart.
Andere 3-Pyridyl- und 4-Isochinolinyl-thiazolderivate sind als C17,20 Lyase-Inhibitoren in der WO 03/027085 A2 beschrieben. Andere heterocyclische Verbindungen sind als KSP-Inhibitoren in der WO 2006/119146 A1 beschrieben.

### ZUSAMMENFASSUNG DER ERFINDUNG

Die Erfindung betrifft Verbindungen der Formel la und Ib 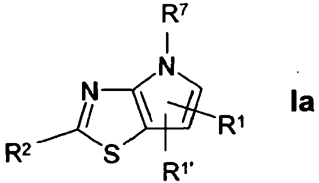 worin
- R¹: H, (CH₂)ₙAr oder (CH₂)ₙHet,
- R^{1'}: H oder A,
- R²: H, A, Hal, (CH₂)ₙHet¹, (CH₂)ₙHet³, -C≡C-Ar, (CH₂)ₙAr², NHCONHAr, COA, COOH, COOA, COHet¹, COAr², CONH₂, CONHA, CONA₂, SO₂NH₂, SO₂NHA, S(O)ₘA, NHCOA, SO₂NHA, SO₂NA₂, SO₂NHHet¹, SO₂NHAr² oder 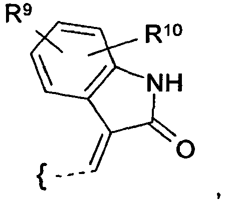
- R⁷: H, A, Ar oder Het¹,
- Ar: unsubstituiertes oder ein-, zwei- oder dreifach durch Hal, A, OH, OA, CN, NO₂, SO₂A COOH, COOA, NH₂, NHA, NA₂, NHCH₂Ar¹, CHO, COA, CHO, CONH₂, CONHA, CONA₂, Het, SO₂NH₂, SO₂NHA, S(O)ₘA und/oder NHCOA substituiertes Phenyl,
- Het: einen ein- oder zweikernigen aromatischen Heterocyclus mit 1 bis 4 N-, O- und/oder S-Atomen, der unsubstituiert oder ein-, zwei- oder dreifach durch Hal, A, NH₂, NHA, NA₂, COOH, COOA, CONH₂, CONHA, CONA₂, CONHAr, CONHHet⁴, S(O)ₘA und/oder NHCH₂Ar¹ substituiert sein kann,
- Ar¹: unsubstituiertes oder ein-, zwei- oder dreifach durch Hal, A, OH und/oder OA substituiertes Phenyl,
- Ar²: einfach durch NHCONHHet⁴ substituiertes Phenyl,
- Het¹: einen ein- oder zweikernigen aromatischen, ungesättigten oder gesättigten Heterocyclus mit 1 bis 4 N-, O- und/oder S-Atomen, der unsubstituiert oder ein-, zwei- oder dreifach durch Hal, A, COOA, NH₂, NHR⁸ und/oder (CH₂)ₙHet² substituiert sein kann,
- R⁸: H, A, Ar¹ oder Het⁴,
- R⁹, R¹⁰: jeweils unabhängig voneinander H oder Hal,
- R¹¹: H oder A',
- Het²: einen ein- oder zweikernigen aromatischen Heterocyclus mit 1 bis 4 N-, O- und/oder S-Atomen, der unsubstituiert oder ein-, zwei- oder dreifach durch A, COOA und/oder NH₂ substituiert sein kann,
- Het³: einen ein- oder zweikernigen aromatischen Heterocyclus mit 1 bis 4 N-, O- und/oder S-Atomen, der unsubstituiert oder ein-, zwei- oder dreifach durch NH₂ substituiert sein kann,
- Het⁴: einen ein- oder zweikernigen aromatischen, ungesättigten oder gesättigten Heterocyclus mit 1 bis 4 N-, O- und/oder S-Atomen, der unsubstituiert oder ein-, zwei- oder dreifach durch Hal, A, Ar¹, NH₂, NHCH₂Ar¹ und/oder =O substituiert sein kann,
- A: unverzweigtes oder verzweigtes Alkyl mit 1-10 C-Atomen, worin 1-7 H-Atome durch F ersetzt sein können und/oder worin eine oder zwei nicht-benachbarte CH- und/oder CH₂-Gruppen durch O, NH, NA', S, SO, SO₂ und/oder durch CH=CH-Gruppen ersetzt sein können,
oder
cyclisches Alkyl mit 3-7 C-Atomen,
- A': unverzweigtes oder verzweigtes Alkyl mit 1-4 C-Atomen,
- Hal: F, Cl, Br oder I,
- m: 0, 1 oder 2,
- n: 0, 1, 2, 3 oder 4,
bedeuten,
sowie ihre pharmazeutisch verwendbaren Salze, Tautomere und Stereoisomere, einschließlich deren Mischungen in allen Verhältnissen,
wobei 4H-Pyrrolo[2,3-d]thiazol, 2,5-dimethyl-4H-pyrrolo[2,3-d]thiazol, 6-methyl-4H-pyrrolo[2,3-d]thiazole, 2-methyl-4H-pyrrolo[2,3-d]thiazole, 5-methyl-4H-pyrrolo[2,3-d]thiazole, 4-methyl-4H-pyrrolo[2,3-d]thiazole, 2,4,5-trimethyl-4H-pyrrolo[2,3-d]thiazole, 2,4-dimethyl-4H-pyrrolo[2,3-d]thiazole, 4,5-dimethyl-4H-pyrrolo[2,3-d]thiazole, 4,6-dimethyl-4H-pyrrolo[2,3-d]thiazole ausgeschlossen sind.

Die Verbindungen der Formel Ib sind synthetische Vorstufen bei der Herstellung von Verbindungen der Formel la, und stellen wie die Verbindungen der Formel la potente Kinase-Inhibitoren dar.

Unter Verbindungen der Formel la versteht man auch die Hydrate und Solvate dieser Verbindungen, ferner pharmazeutisch verwendbare Derivate. Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel la und Ib, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 1995; 115: 61 - 67 beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens,
einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel Ia, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel Ia und ihre Salze sowie ein Verfahren zur Herstellung von Verbindungen der Formel la sowie ihrer pharmazeutisch verwendbaren Salze, Tautomeren und Stereoisomeren, dadurch gekennzeichnet, daß man
a) zur Herstellung einer Verbindung der Formel la', 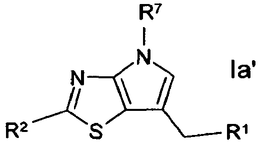 worin
   - R¹: Ar oder Het,
   - R²: außer Hal, die in Anspruch 1 angegebenen Bedeutungen hat,
   - R⁷: tert.-Butyloxycarbonyl
   bedeuten,
   und Ar und Het die in Anspruch 1 angegebenen Bedeutungen haben,
   eine Verbindung der Formel IIc 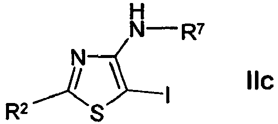 worin
   - R²: außer Hal, die in Anspruch 1 angegebene Bedeutung hat,
   - R⁷: tert.-Butyloxycarbonyl
   bedeuten,
   mit einer Verbindung der Formel IIIc

   R¹-CH=CH-CH₂Br IIIc

   worin R¹ Ar oder Het bedeutet,
   und Ar und Het die in Anspruch 1 angegebenen Bedeutungen haben,
   umsetzt,
   und anschließend oder gleichzeitig die tert.-Butyloxycarbonyl-Gruppe abspaltet,
   oder
b) zur Herstellung einer Verbindung der Formel la" 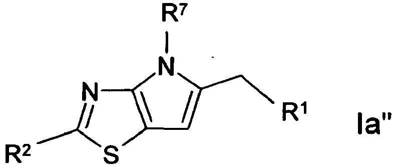 worin
   - R¹: Ar oder Het,
   - R²: H, Cl, (CH₂)ₙHet³
   - R⁷: H
   bedeuten,
   und Ar, Het und Het³ die in Anspruch 1 angegebenen Bedeutungen haben,
   eine Verbindung der Formel Ib' 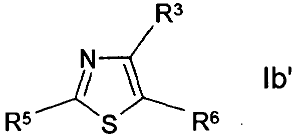 worin
   - R³: NH-COO-tert.-butyl,
   - R⁵: H, Cl, (CH₂)ₙHet³,
   - R⁶: -C≡C-R⁴
   - R⁴: Ar oder Het,
   bedeuten,
   und n, Ar, Het, Het³ und R⁴ die in Anspruch 1 angegebenen bedeutungen haben,
   in Gegenwart einer Base erhitzt,
   oder
c) zur Herstellung einer Verbindung der Formel la" 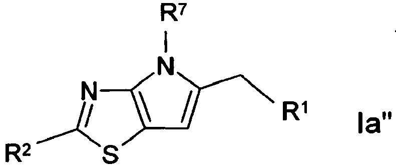 worin
   - R¹: Ar oder Het,
   - R²: Het¹
   - R⁷: H
   bedeuten,
   eine Verbindung der Formel la" 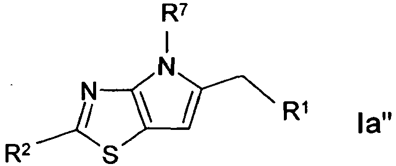 worin
   - R¹: Ar oder Het,
   - R²: Hal
   - R⁷: H
   bedeuten,
   Ar und Het die in Anspruch 1 angegebenen Bedeutungen haben,
   mit einer Verbindung der Formel Va

   Het¹-H Va

   worin Het¹ die in Anspruch 1 angegebene Bedeutung hat,
   umsetzt,
   und/oder
   eine Base oder Säure der Formel la in eines ihrer Salze umwandelt.

Vor- und nachstehend haben die Reste R¹, R^{1'}, R², R³, R⁵, R⁶ und R⁷ die bei den Formeln la und Ib angegebenen Bedeutungen, sofern nicht ausdrücklich etwas anderes angegeben ist.

Für alle Reste, die mehrfach auftreten, daß deren Bedeutungen unabhängig voneinander sind.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. A bedeutet auch unverzweigtes oder verzweigtes Alkyl mit 1-10 C-Atomen, worin 1-7 H-Atome durch OH, F, Cl und/oder Br ersetzt sein können.
A bedeutet weiterhin z.B. 2-Hydroxyethyl, 3-Hydroxypropyl, 2-Methoxyethyl oder 3-Methoxypropyl.
Cyclisches Alkyl (Cycloalkyl) bedeutet vorzugsweise Cyclopropyl, Cyclobutyl, Cylopentyl, Cyclohexyl oder Cycloheptyl.

A' bedeutet Alkyl, dieses ist unverzweigt (linear) oder verzweigt, und hat 1, 2, 3, 4, 5 oder 6 C-Atome. A' bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A' bedeutet ganz besonders bevorzugt Methyl, Ethyl, Propyl, Isopropyl, Butyl, Isobutyl, sek.-Butyl, tert.-Butyl, Pentyl, Hexyl oder Trifluormethyl.

R¹ bedeutet vorzugsweise 4-Fluorphenyl, Benzyl, 3-Aminophenyl, 3-(Benzylamino)phenyl oder 2-Amino-pyridin-4-yl.
R^{1'} bedeutet vorzugsweise H.
R² bedeutet vorzugsweise H, Hal, Het¹ oder -C≡C-Ar.
R³ bedeutet vorzugsweise NHCOOA' oder NH₂.
R⁷ bedeutet vorzugsweise H.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, o-, m- oder p-Cyanphenyl, o-, m- oder p-Carboxyphenyl, o-, m- oder p-Methoxycarbonylphenyl, o-, m- oder p-Aminosulfonylphenyl, o-, m- oder p-(Benzylamino)phenyl weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet besonders bevorzugt unsubstituiertes oder ein-, zwei- oder dreifach durch Hal, A, OH, OA, CN, NO₂, SO₂A, COOH, COOA, NH₂, NHA, NA₂, NHCH₂Ar¹, CHO, COA, CHO, CONH₂, CONHA, CONA₂, SO₂NH₂, SO₂NHA und/oder NHCOA substituiertes Phenyl.
Ar bedeutet ganz besonders bevorzugt unsubstituiertes oder ein-, zwei- oder dreifach durch Hal, A, OH, OA, NH₂, NHA, NA₂ und/oder NHCH₂Ar¹ substituiertes Phenyl.
Ar¹ bedeutet vorzugsweise Phenyl.

Het bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, Pyrrolopyridinyl, Purinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.
Het bedeutet besonders bevorzugt unsubstituiertes oder ein- oder zweifach durch NH₂ und/oder NHCH₂Ar¹ substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl.

Het¹ bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, Pyrrolopyridinyl, Purinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl, Aza-bicyclo[3.2.1]-octyl oder Dibenzofuranyl.

Die heterocyclischen Reste können auch teilweise oder vollständig hydriert sein.

Ungeachtet weiterer Substitutionen kann Het¹ also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, - 6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydro-benzofuranyl, 2,3-Dihydro-2-oxo-furanyl, 3,4-Dihydro-2-oxo-1 H-chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.
Het¹ bedeutet besonders bevorzugt unsubstituiertes oder ein- oder zweifach durch NH₂ und/oder CH₂Het² substituiertes Piperidinyl, Pyrrolidinyl, Morpholinyl, Piperazinyl, Imidazolidinyl, Oxazolidinyl, Tetrahydropyranyl, Thiazolyl, Thiophenyl, Furanyl, Pyrrolyl, Oxazolyl, Isoxazolyl, Oxadiazolyl, Pyrazolyl, Imidazolyl, Triazolyl, Thiadiazolyl, Pyridazinyl, Pyrazinyl, Pyridinyl oder Pyrimidinyl.

Het² bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, Pyrrolopyridinyl, Purinyl, weiter bevorzugt 1,3-Benzo-dioxol-5-yl, 1,4-Benzodioxan-6-yl, 2,1,3-Benzothiadiazol-4-oder-5-yl, 2,1,3-Benzoxadiazol-5-yl oder Dibenzofuranyl.
Het² bedeutet besonders bevorzugt unsubstituiertes oder ein- oder zweifach durch A und/oder NH₂ substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl.

Het³ bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, Pyrrolopyridinyl, Purinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.
Het³ bedeutet besonders bevorzugt unsubstituiertes oder ein- oder zweifach durch A und/oder NH₂ substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl.

Het⁴ bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder-5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, Pyrrolopyridinyl, Purinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl, Aza-bicyclo[3.2.1]-octyl oder Dibenzofuranyl.
Die heterocyclischen Reste können auch teilweise oder vollständig hydriert sein.
Ungeachtet weiterer Substitutionen kann Het⁴ also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5-oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, - 6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl, 3,4-Dihydro-2-oxo-1H-chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.
Het⁴ bedeutet besonders bevorzugt unsubstituiertes oder ein- oder zweifach durch NH₂, NHCH₂Ar¹ und/oder =O substituiertes Piperidinyl, Pyrrolidinyl, Morpholinyl, Piperazinyl, Imidazolidinyl, Oxazolidinyl, Tetrahydropyranyl, Dihydroisoindolyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl.

Hal bedeutet vorzugsweise F, Cl oder Br, aber auch I, besonders bevorzugt F oder Cl; m bedeutet vorzugsweise 1 oder 2; n bedeutet vorzugsweise 0, 1, 2 oder 3.

Für die gesamte Erfindung gilt, daß sämtliche Reste, die mehrfach auftreten, gleich oder verschieden sein können, d.h. unabhängig voneinander sind.
Die Verbindungen der Formel la können ein oder mehrere chirale Zentren besitzen und daher in verschiedenen stereoisomeren Formen vorkommen. Die Formel la umschließt alle diese Formen.

Dementsprechend sind Gegenstand der Erfindung insbesondere diejenigen Verbindungen der Formel Ia, in denen mindestens einer der genannten Reste eine der vorstehend angegebenen bevorzugten Bedeutungen hat. Einige bevorzugte Gruppen von Verbindungen können durch die folgenden Teilformeln laa bis lah ausgedrückt werden, die der Formel la entsprechen und worin die nicht näher bezeichneten Reste die bei der Formel la angegebene Bedeutung haben, worin jedoch
- in Iaa Ar: unsubstituiertes oder ein-, zwei- oder dreifach durch Hal, OH, OA, NH₂, NHA, NA₂ und/oder NHCH₂Ar¹ substituiertes Phenyl
bedeutet;
- in lab Het: unsubstituiertes oder ein- oder zweifach durch NH₂ und/oder NHCH₂Ar¹ substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl
bedeutet;
- in lac Ar¹: Phenyl bedeutet;
- in lad Het¹: unsubstituiertes oder ein- oder zweifach durch NH₂ und/oder CH₂Het² substituiertes Piperidinyl, Pyrrolidinyl, Morpholinyl, Piperazinyl, Imidazolidinyl, Oxazolidinyl, Tetrahydropyranyl, Thiazolyl, Thiophenyl, Furanyl, Pyrrolyl, Oxazolyl, Isoxazolyl, Oxadiazolyl, Pyrazolyl, Imidazolyl, Triazolyl, Thiadiazolyl, Pyridazinyl, Pyrazinyl, Pyridinyl oder Pyrimidinyl
bedeutet;
- in lae Het²: unsubstituiertes oder ein- oder zweifach durch A und/oder NH₂ substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl,
bedeutet;
- in laf Het³: unsubstituiertes oder ein- oder zweifach durch A und/oder NH₂ substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl,
bedeutet;
- in lag Het⁴: unsubstituiertes oder ein- oder zweifach durch NH₂, NHCH₂Ar¹ und/oder =O substituiertes Piperidinyl, Pyrrolidinyl, Morpholinyl, Piperazinyl, Imidazolidinyl, Oxazolidinyl, Tetrahydropyranyl, Dihydroisoindolyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl,
bedeutet;
- in lah R¹: H, (CH₂)ₙAr oder (CH₂)ₙHet,
- R^{1'}: H,
- R²: H, Hal, (CH₂)ₙHet¹, (CH₂)ₙHet³ oder -C≡C-Ar,
- R⁷: H,
- Ar: unsubstituiertes oder ein-, zwei- oder dreifach durch Hal, A, OH, OA, CN, NO₂, SO₂A, COOH, COOA, NH₂, NHA, NA₂, NHCH₂Ar¹, CHO, COA, CHO, CONH₂, CONHA, CONA₂, SO₂NH₂, SO₂NHA und/oder NHCOA substituiertes Phenyl,
- Het: einen ein- oder zweikernigen aromatischen Heterocyclus mit 1 bis 4 N-, O- und/oder S-Atomen, der unsubstituiert oder ein-, zwei- oder dreifach durch Hal, A, NH₂ und/oder NHCH₂Ar¹ substituiert sein kann,
- Ar¹: unsubstituiertes oder ein-, zwei- oder dreifach durch Hal, A, OH und/oder OA substituiertes Phenyl,
- Het¹: einen ein- oder zweikernigen aromatischen, ungesättigten oder gesättigten Heterocyclus mit 1 bis 4 N-, O- und/oder S-Atomen, der unsubstituiert oder ein-, zwei- oder dreifach durch Hal, A, NH₂ und/oder CH₂Het² substituiert sein kann,
- Het²: einen ein- oder zweikernigen aromatischen Heterocyclus mit 1 bis 4 N-, O- und/oder S-Atomen, der unsubstituiert oder ein-, zwei- oder dreifach durch A und/oder NH₂ substituiert sein kann,
- Het³: einen ein- oder zweikernigen aromatischen Heterocyclus mit 1 bis 4 N-, O- und/oder S-Atomen, der unsubstituiert oder ein-, zwei- oder dreifach durch NH₂ substituiert sein kann,
- Het⁴: einen ein- oder zweikernigen aromatischen, ungesättigten oder gesättigten Heterocyclus mit 1 bis 4 N-, O- und/oder S-Atomen, der unsubstituiert oder ein-, zwei- oder dreifach durch Hal, A, NH₂, NHCH₂Ar¹ und/oder =O substituiert sein kann,
- A: unverzweigtes oder verzweigtes Alkyl mit 1-10 C-Atomen, worin 1-7 H-Atome durch OH, F, Cl und/oder Br ersetzt sein können,
- A': unverzweigtes oder verzweigtes Alkyl mit 1-4 C-Atomen,
- Hal: F, Cl, Br oder I,
- n: 0, 1, 2, 3 oder 4,
bedeuten;
sowie ihre pharmazeutisch verwendbaren Salze, Tautomere und Stereoisomere, einschließlich deren Mischungen in allen Verhältnissen.

Gegenstand der Erfindung sind weiterhin Verbindungen ausgewählt aus der Gruppe

| Nr. | Name und/oder Struktur |
|---|---|
| "2" | *tert*.-Butyl-*N*-(5-iodo-1,3-thiazol-4-yl)carbamat |
| "A15" | *tert*.-Butyl-*N*-{5-[2-(4-fluorphenyl)ethinyl]-1,3-thiazol-4-yl}carbamat |
| | 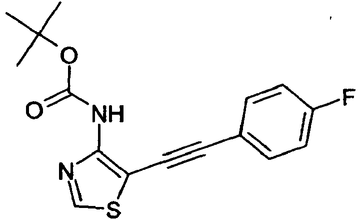 |
| "A16" | [5-(3-Phenyl-prop-1-inyl)-thiazol-4-yl]-carbaminsäure-tert.-butylester |
| | 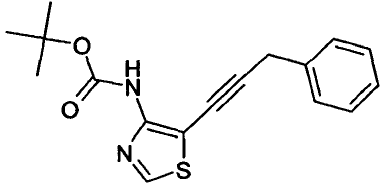 |
| "A17" | (5-Triethylsilanylethinyl-thiazol-4-yl)-carbaminsäure-tert.-butylester |
| | 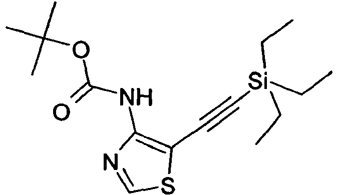 |
| "A18" | [5-(3-Amino-phenylethinyl)-thiazol-4-yl]-carbaminsäure-tert.-butylester |
| | 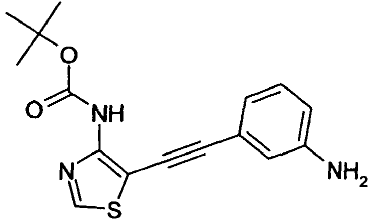 |
| "A19" | [5-(3-Benzylamino-phenylethinyl)-thiazol-4-yl]-carbaminsäure-tert.-butylester |
| | 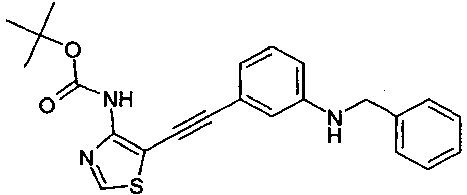 |
| "A20" | [5-(2-Benzylamino-pyridin-4-ylethinyl)-thiazol-4-yl]-carbaminsäure-tert.-butyl ester |
| | 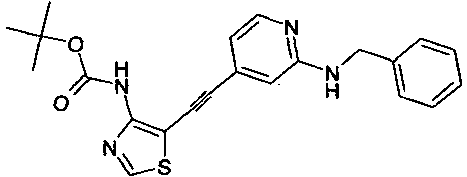 |
| "A21" | [5-(1H-Pyrrolo[2,3-b]pyridin-5-ylethinyl)-thiazol-4-yl]-carbaminsäure-tert.-butyl ester |
| | 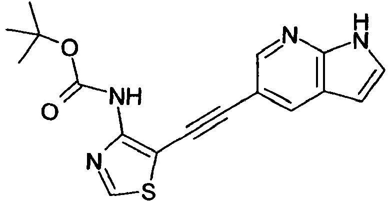 |
| "A22" | 5-(1H-Pyrrolo[2,3-b]pyridin-5-ylethinyl)-thiazol-4-ylamin |
| | 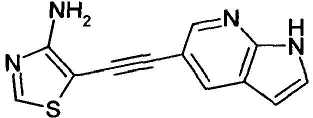 |
| "A24" | *tert.*-Butyl-*N*-(2-pyridin-4-yl-5-iodo-1,3-thiazol-4-yl)carbamat |
| | 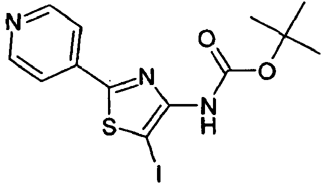 |
| "A25" | [5-(3-Morpholin-4-yl-prop-1-inyl)-2-pyridin-4-yl-thiazol-4-yl]-carbaminsäure-tert.-butylester |
| | 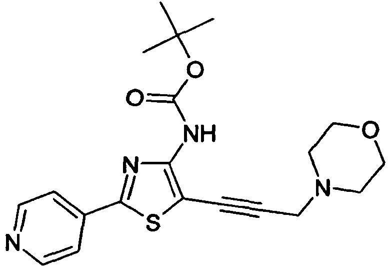 |
| "A26" | {5-[3-(1,3-Dioxo-1,3-dihydro-isoindol-2-yl)-prop-1-inyl]-thiazol-4-yl}-carbaminsäure-tert.-butylester |
| | 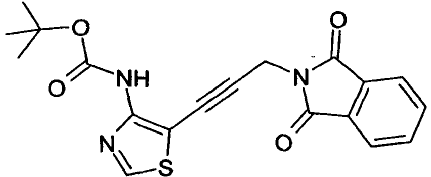 |
| "A27" | 3-(4-Amino-thiazol-5-ylethinyl)-phenol |
| | 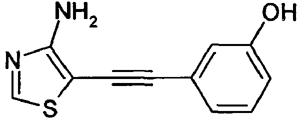 |
| "A28" | [2-(2-Amino-pyridin-3-yl)-5-(4-fluor-phenylethinyl)-thiazol-4-yl]-carbaminsäure-tert.-butylester |
| | 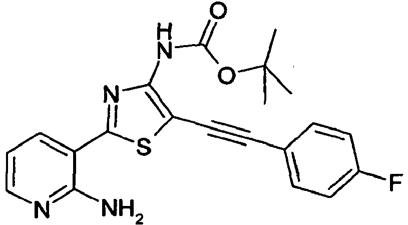 |
| "A29a" | *tert*.-Butyl-*N*-{2-chlor-5-[2-(4-fluorphenyl)ethinyl]-1,3-thiazol-4-yl}carbamat |
| | 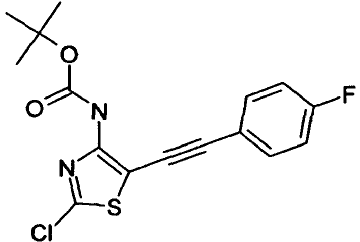 |
| "A29" | *tert*.-Butyl-*N*-[2-(2-aminopyrimidin-5-yl)-5-[2-(4-fluorphenyl)ethinyl]-1,3-thiazol-4-yl]carbamat |
| | 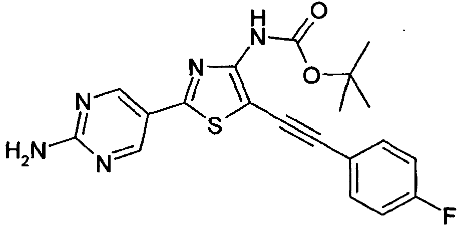 |

sowie ihre pharmazeutisch verwendbaren Salze, Tautomere und Stereoisomere, einschließlich deren Mischungen in allen Verhältnissen. Gegenstand der Erfindung sind auch Arzneimittel enthaltend mindestens eine der oben genannten Verbindungen und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

Die Verbindungen der Formel la und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel la' können vorzugsweise erhalten werden, indem man eine Verbindung der Formel IIc mit einer Verbindung der Formel IIIc umsetzt.
Die Ausgangsverbindungen der Formeln IIc und IIIc sind in der Regel bekannt. Sind sie neu, so können sie aber nach an sich bekannten Methoden hergestellt werden.
Die Umsetzung erfolgt unter Bedingungen wie sie dem Fachmann für eine Amino-Palladierungsreaktion-Reaktion bekannt sind (Beispiel 1). Diverse Katalysatoren können Anwendung finden.

Die Reaktionszeit liegt je nach den angewendeten Bedingungen zwischen einigen Minuten und 14 Tagen, die Reaktionstemperatur zwischen etwa -30° und 140°, normalerweise zwischen 30° und 120°, insbesondere zwischen etwa 60° und etwa 110°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist DMF.

Verbindungen der Formel la" können auch vorzugsweise erhalten werden, indem man eine Verbindung der Formel Ib" in Gegenwart einer Base erhitzt (Beispiel 5). Als Base eignet sich vorzugsweise Kalium-tert.-butylat.
Die Reaktionszeit liegt je nach den angewendeten Bedingungen zwischen einigen Minuten und 14 Tagen, die Reaktionstemperatur zwischen etwa -30° und 140°, normalerweise zwischen 30° und 120°, insbesondere zwischen etwa 80° und etwa 110°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist NMP (N-Methylpyrrolidon).

Analog zum Verfahrensschritt b) erhält man die erfindungsgemäßen Verbindungen der Formel la" 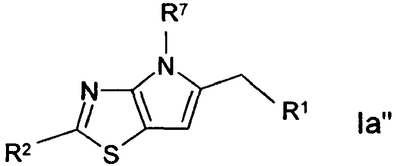 worin
- R¹: Ar oder Het,
- R⁷: H
bedeuten,
und R², Ar, Het die in Anspruch 1 angegebenen Bedeutungen haben,
indem man eine Verbindung der Formel Ib' 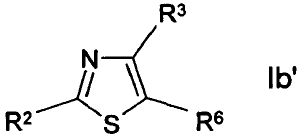 worin
- R³: NH-COO-tert.-butyl,
- R⁶: -C≡C-R⁴
- R⁴: Ar oder Het,
bedeuten,
und Ar, Het, R² und R⁴ die in Anspruch 1 angegebenen Bedeutungen haben,
in Gegenwart einer Base erhitzt (Beispiel 5).

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Die Verbindungen der Formeln la können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel la oder Ib entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel la oder Ib entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel la oder Ib aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel la werden größtenteils konventionell hergestellt. Sofern die Verbindung der Formel la eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel Ia zählen ebenfalls dazu. Bei bestimmten Verbindungen der Formel la lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel la die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel Ia, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkyl-halogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser-als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Besonders bevorzugt sind Hydrochlorid, Dihydrochlorid, Hydrobromid, Maleat, Mesylat, Phosphat, Sulfat und Succinat.

Die Säureadditionssalze basischer Verbindungen der Formel la werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel la mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel la in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel la und/oder ihre pharmazeutisch verwendbaren Salze und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, liche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel la und/oder ihre pharmazeutisch verwendbaren Salze und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-ÖI-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel la sowie deren Salze davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel la sowie die Salze davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Res. 1986; 3 (6): 318 ff. allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel la hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind. Gegenstand der Erfindung sind ferner Arzneimittel enthaltend mindestens eine Verbindung der Formel la und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel la und/oder ihrer pharmazeutisch verwendbaren Salze und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel la und/oder ihrer pharmazeutisch verwendbaren Salze und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel la und/oder ihre physiologisch unbedenklichen Salze zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.
Ebensfalls umfasst ist die Verwendung der erfindungsgemäßen Verbindungen nach Anspruch 1 und/oder ihre physiologisch unbedenklichen Salze zur Herstellung eines Arzneimittels zur Behandlung oder Vorbeugung einer Krankheit, an der Angiogenese beteiligt ist.
Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die Verwendung von Verbindungen der Formel la und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung der Verbindungen der Formel la und/oder ihre physiologisch unbedenklichen Salze zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.
Die vorliegende Erfindung umfasst auch die Verwendung Verbindungen der Formel la und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.
Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel la können an Patienten zur Behandlung von Krebs, insbesondere schnell wachsenden Tumoren, verabreicht werden.

Gegenstand der Erfindung ist somit die Verwendung von Verbindungen der Formel la, sowie ihrer pharmazeutisch verwendbaren Salze und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt ist die Verwendung von Verbindungen der Formel I, sowie ihrer pharmazeutisch verwendbaren Salze und Stereoisomere, einschließlich deren Mischungen in allen Verhältnissen,
zur Herstellung eines Arzneimittels zur Behandlung von Krankheiten, die durch Inhibierung der Tyrosinkinasen durch die Verbindungen nach Anspruch 1 beeinflußt werden.

Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren der Lunge, des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens und/oder des Kehlkopfs.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die offenbarten Verbindungen der Formel la können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD-1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (Cl-1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-ανβ3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS-2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und Invivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | BBR-3464 (Hoffrnann-La Roche) |
| | Ormiplatin | SM-11355 (Sumitomo) |
| | Iproplatin | |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour- Ipsen) |
| | 7-Ethyl-10-hydroxycamptothecin | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon (Exelixis) | CKD-602 (Chong Kun Dang) |
| | BBR-3576 (Novuspharrna) | KW-2170 (Kyowa Hakko) |
| | | |
| Antitumor-Antibiotika | Dactinomycin (Actinomycin D) | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | |
| | Therarubicin | Bleomycinsulfat (Blenoxan) |
| | Idarubicin | Bleomycinsäure |
| | Rubidazon | Bleomycin A |
| | Plicamycinp | Bleomycin B |
| | Porfiromycin | Mitomycin C |
| | Cyanomorpholinodoxorubicin | MEN-10755 (Menarini) |
| | Mitoxantron (Novantron) | GPX-100 (Gem Pharmaceuticals) |
| | | |
| Antimitotische Mittel | Paclitaxel | SB 408075 |
| | Docetaxel | (GlaxoSmithKline) |
| | Colchicin | E7010 (Abbott) |
| | Vinblastin | PG-TXL (Cell Therapeutics) |
| | Vincristin | IDN 5109 (Bayer) |
| | Vinorelbin | A 105972 (Abbott) |
| | Vindesin | A 204197 (Abbott) |
| | Dolastatin 10 (NCl) | LU 223651 (BASF) |
| | Rhizoxin (Fujisawa) | D 24851 (ASTA Medica) |
| | Mivobulin (Warner-Lambert) | ER-86526 (Eisai) |
| | Cemadotin (BASF) | Combretastatin A4 (BMS) |
| | RPR 109881A (Aventis) | Isohomohalichondrin-B (PharmaMar) |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | IDN-5109 (Indena) |
| | Cryptophycin 52 (Eli Lilly) | AVLB (Prescient NeuroPharma) |
| | Vinflunin (Fabre) | Azaepothilon B (BMS) |
| | Auristatin PE (Teikoku Hormone) | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase-Inhibitoren | Pemetrexed (Eli Lilly) ZD-9331 (BTG) | Nolatrexed (Eximias) CoFactor™ (BioKeys) |
| | | |
| DNA-Antagonisten | Trabectedin (PharmaMar) | Mafosfamid (Baxter International) |
| | Glufosfamid (Baxter International) | Apaziquon (Spectrum Pharmaceuticals) |
| | Albumin + 32P (Isotope Solutions) | O6-Benzylguanin (Paligent) |
| | Thymectacin (NewBiotics) | |
| | Edotreotid (Novartis) | |
| | | |
| Farnesyltransferase-Inhibitoren | Arglabin (NuOncology Labs) | Tipifarnib (Johnson & Johnson) |
| | lonafarnib (Schering-Plough) | |
| | BAY-43-9006 (Bayer) | Perillylalkohol (DOR BioPharma) |
| | | |
| Pumpen-Inhibitoren | CBT-1 (CBA Pharma) | Zosuquidar-Trihydrochlorid |
| | Tariquidar (Xenova) | (Eli Lilly) |
| | MS-209 (Schering AG) | Biricodar-Dicitrat (Vertex) |
| | | |
| Histonacetyltrans- | Tacedinalin (Pfizer) | Pivaloyloxymethylbutyrat |
| ferase-Inhibitoren | SAHA (Aton Pharma) | (Titan) |
| | MS-275 (Schering AG) | Depsipeptid (Fujisawa) |
| | | |
| Metalloproteinase-Inhibitoren | Neovastat (Aeterna Laboratories) | CMT -3 (CollaGenex) |
| | | BMS-275291 (Celltech) |
| Ribonucleosid | Marimastat (British Biotech) | Tezacitabin (Aventis) |
| reduktase-Inhibitoren | Galliummaltolat (Titan) | Didox (Molecules for Health) |
| | Triapin (Vion) | |
| | | |
| TNF-alpha-Agonisten / Antagonisten | Virulizin (Lorus Therapeutics) CDC-394 (Celgene) | Revimid (Celgene) |
| | | |
| Endothelin-A-Rezeptor-Antagonisten | Atrasentan (Abbot) ZD-4054 (AstraZeneca) | YM-598 (Yamanouchi) |
| | | |
| Retinsäurerezeptor-Agonisten | Fenretinid (Johnson & Johnson LGD-1550 (Liqand) | Alitretinoin (Ligand) |
| | | |
| Immunmodulatoren | Interferon | Dexosom-Therapie (Anosys |
| | Oncophage (Antigenics) | Pentrix (Australian C ancer Technology) |
| | GMK (Progenics) | |
| | Adenokarzinom-Impfstoff (Biomira) | |
| | | JSF-154 (Tragen) |
| | CTP-37 (AVI BioPharma) | Krebsimpfstoff (Intercell) |
| | JRX-2 (Immuno-Rx) | |
| | PEP-005 (Peplin Biotech) | Norelin (Biostar) |
| | Synchrovax-Impfstoffe (CTL Immuno) | BLP-25 (Biomira) |
| | | MGV (Progenics) |
| | Melanom-Impfstoff (CTL Immuno) | !3-Alethin (Dovetail) |
| | | CLL-Thera (Vasogen) |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | |
| | Testosteron | Leuporelin |
| | Testosteronpropionat | Bicalutamid |
| | Fluoxymesteron | Flutamid |
| | Methyltestosteron | Octreotid |
| | Diethylstilbestrol | Nilutamid |
| | Megestrol | Mitotan |
| | Tamoxifen | P-04 (Novogen) |
| | Toremofin | 2-Methoxyöstradiol |
| | Dexamethason | |
| | | (EntreMed) |
| | | Arzoxifen (Eli Lilly) |
| | | |
| Photodynamische Mittel | Talaporfin (Light Sciences) | Pd-Bacteriopheophorbid (Yeda) |
| | Theralux (Theratechnologies) Motexafin-Gadolinium (Pharmacyclics) | |
| | | Lutetium-Texaphyrin (Pharmacyclics) |
| | | Hypericin |
| | | |
| Tyrosinkinase-Inhibitoren | Imatinib (Novartis) | Kahalid F (PharmaMar) |
| | Leflunomid (Sugen/Pharmacia | CEP- 701 (Cephalon) |
| | ZDI839 (AstraZeneca) | CEP-751 (Cephalon) |
| | Erlotinib (Oncogene Science) | MLN518 (Millenium) |
| | Canertjnib (Pfizer) | PKC412 (Novartis) |
| | Squalamin (Genaera) | Phenoxodiol O |
| | SU5416 (Pharmacia) | Trastuzumab (Genentech) |
| | SU6668 (Pharmacia) | C225 (ImClone) |
| | ZD4190 (AstraZeneca) | rhu-Mab (Genentech) |
| | ZD6474 (AstraZeneca) | MDX-H210 (Medarex) |
| | Vatalanib (Novartis) | |
| | PKI166 (Novartis) | 2C4 (Genentech) |
| | GW2016 (GlaxoSmithKline) | MDX-447 (Medarex) |
| | EKB-509 (Wyeth) | ABX-EGF (Abgenix) |
| | EKB-569 (Wyeth) | IMC-1C11 (ImClone) |
| Verschiedene Mittel | SR-27897 (CCK-A-Inhibitor, Sanofi-Synthelabo) | BCX-1777 (PNP-Inhibitor, BioCryst) |
| | Tocladesin (cyclisches-AMP-Agonist, Ribapharm) | Ranpirnase (Ribonuclease-Stimulans, Alfacell) |
| | Alvocidib (CDK-Inhibitor, Aven CV-247 (COX-2-Inhibitor, Ivy | Galarubicin (RNA-Synthese-Inhibitor, Dong-A) |
| | Medical) P54 (COX-2-Inhibitor, Phytopharm) | Tirapazamin (Reduktionsmittel, SRI International) |
| | CapCell™ (CYP450-Stimulans Bavarian Nordic) | |
| | | N-Acetylcystein (Reduktionsmittel, Zambon) |
| | GCS-IOO (gal3-Antagonist, GlycoGenesys) | |
| | | R-Flurbiprofen (NF-kappaB-Inhibitor, Encore) |
| | G17DT-Immunogen (Gastrin-Inhibitor, Aphton) | |
| | | 3CPA (NF-kappaB-Inhibitor Active Biotech) |
| | Efaproxiral (Oxygenator, Allos Therapeutics) | |
| | | Seocalcitol (Vitamin-D-Rezeptor-Agonist, Leo) |
| | PI-88 (Heparanase-Inhibitor, Progen) | |
| | | 131-I-TM-601 (DNA-Antagonist, Trans Molecular) |
| | Tesmilifen (Histamin-Antagonist, YM BioSciences) | |
| | Histamin (Histamin-H2-Rezept Agonist, Maxim) | |
| | | Eflornithin (ODC-Inhibitor, ILEX Oncology) |
| | Tiazofurin (IMPDH-Inhibitor, Ribapharm) | |
| | | Minodronsäure |
| | Cilengitid (Integrin-Antagonist, | (Osteoclasten-Inhibitor, |
| | Merck KGaA) | Yamanouchi) |
| | SR-31747 (IL-1-Antagonist, Sanofi-Synthelabo) | Indisulam (p53-Stimulans, Eisai) |
| | CCI-779 (mTOR-Kinase-Inhibitor, Wyeth) | Aplidin (PPT-Inhibitor, PharmaMar) |
| | Exisulind (PDE-V-Inhibitor, Cell Pathways) | Rituximab (CD20-Antikörpen Genentech) |
| | CP-461 (PDE-V-Inhibitor, Cell Pathways) | Gemtuzumab (CD33-Antikörper, Wyeth Ayerst) |
| | AG-2037 (GART-Inhibitor, Pfizer) | |
| | | PG2 (Hämatopoese-Verstärker, Pharmagenesis, |
| | WX-UK1 (Plasminogenaktivator- Inhibitor, Wilex) | |
| | | Immunol™ (Triclosan-Oralspülung, Endo) |
| | PBI-1402 (PMN-Stimulans, ProMetic LifeSciences) | |
| | | Triacetyluridin (Uridin-Prodrug, Wellstat) |
| | Bortezomib (Proteasom-Inhibit Millennium) | |
| | | SN-4071 (Sarkom-Mittel, Signature BioScience) |
| | SRL-172 (T-Zell-Stimulans, SF Pharma) | |
| | | TransMID-107™ (Immunotoxin, KS Biomedix) |
| | TLK-286 (Glutathion-S-Transferase-Inhibitor, Telik) | |
| | | |
| | PT-100 (Wachstumsfaktor-Agonist, Point Therapeutics) | |
| | | PCK-3145 (Apoptose-Förderer, Procyon) |
| | Midostaurin (PKC-Inhibitor, Novartis) | |
| | | Doranidazol (Apoptose-Förderer, Pola) |
| | Bryostatin-1 (PKC-Stimulans, GPC Biotech) | |
| | | CHS-828 (cytotoxisches Mittel, Leo) |
| | CDA-II (Apoptose-Förderer, Everlife) | |
| | | trans-Retinsäure (Differentiator, NIH) |
| | SDX-101 (Apoptose-Förderer, Salmedix) | |
| | | MX6 (Apoptose-Förderer, MAXIA) |
| | Ceflatonin (Apoptose-Förderer ChemGenex) | |
| | | Apomin (Apoptose-Förderer, ILEX Oncology) |
| | | Urocidin (Apoptose-Fördere Bioniche) |
| | | Ro-31-7453 (Apoptose-Förderer, La Roche) |
| | | Brostallicin (Apoptose-Förderer, Pharmacia) |
| | | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel la wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al. Cancer Res.1999; 59: 189 - 97; Xin et al. J. Biol. Chem.1999 ; 274: 9116 - 21; Sheu et al. Anticancer Res.1998 ; 18: 4435 - 41; Ausprunk et al. Dev. Biol.1974; 38: 237 - 48; Gimbrone et al. J. Natl. Cancer Inst. 1974; 52: 413 - 27; Nicosia et al. In Vitro 1982; 18: 538 - 49).

### Filterbindungsassay

Die Substanzen werden nach folgenden Bestimmungen getestet:
Alle verwendeten Geräte, Lösungsmittel, Buffer und Enzyme sind kommerziell erhältlich.
Alle Kinase Assays sind mit dem Multidrop 384 von Thermo-Fisher bei Raumtemperatur in einem totalen Assay Volumen von 25.5 µL durchgeführt worden. Zu dem Testansatz aus entweder 0.5 µL Testsubstanz, DMSO Kontrolle oder unbesetztem Behälter werden 15 µL eines Enzymmixes aus Enzym und Substrat in Puffer gegeben. Testsubstanzen werden in Gegenwart des Enzyms und des Substrates über 5 Minuten vorinkubiert bevor durch Zusatz von 10 µL ATP (Konzentration kinaseabhängig von 5, 20 oder 50 mM) die Reaktion initiiert wird. Die Reaktion wird durch Zugabe von 5 µL Orthophosphorsäure (50 mM) beendet. Die Testbehälter werden dann auf P81 Unifilter Platten mittels eines "Packard Harvester" überführt und an der Luft getrocknet. Die trockenen Unifilter Platten werden nach der Zugabe von MicroScint O verschlossen und die Radioaktivität in einem Packard Topcount NXT ermittelt.

### Assay zur NUAK1-Inhibierung

NUAK1 (5-20 mU verdünnt in 50 mM Tris pH 7.5, 0.1 mM EGTA, 0.1% β-Mercaptoethanol, 1mg/ml BSA) wird gegen ALNRTSSDSALHRRR als Substrat getestet. Das endgültige Volumen von 25.5 µl enthält 50 mM Tris pH 7.5, 0.1 mM EGTA, 0.3 mM ALNRTSSDSALHRRR, 10 mM Magnesium Azetat and 0.02 mM [³³P-γ-ATP] (50-1000 cpm/pmole) und wird für 30 min bei Raumtemperatur inkubiert. Tests werden durch die Zugabe von 5 µl einer 0.5 M (3%) Orthophosphorsäure-Lsg. gestoppt und dann auf P81 Unifilter Platten übertragen und mit 50 mM Orthophosphorsäure Puffer gewaschen.

### Messung der Met Kinase Aktivität

Die Met Kinase wird laut Herstellerangaben (Met, active, Upstate, Katalog-Nr. 14-526) zum Zweck der Proteinproduktion in Insektenzellen (Sf21; S. frugiperda) und der anschließenden affinitätschromatographischen Aufreinigung als "N-terminal 6His-tagged" rekombinantes humanes Protein in einem Baculovirus-Expressionsvektor exprimiert.

Zur Messung der Kinase-Aktivität kann auf verschiedene zur Verfügung stehender Meßsysteme zurückgegriffen werden. Beim Scintillation-Proximity-(Sorg et al., J. Biomol. Screen. 2002; 7: 11 - 19), dem FlashPlate-Verfahren oder dem Filterbindungstest wird die radioaktive Phosphorylierung eines Proteins oder Peptids als Substrat mit radioaktiv markiertem ATP (³²P-ATP, ³³P-ATP) gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. Biomol. Screen. 2002; 7 (3): 191 - 214). Andere nicht radioaktive ELISA-Assay-Verfahren verwenden spezifische Phospho-Antikörper (Phospho-AK). Der Phospho-Antikörper bindet nur das phosphorylierte Substrat. Diese Bindung ist mit einem zweiten Peroxidasekonjugierten Antikörper durch Chemilumineszenz nachweisbar (Ross et al. Biochem. J. 2002, 366: 977 - 981).

### Flashplate-Verfahren (Met Kinase):

Als Testplatten dienen 96-well Flashplate^{R} Mikrotiterplatten der Firma Perkin Elmer (Kat.-Nr. SMP200). In die Assay Platte werden die Komponenten der unten beschriebenen Kinasereaktion pipettiert.
Die Met Kinase und das Substrat poly Ala-Glu-Lys-Tyr, (pAGLT, 6:2:5:1). werden mit radioaktiv markiertem ³³P-ATP in An- und Abwesenheit von Testsubstanzen in einem Gesamtvolumen von 100 µl bei Raumtemperatur 3 Std. inkubiert. Die Reaktion wird mit 150 µl einer 60mM EDTA-Lösung abgestoppt. Nach Inkubation für weitere 30 min bei Raumtemperatur werden die Überstände abgesaugt und die Wells dreimal mit je 200 µl 0,9% NaCl-Lösung gewaschen. Die Messung der gebundenen Radioaktivität erfolgt mittels eines Szintillationsmessgerätes (Topcount NXT, Fa. Perkin-Elmer). Als Vollwert wird die Inhibitor-freie Kinasereaktion verwendet. Dieser sollte ca. im Bereich von 6000-9000 cpm liegen. Als pharmakologischer Nullwert wird Staurosporin in einer Endkonzentration von 0,1 mM verwendet. Eine Bestimmung der Hemmwerte (IC₅₀) erfolgt unter Verwendung des Programms RS1_MTS.

Kinase-Reaktionsbedingungen pro well:
30 µl Assaypuffer
10 µl zu testende Substanz in Assaypuffer mit 10 % DMSO
10 µl ATP (Endkonzentration 1 µM kalt, 0,35 pCi ³³P-ATP)
50 µl Gemisch Met Kinase/Substrat in Assaypuffer;
   (10 ng Enzym/well, 50 ng pAGLT/well)

### Verwendete Lösungen:

- Assay-Puffer:
   50 mM HEPES
   3 mM Magnesiumchlorid
   3 µM Natrium orthovanadat
   3 mM Mangan (II) chlorid
   1 mM Dithiothreitol (DTT)
   pH= 7,5 (einzustellen mit Natriumhydroxid)
- Stopp-Lösung:
   60 mM Titriplex III (EDTA)
- ³³P-ATP: Perkin-Elmer;
- Met Kinase: Upstate, Kat.-Nr. 14-526, Stock 1 µg/10 µl; spez. Aktivität 954 U/mg;
- Poly-Ala-Glu-Lys-Tyr, 6:2:5:1: Sigma Kat.-Nr. P1152

### In vivo-Tests

Experimenteller Ablauf: Weibliche Balb/C Mäuse (Züchter: Charles River Wiga) waren bei der Ankunft im Alter von 5 Wochen. Sie wurden 7 Tage lang an unsere Haltungsbedingungen akklimatisiert. Anschließend wurden jeder Maus 4 Millionen TPR-Met / NIH3T3 - Zellen in 100 µl PBS (ohne Ca++ und Mg++) subkutan im Beckenbereich injiziert. Nach 5 Tagen wurden die Tiere in 3 Gruppen randomisiert, so dass jede Gruppe von 9 Mäusen ein mittleres Tumorvolumen von 110 µl (Spanne: 55 - 165) hatte. Der Kontrollgruppe wurden 100 µl Vehikel (0,25 % Methylzellulose / 100 mM Acetatpuffer, pH 5.5), den Behandlungsgruppen wurden 200 mg/kg "A56" bzw. "A91" gelöst im Vehikel (Volumen ebenfalls 100 µl / Tier) per Schlundsonde täglich verabreicht. Nach 9 Tagen hatten die Kontrollen ein mittleres Volumen von 1530 µl und der Versuch wurde beendet.

Messung des Tumorvolumens: Die Länge (L) und Breite (B) wurde mit einer Schubleere gemessen und das Tumorvolumen nach der Formel LxBxB/2 berechnet.

Haltungsbedingungen: je 4 bzw. 5 Tiere pro Käfig, Fütterung mit kommerziellem Mäusefutter (Fa. Sniff).

### Bedingungen für die auf HPLC basierenden Analytik-Methoden:

**ESI-MS:** Agilent 1200 Binary Pump
Solvent A: Wasser/0.05% Ameisensäure
Solvent B: ACN/0.04% Ameisensäure
Minimun pressure limit (bar): 0.0; Maximum pressure limit (bar): 200.0;
Post run time (min): 0.00;
Gradient program: Time Flow Rate Composition
0.00(min) 2.40(ml/min) A=96.0% B=4.0% gradient to
2.80(min) 2.40 (ml/min) A=0.0% B=100.0%
3.30(min) 2.40 (ml/min) A=0.0% B=100.0% gradient to
3.40(min) 2.40 (ml/min) A=96.0% B=4.0%
Column: Chromolith Performance Speed ROD RP18e/50-4.6nm
Autosampler: Agilent 1200 ALS G1329A
Pump: Agilent 1200 BinPump G1312A, Degasser: Agilent 1200 G1379B
Detector: Agilent 1200 VWL G1314B
MS: Agilent 6110 Quadrupole LC/MS
ESI interface G1946-60450, positive ionization (or negative ionization as indicated below), Injection volume: 10 µL

**ESI-HRMS:** Agilent 1100 Binary Pump
Solvent A: Wasser/0.1% FAc
Solvent B: ACN/0.1% FAc
Minimun pressure limit (bar): 0.0; Maximum pressure limit (bar): 400.0;
Post run time (min): 0.00
Gradient program: Time Flow Rate Composition
0.00(min) 0.50(ml/min) A=98.0% B=2.0%
5.00(min) 0.50(ml/min) A=2.0% B=98.0%
8.00(min) 0.50(ml/min) A=2.0% B=98.0%
8.10(min) 0.50(ml/min) A=98.0% B=2.0%
13.00(min) 0.50(ml/min) A=98.0% B=2.0%
Column: 135 - Purospher Star RP-18e (3µm), 55-2, Art. 1.50241, No. 242994
Autosampler: Agilent 1100 ALS G1329A
Pump: Agilent 1100 BinPump G1312A
Column oven: Agilent 1100 Column Oven G1316A
Detektion: Agilent 1100 DAD G1315B
MS: Thermo LTQ XL Orbitrap
ESI interface
positive ionization
Injection volume: 5 - 20 uL

### APCI-MS:

Solvent A:Wasser + 0.1 %HCOOH
Solvent B:Acetonitril + 0.1% HCOOH
Solvent C:C
Solvent D:D
Min pressure (Bar):0
Max pressure (Bar):300
Delay volume (ml):0.00
Equilibration time (min):0.00
Gradient curve: Linear

**Gradient program:**

| Time(min) | Flow(mL/min) | A(%) | B(%) | C(%) | D(%) |
|---|---|---|---|---|---|
| 0.00 | 0.50 | 98 | 2 | 0 | 0 |
| 5.00 | 0.50 | 2 | 98 | 0 | 0 |
| 8.00 | 0.50 | 2 | 98 | 0 | 0 |
| 8.10 | 0.50 | 98 | 2 | 0 | 0 |
| 13.00 | 0.50 | 98 | 2 | 0 | 0 |

Säule: Purosphere RP-18, 55-2, Art. 1.50241.0001, Lot.: 641047
Pumpe: Finnigan Spectra System P4000
Detektion : Finnigan UV6000LP
MS: Finnigan LCQ Deca
APCI interface, Positive ionisation
Inj. Vol.: 10 µL

### $

Agilent
Säule: Chromolite Performance RP18-e 50-4,6mm
A: Acetonitril mit 0,05% Ameisensäure
B:H₂O mit 0,05% Ameisensäure
Fluß: 2,4mL/min

**Methode:**

| Zeit [min] | %B |
|---|---|
| 0 | 4 |
| 2.8 | 100 |
| 3.3 | 100 |
| 3.4 | 4 |

### §

Agilent
Säule:
   XBridge C8 (50x4.6 mm, 3.5 µ), +ve mode
Methode:
   A: 0.1% TFA in H₂O
   B: 0.1% TFA in Acetonitril
Flußrate: 2.0 ml/min

**Gradient:**

| Zeit [min] | %B |
|---|---|
| 0 | 5 |
| 8.0 | 100 |
| 8.1 | 100 |
| 8.5 | 5 |
| 10 | 5 |

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | EI (Elektronenstoß-Ionisation) M⁺ |
| | FAB (Fast Atom Bombardment) (M+H)⁺ |
| | ESI (Electrospray lonization) (M+H)⁺ |

APCI-MS (atmospheric pressure chemical ionization - mass spectrometry) (M+H)⁺;
Schmelzpunkt F. [°C].

### Beispiel 1

### Herstellung von 6-Benzyl-4H-pyrrolo[2,3-d]thiazol ("A7")

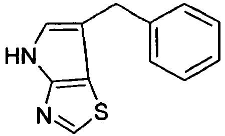

### 1.1 tert.-Butyl-N-(1,3-thiazol-4-yl)carbamat ("1")

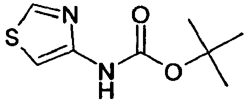

In eine Lösung von 4.85 g (37.55 mmol) 4-Thiazolcarbonsäure in 180 ml *tert.-*Butanol werden unter Stickstoff 5.80 ml (41.84 mmol) Triethylamin und anschließend unter Eisbadkühlung 9.10 ml (42.19 mmol) Diphenylazidophosphat eingetragen und das Reaktionsgemisch über 16 h unter Rückfluss erhitzt. Das Lösungsmittel wird im Vakuum entfernt, der Rückstand in Dichlormethan aufgenommen und zweimal mit Wasser und gesättigter NaCl-Lösung gewaschen. Die organische Phase wird über Natriumsulfat getrocknet, im Vakuum eingeengt, und der Rückstand über Kieselgel chromatographisch aufgereinigt (Eluent: Cyclohexan/Ethylacetat 1/1). Es werden 7.07 g (35.30 mmol, 94 %) *tert*.-butyl-*N*-(1,3-thiazol-4-yl)carbamat als beige Kristalle erhalten; ESI-MS: m/z: 201 ([M+H]⁺).

### 1.2 tert.-Butyl-N-(5-iodo-1,3-thiazol-4-yl)carbamat ("2")

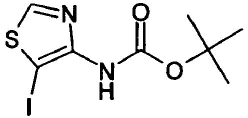

In eine Lösung von 800 mg (3.99 mmol) *tert*.-butyl-*N*-(1,3-thiazol-4-yl)-carbamat in 40 ml Dichlorethan werden 1033 mg (4.59 mmol) *N*-Iodsuccinimid eingetragen und das Reaktionsgemisch über 2h zum Rückfluss erhitzt. Nach Abkühlen wird zweimal mit Wasser und mit gesättigter Natriumthiosulfat-Lösung gewaschen. Die vereinigten organischen Phasen werden über Natriumsulfat getrocknet, im Vakuum eingeengt, und der Rückstand über Kieselgel flash-chromatographisch aufgereinigt (Eluent: Cyclohexan / Ethylacetat 8/2 zu 1/1). Es werden 1.19 g (3.43 mmol, 85%) *tert*.-butyl-*N*-(5-iodo-1,3-thiazol-4-yl)carbamat als weiße Kristalle nach Kristallisation aus Diethylether erhalten; ESI-MS: m/z: 327 ([M+H]⁺).

1.3 In einem getrockneten Schlenkkolben werden unter Stickstoff 652 mg (2.00 mmol) *tert*.-Butyl-*N*-(5-iodo-1,3-thiazol-4-yl)carbamat und 2.6 g (8.00 mmol) Cäsiumcarbonat in 10 ml trockenem DMF gelöst und mit 622 mg (3.00 mmol) Cinnamylbromid versetzt. Nach 2 h Rühren bei Raumtemperatur und positiver Reaktionskontrolle mittels Dünschichtchromatographie werden 22 mg (0.10 mmol) Pd(OAc)₂ und 52 mg (0.20 mmol) PPh₃ eingetragen und das Reaktionsgemisch für 19 h bei 100°C gerührt. Das Reaktionsgemisch wird dann mit gesättigter NaCl-Lösung versetzt und mit Ethylacetat extrahiert. Die vereinigten organischen Phasen werden über Natriumsulfat getrocknet, im Vakuum zusammen mit der ca. dreifachen Menge an SiO₂ eingeengt und über 5 h bei ca. 10 mbar auf 100°C erhitzt. Das Substrat-Kieselgel-Gemisch wird anschließend direkt chromatographisch über eine Kieselgelsäule aufgereinigt (Eluent: Cyclohexan/Ethylacetat 1/1). Es werden 214 mg (0.99 mmol, 49%) 6-Benzyl-4*H*-pyrrolo[2,3-d]thiazol als gelber Rückstand erhalten;
F. 133-135;
APCI-MS: Rₜ: 3.90 min; m/z (%): 215 (100, [M+H]⁺);
¹H NMR (400 MHz, DMSO-d₆): δ [ppm] 3.90 (s, 2H), 7.01 (dd, *J*_{H,H} = 2.3 Hz, *J*_{H,H} =1.3 Hz, 1H), 7.14-7.34 (m, 5H), 8.65 (d, *J*_{H,H} = 1.3 Hz, 1H), 11.58 (s, 1 H);
¹³C NMR (100 MHz, DMSO-d₆): δ [ppm] 32.14, 111.80, 113.02, 119.80, 125.42, 127.78, 127.96, 140.00, 149.75, 151.27.

### Beispiel 2

### Herstellung von tert.-Butyl-N-{5-[2-(4-fluorphenyl)ethinyl]-1,3-thiazol-4-yl}carbamat ("A15")

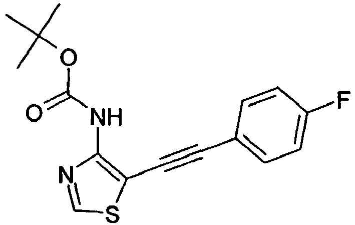

2.93 g (9 mmol) Cäsiumcarbonat werden im evakuierten Schlenkkolben unter Temperatur getrocknet und anschließend unter Stickstoff 50 ml trockenes Tetrahydrofuran, 978 mg (3.00 mmol) *tert*.-Butyl-*N*-(5-iodo-1,3-thiazol-4-yl)carbamat, 396 mg (3.30 mmol) 1-Ethinyl-4-Fluorbenzol, 122 mg (0.15 mmol) Pd(dppf)₂Cl₂•CH₂Cl₂ und 57 mg (0.30 mmol) Cul zugegeben. Das Reaktionsgemisch wird über 6 h auf 50°C erhitzt, anschließend abgekühlt und über Celite filtriert und das Lösungsmittel im Vakuum entfernt. Der Rückstand wird mit Ethylacetat aufgenommen und mit gesättigter NaCl-Lösung gewaschen. Die vereinigten organischen Phasen werden über Natriumsulfat getrocknet, im Vakuum eingeengt, und der Rückstand über Kieselgel chromatographisch aufgereinigt (Eluent: Cyclohexan/Ethylacetat 1/1). Es werden 820 mg (2.57 mmol, 85%) *tert*.-Butyl-*N*-{5-[2-(4-fluorphenyl)ethinyl]-1,3-thiazol-4-yl}carbamat als gelber Feststoff aus Diethylether erhalten;
F. 139-144;
¹H NMR (400 MHz, DMSO-d₆): δ [ppm] 1.45 (s, 9H), 7.22 - 7.39 (m, 2H), 7.50 - 7.68 (m, 2H), 8.98 (s, 1 H), 9.62 (s, 1 H);
¹³C NMR (100 MHz, DMSO-d₆): δ [ppm] 27.43, 78.84, 96.31, 105.54, 115.35 (d, ²*J*_{C,F} = 22 Hz), 117.92, 117.95, 132.88 (d, ³*J*_{C,F} = 8 Hz), 150.65, 151.83, 152.1, 161.63 (d, ¹*J*_{C,F} = 246 Hz);
ESI-MS: Rₜ: 2.47 min; m/z (%): 319 (100, [M+H]⁺).

### Beispiel 3

### Herstellung von tert.-Butyl-N-{2-chlor-5-[2-(4-fluorphenyl)ethinyl]-1,3-thiazol-4-yl}carbamat ("A29a")

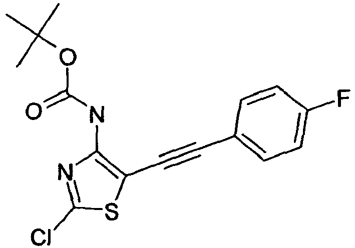

Im getrockneten Schlenkkolben werden unter Stickstoff 2.63 g (8.26 mmol) *tert*.-Butyt-*N*-{5-[2-(4-fluorphenyl)ethinyl]-1,3-thiazol-4-yl}carbamat in 100 ml trockenem THF gelöst und auf -78°C abgekühlt. Dann werden 11 ml einer 15%-igen Lösung von *n*-BuLi in n-Hexan langsam zugetropft und 20 min bei -78°C gerührt. Anschließend werden 1120 mg (8.38 mmol) *N*-Chlorsuccinimid in 1 ml trockenem THF gelöst hinzugetropft und bei -78°C 15 min gerührt, dann mit 10 ml *n*-Butanol gequencht und auf RT erwärmt. Das Lösungsmittel wird im Vakuum entfernt und der Rückstand direkt über Kieselgel chromatographisch aufgereinigt (Eluent: Cyclohexan/Ethylacetat 99/1). Es werden 2.3 g (6.51 mmol, 78%) *tert*.-Butyl-*N*-{2-chlor-5-[2-(4-fluorphenyl)ethinyl]-1,3-thiazol-4-yl}carbamat als beiger Feststoff aus PE erhalten; ESI-MS: m/z: 353 ([M+H]⁺).

### Beispiel 4

### Herstellung von tert.-Butyl-N-[2-(2-aminopyrimidin-5-yl)-5-[2-(4-fluorphenyl)ethinyl]-1,3-thiazol-4-yl]carbamat ("A29")

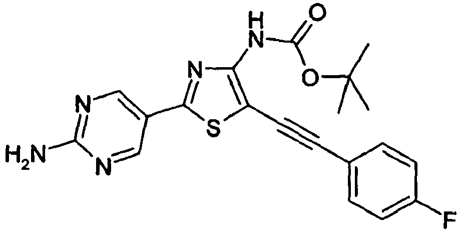

705 mg (2.00 mmol) *tert*.-Butyl-*N*-{2-chlor-5-[2-(4-fluorphenyl)ethinyl]-1,3-thiazol-4-yl}carbamat werden zusammen mit 663 mg (3.00 mmol) 2-Aminopyrimidine-5-boronsäurepinacolester in einer Mischung aus 4 ml DMF, 2 ml Wasser und 8 ml DME gelöst und 829 mg (6.00 mmol) Kaliumcarbonat und 81 mg (0.10 mmol) Pd(dppf)₂Cl₂•CH₂Cl₂ eingetragen. Durch das Reaktionsgemisch wird anschließend 10 min Argon geleitet, das Gefäß verschlossen und für 44 h auf 85°C erhitzt. Nach Abkühlen wird das Reaktionsgemisch zweimal mit gesättigter NaCl-Lösung extrahiert und die wässrigen Phasen mit Ethylacetat extrahiert. Die vereinigten organischen Phasen werden über Natriumsulfat getrocknet, im Vakuum eingeengt, und der Rückstand über Kieselgel flash-chromatographisch aufgereinigt (Eluent: Cyclohexan/Ethylacetat 1/2; 1% Triethylamin). Es werden 205 mg (0.49 mmol, 25%) *tert*.-Butyl-*N*-[2-(2-aminopyrimidin-5-yl)-5-[2-(4-fluorphenyl)ethinyl]-1,3-thiazol-4-yl]carbamat als gelber Feststoff erhalten; F. 201-203;
¹H NMR (300 MHz, DMSO-d₆): δ [ppm] 1.46 (s, 9H), 7.23 - 7.49 (m, 4H), 7.51 - 7.70 (m, 2H), 8.72 (s, 2H), 9.63 (s, 1H);
ESI-MS: Rₜ: 2.43 min; m/z (%): 412 (100, [M+H]⁺), 356 (60, [M-tBu+2H]⁺).

### Beispiel 5

### Herstellung von 5-(4-Fluorphenyl)-4H-pyrrolo[2,3-d][1,3]thiazol ("A1")

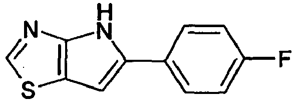

238 mg (0.75 mmol) *tert*.-Butyl-*N*-{5-[2-(4-fluorphenyl)ethinyl]-1,3-thiazol-4-yl}carbamat werden in 4.5 ml NMP im Mikrowellengefäß gelöst und mit 143 mg (1.27 mmol) KO*^{t}*Bu versetzt. Das Reaktionsgefäß wird unter Stickstoff verschlossen und in der Mikrowelle unter Kühlung im Stickstoff-Strom auf 90°C über 20 min erhitzt. Es wird mit 50 ml Diethylether verdünnt und mit 50 ml ges. NaCl-Lösung gewaschen. Die wässrige Phase wird erneut mit Diethylether extrahiert. Die vereinigten organischen Phasen werden über Natriumsulfat getrocknet, im Vakuum eingeengt, und der Rückstand über Kieselgel chromatographisch aufgereinigt (Eluent: Cyclohexan/Ethylacetat 1/1). Es werden 109 mg (0.49 mmol, 66%) 5-(4-Fluorphenyl)-4H-pyrrolo[2,3-d][1,3]thiazol als weiße Kristalle erhalten; F. 244-245;
¹H NMR (300 MHz, DMSO-d₆): δ [ppm] 6.87 (d, *J*_{H,H} = 2.0 Hz, 1H), 7.20-7.29 (m, 2H), 7.75-7.83 (m, 2H), 8.77 (s, 1 H), 12.33 (bs, 1 H).
¹³C NMR (100 MHz, DMSO-d₆): δ [ppm] 96.58, 114.59, 115.75 (d, ²*J*_{C,F} = 19 Hz), 126.05 (d, ³*J*_{C,F} = 7 Hz), 129.24, 135.17, 150.79, 152.97, 161.05 (d, ¹*J*_{C,F} = 250 Hz);
ESI-MS: Rₜ: 2.28 min; m/z (%): 219 (100, [M+H]⁺).

### Beispiel 6

### Herstellung von 5-(4-Fluor-phenyl)-2-morpholin-4-yl-4H-pyrrolo[2,3-d]thiazol ("A12")

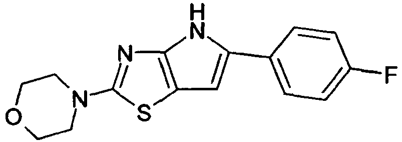

126 mg (0.50 mmol) 2-Chlor-5-(4-fluor-phenyl)-4*H*-pyrrolo[2,3-*d*]thiazol werden im Mikrowellengefäß in 2 ml Morpholin suspendiert und über 2 h bei 130°C bestrahlt und anschließend direkt über Kieselgel chromatographisch aufgereinigt (Eluent: Cyclohexan/Ethylacetat 1/1). Es werden 139 mg (0.45 mmol, 91%) 5-(4-Fluor-phenyl)-2-morpholin-4-yl-4*H*-pyrrolo[2,3-*d*]thiazol als beige Kristalle erhalten; F. 240-248;
¹H NMR (300 MHz, DMSO-d₆): δ [ppm] 3.35-3.51 (m, 4H), 3.66-3.81 (m, 4H), 6.65 (d, *J*_{H,H} = 1.3 Hz, 1H), 7.16 (t, *J*_{H,H} = 8.9 Hz, 2H), 7.55-7.69 (m, 2H), 11.78 (s, 1 H);
¹³C NMR (75 MHz, DMSO-d₆): δ [ppm] 48.03, 65.34, 97.17, 105.45, 115.48 (d, ²*J*_{C,F} = 21 Hz), 124.52 (d, ³*J*_{C,F} = 7 Hz), 128.77, 129.90, 148.59, 160.11 (d, ¹*J*_{C,F} = 240 Hz), 170.62;
ESI-MS: Rₜ: 2.33 min; m/z (%): 304 (100, [M+H]⁺).

### Analog den oben beschriebenen Beispielen erhält man die nachstehenden Verbindungen

| Verbindung Nr. | Name und/oder Struktur | F. [°C]; MS |
|---|---|---|
| "A3" | Referenzbeispiel: 4*H*-Pyrrolo[2,3-*d*]thiazol | 150-151; APCI-MS: Rₜ: 2.50 min; m/z (%): 125 (100, [M+H]⁺) |
| | 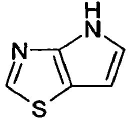 | |
| ¹H NMR (300 MHz, DMSO-d₆): δ [ppm] 6.39 (dd, *J*_{H,H} = 3.1 Hz, *J*_{H,H} = 1.8 Hz, 1H), 7.12 (td, *J*_{H,H} = 3.0 Hz, *J*_{H,H} = 1.4 Hz, 1H), 8.73 (d, *J*_{H,H} = 1.3 Hz, 1H), 11.79 (s, 1H); | | |
| ¹³C NMR (75 MHz, DMSO-d₆) δ [ppm] 98.83, 112.86, 122.94, 150.39, 151.94. | | |
| "A4" | 3-(4*H*-Pyrrolo[2,3-*d*]thiazol-5-yl)-phenylamin | 160-161; ESI-MS: Rₜ: 1.57 min; m/z (%): 216 (100, [M+H]⁺) |
| | 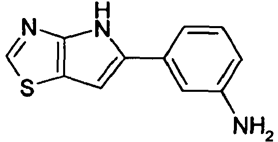 | |
| ¹H NMR (300 MHz, DMSO-d₆): δ [ppm] 5.10 (s, 2H), 6.49 (d, *J*_{H,H} = 7.5 Hz, 1H), 6.69 (d, *J*_{H,H} = 1.6 Hz, 1H), 6.98-6.83 (m, 3H), 7.05 (t, *J*_{H,H} = 8.0 Hz, 1H), 8.73 (s, 1H), 12.15 (s, 1H); | | |
| ¹³C NMR (75 MHz, DMSO-d₆) δ [ppm] 95.82, 109.74, 112.33, 112.90, 114.34, 129.23, 133.16, 137.13, 148.78, 150.08, 152.66. | | |
| "A5" | Benzyl-[4-(4*H*-pyrrolo[2,3-*d*]thiazol-5-yl)-pyridin-2-yl]-amin | ESI-MS: m/z (%): 307 (100, [M+H]⁺) |
| | 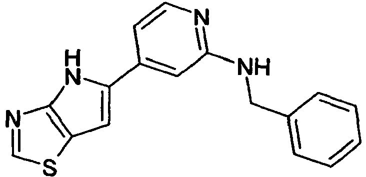 | |
| "A6" | Benzyl-[3-(4*H*-pyrrolo[2,3-*d*]thiazol-5-yl)-phenyl]-amin | 169-170; ESI-MS: m/z (%): 306 (100, [M+H]⁺) |
| | 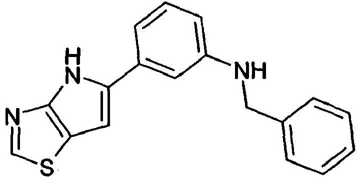 | |
| ¹H NMR (400 MHz, DMSO-d₆): δ [ppm] 4.34 (d, *J*_{H,H} = 6.1 Hz, 2H), 6.27 (t, *J*_{H,H} = 6.1 Hz, 1H), 6.51 (dd, *J*_{H,H} = 8.0 Hz, *J*_{H,H} = 1.5 Hz, 1H), 6.71 (d, *J*_{H,H} = 1.9 Hz, 1H), 6.88 - 7.01 (m, 2H), 7.07 (t, *J*_{H,H} = 7.8 Hz, 1H), 7.22 (t, *J*_{H,H} = 7.3 Hz, 1H), 7.33 (t, *J*_{H,H} = 7.6 Hz, 2H), 7.41 (d, *J*_{H,H} = 7.6 Hz, 2H), 8.74 (s, 1H), 12.20 (s, 1H); | | |
| ¹³C NMR (100 MHz, DMSO-d₆): δ [ppm] 45.83, 95.44, 107.06, 111.13, 111.73, 113.85, 126.07, 126.75, 127.72, 128.69, 132.61, 136.59, 139.79, 148.45, 149.75, 152.14. | | |
| "A2" | 5-Benzyl-4*H*-pyrrolo[2,3-*d*]thiazol | F. 117-122; ESI-MS: m/z: 215 ([M+H]⁺); |
| | 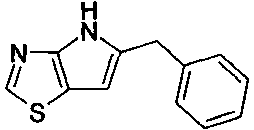 | |
| ¹H NMR (400 MHz, DMSO-d₆): δ [ppm] 3.99 (s, 2H), 6.13 (bs, 1H), 7.17-7.32 (m, 5H), 8.62 (s, 1H), 11.78 (bs, 1H); | | |
| ¹³C NMR (100 MHz, DMSO-d₆): δ [ppm] 33.86, 96.48, 112.32, 125.58, 127.80, 127.90, 136.04, 139.35, 148.15, 150.93; | | |
| APCI-MS: Rₜ: 3.97 min; m/z (%): 215 (100, [M+H]⁺). | | |
| "A8" | 6-Benzyl-2-pyridin-4-yl-4*H*-pyrrolo[2,3-*d*]thiazol | 185-187; ESI-MS: Rₜ: 1.89 min; m/z (%): 292 (100, [M+H]⁺) |
| | 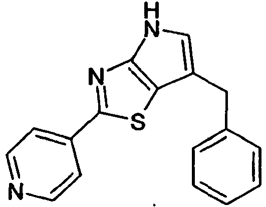 | |
| ¹H NMR (400 MHz, DMSO-d₆): δ [ppm] 3.94 (s, 2H), 7.08-7.39 (m, 6H), 7.75 (dd, *J*_{H,H} = 4.5 Hz, *J*_{H,H} = 1.6 Hz, 2H), 8.61 (dd, *J*_{H,H} = 4.5 Hz, *J*_{H,H} = 1.6 Hz, 2H), 11.86 (s, 1H); | | |
| ¹³C NMR (100 MHz, DMSO-d₆): δ [ppm] 31.87, 113.71, 114.42, 118.53, 122.03, 125.58, 127.91, 128.00, 139.68, 140.45, 150.01, 151.13, 159.15 | | |
| "A9" | 4-(4*H*-Pyrrolo[2,3-*d*]thiazol-5-yl)-pyridin-2-ylamin | ESI-MS: Rₜ: 1.27 min; m/z (%): 217 (100, [M+H]⁺) |
| | 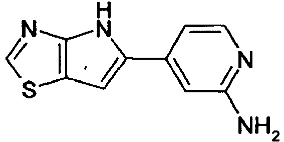 | |
| ¹H NMR (500 MHz, DMSO-d₆): δ [ppm] 6.05 (s, 2H), 6.51 (d, *J*_{H,H} = 8.6 Hz, 1H), 6.66 (d, *J*_{H,H} = 1.9 Hz, 1H), 7.75 (dd, *J*_{H,H} = 8.6 Hz, *J*_{H,H} = 2.4 Hz, 1H), 8.35 (d, *J*_{H,H} = 2.2 Hz, 1H), 8.68 (d, *J*_{H,H} = 3.5 Hz, 1H), 12.09 (s, 1H) | | |
| "A10" | 2-Chlor-5-(4-fluor-phenyl)-4*H*-pyrrolo[2,3-*d*]thiazol | 150-151; ESI(-)-MS: Rₜ: 2.54 min; m/z (%): 250 (100, [M-2H]⁻), 252 (45, [M]⁻), 251 (20, [M-1H]⁻) |
| | 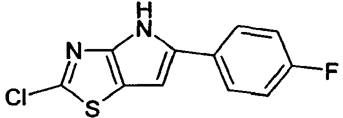 | |
| ¹H NMR (300 MHz, DMSO-d₆): δ [ppm] 6.83 (s, 1H), 7.13-7.35 (m, 2H), 7.66-7.90 (m, 2H), 12.48 (s, 1H); | | |
| ¹³C NMR (75 MHz, DMSO-d₆): δ [ppm] 97.24, 115.78 (d, ²*J*_{C,F} = 21 Hz), 126.15 (d, ³*J*_{C,F} = 7 Hz), 128.78, 134.37, 145.89, 147.25, 150.15, 161.17 (d, ¹*J*_{C,F} = 243 Hz) | | |
| "A11" | 5-(4-Fluor-phenyl)-2-(4-fluor-phenylethinyl)-4*H*-pyrrolo[2,3-*d*]thiazol | >200; ESI-MS: Rₜ: 2.91 min; m/z (%): 337 (100, [M+H]⁺) |
| | 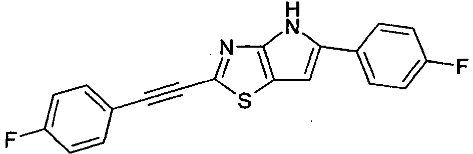 | |
| ¹H NMR (400 MHz, DMSO-d₆): δ [ppm] 6.92 (s, 1H), 7.25-7.36 (m, 4H), 7.68-7.76 (m, 2H), 7.84 (dd, *J*_{H,H} = 8.8 Hz, *J*_{H,H} 5.4 Hz, 2H), 12.55 (s, 1H) | | |
| "A13" | 5-(4-Fluor-phenyl)-2-(4-pyridin-4-ylmethyl-piperazin-1-yl)-4*H*-pyrrolo[2,3-*d*]thiazol | >170; ESI-MS: Rₜ: 1.88 min; m/z (%): 394 (100, [M+H]⁺) |
| | 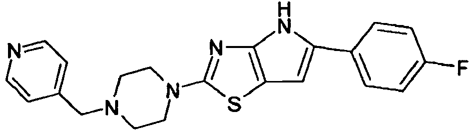 | |
| ¹H NMR (400 MHz, DMSO-d₆): δ [ppm] 2.52-2.56 (m, 4H), 3.42-3.49 (m, 5H), 3.59 (s, 2H), 6.65 (d, *J*_{H,H} = 1.9 Hz, 1H), 7.16 (t, *J*_{H,H}= 8.9, 2H), 7.37 (d, *J*_{H,H}= 5.6 Hz, 2H), 7.62 (dd, *J*_{H,H} = 8.8 Hz, *J*_{H,H} = 5.4 Hz, 2H), 8.53 (d, *J*_{H,H} = 5.3 Hz, 2H), 11.77 (s, 1H); | | |
| ¹³C NMR (100 MHz, DMSO-d₆): δ [ppm] 47.37, 51.16, 59.87, 96.69, 105.00, 115.00 (d, ²*J*_{C,F} = 22 Hz), 120.53, 123.24, 123.94 (d, ³*J*_{C,F} = 8 Hz), 128.08, 129.41, 147.64 (d, ¹*J*_{C,F} = 220 Hz), 148.20, 149.06, 169.79 | | |
| "A14" | 5-[5-(4-Fluor-phenyl)-4*H*-pyrrolo[2,3-*d*]thiazol-2-yl]-pyrimidin-2-ylamin | >220; ESI-MS: Rₜ: 2.20 min m/z (%): 312 (100, [M+H]⁺) |
| | 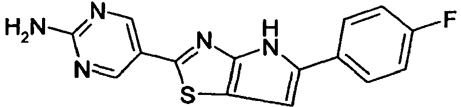 | |
| ¹H NMR (400 MHz, DMSO-d₆): δ [ppm] 6.89 (d, *J*_{H,H}= 1.9 Hz, 1H), 7.20 (s, 2H), 7.21 - 7.30 (m, 4H), 8.74 (s, 2H), 12.37 (s, 1H) | | |
| "A16" | [5-(3-Phenyl-prop-1-inyl)-thiazol-4-yl]-carbaminsäure-tert.-butylester | 121-122; ESI-MS: m/z (%): 259 (100, [M-tBu+2H]⁺), 314 (68, [M]⁺), 315 (10, [M+H]⁺) |
| | 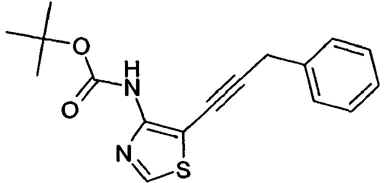 | |
| ¹H NMR (300 MHz, DMSO-d₆): δ [ppm] 1.41 (s, 9H), 3.93 (s, 2H), 7.15 - 7.45 (m, 5H), 8.88 (s, 1H), 9.33 (s, 1H); | | |
| ¹³C NMR (75 MHz, DMSO-d₆): δ [ppm] 25.15, 27.94, 71.64, 79.12, 97.52, 126.59, 127.84, 128.43, 136.01, 150.46, 2x 151.35, 152.64 | | |
| "A17" | (5-Triethylsilanylethinyl-thiazol-4-yl)-carbaminsäure-tert.-butylester | 88-90; APCI-MS: Rₜ: 5.52 min; m/z (%): 282 (100, [M-tBu+2H]⁺), 239 (40, [M-BOC+H]⁺), 338 (17, [M]⁺) |
| | 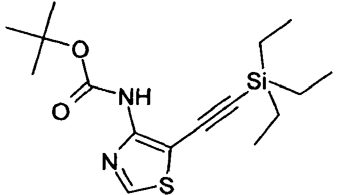 | |
| ¹H NMR (300 MHz, DMSO-d₆): δ [ppm] 0.62 (dt, *J*_{H,H} = 8.3 Hz, *J*_{H,H} = 4.2 Hz, 6H), 0.98 (t, *J*_{H,H} = 7.8 Hz, 9H), 1.43 (s, 9H), 8.91 (s, 1H), 9.39 (s, 1H); | | |
| ¹³C NMR (75 MHz, DMSO-d₆): δ [ppm] 3.71, 7.28, 27.91, 79.17, 94.95, 101.41, 107.65, 151.40, 152.28, 152.37 | | |
| "A18" | [5-(3-Amino-phenylethinyl)-thiazol-4-yl]-carbaminsäure-tert.-butylester | 166-167; ESI-MS: Rₜ: 1.99 min; m/z (%): 316 (100, [M+H]⁺) |
| | 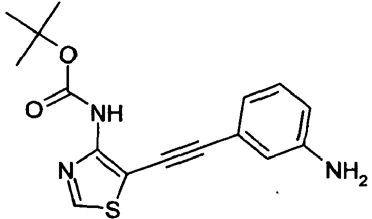 | |
| ¹H NMR (400 MHz, DMSO-d₆): δ [ppm] 1.45 (s, 9H), 5.27 (s, 2H), 6.57 - 6.71 (m, 3H), 7.05 (t, *J*_{H,H} = 7.8 Hz, 1H), 8.95 (s, 1H), 9.50 (s, 1H). | | |
| EI-MS: m/z (%): 215 (100, [M-BOC]⁺), 315 (20, [M]⁺); | | |
| ¹³C NMR (100 MHz, DMSO-d₆): δ [ppm] 27.45, 77.09, 78.79, 98.37, 106.73, 114.36, 115.24, 117.99, 121.57, 128.68, 148.30, 150.13, 151.64, 151.95 | | |
| "A19" | [5-(3-Benzylamino-phenylethinyl)-thiazol-4-yl]-carbaminsäure-tert.-butylester | 126-129; ESI-MS: Rₜ: 2.61 min; m/z (%): 250 (100, [M-tBu+2H]⁺), 406 (20, [M+H]⁺) |
| | 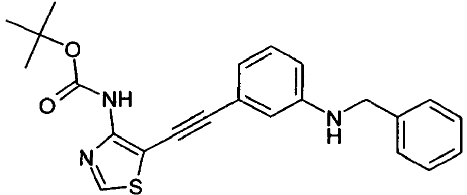 | |
| ¹H NMR (400 MHz, DMSO-d₆): δ [ppm] 1.43 (s, 9H), 4.28 (d, *J*_{H,H} = 6.0 Hz, 2H), 6.47 (t, *J*_{H,H} = 6.1 Hz, 1H), 6.61 - 6.70 (m, 3H), 7.04 - 7.13 (m, 1H), 7.18 - 7.31 (m, 1H), 7.31 - 7.45 (m, 4H), 8.94 (s, 1H), 9.49 (s, 1H); | | |
| ¹³C NMR (100 MHz, DMSO-d₆): δ [ppm] 27.44, 45.61, 77.28, 78.77, 98.37, 106.61, 113.10, 113.23, 118.24, 121.67, 126.17, 126.54, 127.80, 128.67, 139.24, 148.18, 150.16, 151.70, 151.91 | | |
| "A20" | [5-(2-Benzylamino-pyridin-4-ylethinyl)-thiazol-4-yl]-carbaminsäure-tert.-butyl ester | 185-189; EI-MS: m/z (%): 57 (65, [CCH₃]^{+•}), 105 (100, [C₇H₇N]^{+•}), 306 (55, [M-OCOCCH₃]^{+•}), 406 (40, [M]^{+•}) |
| | 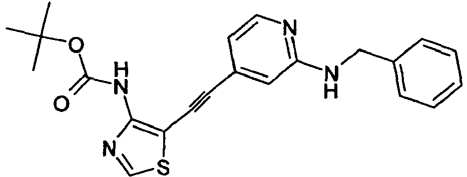 | |
| ¹H NMR (400 MHz, DMSO-d₆): δ [ppm] 1.44 (s, 9H), 4.48 (d, *J*_{H,H} = 6.0 Hz, 2H), 6.49 - 6.59 (m, 2H), 7.20 - 7.29 (m, 2H), 7.31 (d, *J*_{H,H} = 4.4 Hz, 4H), 7.98 (d, *J*_{H,H} = 5.2 Hz, 1H), 9.01 (s, 1H), 9.68 (s, 1H); | | |
| ¹³C NMR (100 MHz, DMSO-d₆): δ [ppm] 27.43, 43.50, 78.97, 80.87, 95.75, 104.86, 108.56, 112.22, 126.05, 126.59, 127.70, 129.54, 139.70, 147.59, 151.21, 151.73, 152.89, 158.15 | | |
| "A21" | [5-(1H-Pyrrolo[2,3-b]pyridin-5-ylethinyl)-thiazol-4-yl]-carbaminsäure-tert.-butyl ester | >158 (decomposition); ESI-MS: Rₜ: 2.04 min; m/z (%): 341 (100, [M+H]⁺) |
| | 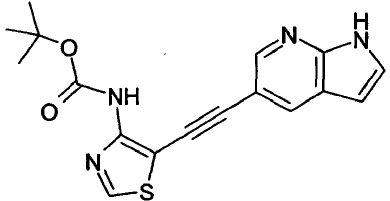 | |
| ¹H NMR (300 MHz, DMSO-d₆): δ [ppm] 1.47 (s, 9H), 6.50 (d, *J*_{H,H} = 3.4 Hz, 1H), 7.41 - 7.67 (m, 2H), 8.11 (d, *J*_{H,H} = 2.0 Hz, 1H), 8.34 (d, *J*_{H,H} = 1.9 Hz, 1H), 8.96 (s, 1H), 9.54 (s, 1H), 11.93 (s, 1H); | | |
| ¹³C NMR (75 MHz, DMSO-d₆): δ [ppm] 27.95, 79.30, 97.06, 100.28, 106.89, 110.16, 119.11, 127.66, 130.63, 144.87, 146.02, 147.54, 150.75, 152.10, 152.43 | | |
| "A22" | 5-(1H-Pyrrolo[2,3-b]pyridin-5-ylethinyl)-thiazol-4-ylamin | ESI-MS: m/z (%): 241 (100, [M+H]⁺) |
| | 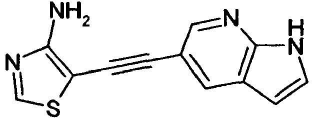 | |
| "A23" | Referenzbeispiel: (2-Pyridin-4-yl-thiazol-4-yl)-carbaminsäure-tert.-butylester | 192-193; EI-MS: m/z (%): 57 (100, [CCH₃]^{+•}), 177 (80, [M-BOC]^{+•}), 277 (10, [M]^{+•}). ESI-MS: m/z (%): 278 (100, [M+H]⁺) |
| | 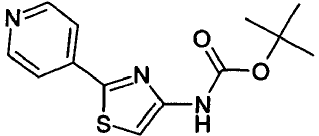 | |
| ¹H NMR (300 MHz, DMSO-d₆): δ [ppm] 1.49 (s, 9H), 7.45 (s, 1H), 7.81 (dd, *J*_{H,H} = 4.5 Hz, *J*_{H,H} = 1.6 Hz, 2H), 8.70 (dd, *J*_{H,H} = 4.5 Hz, *J*_{H,H} = 1.6 Hz, 2H), 10.38 (s, 1H); | | |
| ¹³C NMR (75 MHz, DMSO-d₆): δ [ppm] 27.98, 79.64, 101.56, 119.47, 139.30, 149.91, 150.68, 152.76, 161.16 | | |
| "A24" | *tert*.-Butyl-N-(2-pyridin-4-yl-5-iodo-1,3-thiazol-4-yl)carbamat | 197; EI-MS: m/z (%): 57 (100, [CCH₃]^{+•}), 303 (80, [M-BOC]^{+•}), 403 (10, [M]^{+•}) |
| | 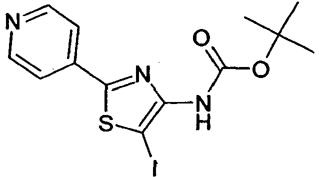 | |
| ¹H NMR (400 MHz, DMSO-d₆): δ [ppm] 1.46 (s, 9H), 7.80 (dd, *J*_{H,H} = 4.5 Hz, *J*_{H,H} = 1.6 Hz, 2H), 8.72 (dd, *J*_{H,H} = 5.9 Hz, *J*_{H,H} = 1.5 Hz, 2H), 9.27 (s, 1H); | | |
| ¹³C NMR (100 MHz, DMSO-d₆): δ [ppm] 27.52, 73.10, 78.88, 118.88, 138.16, 150.32, 152.32, 165.41 | | |
| "A25" | [5-(3-Morpholin-4-yl-prop-1-inyl)-2-pyridin-4-yl-thiazol-4-yl]-carbaminsäure-tert.-butylester | 94-100; ESI-MS: Rₜ: 1.37 min; m/z (%): 401 (100, [M+H]⁺) |
| | 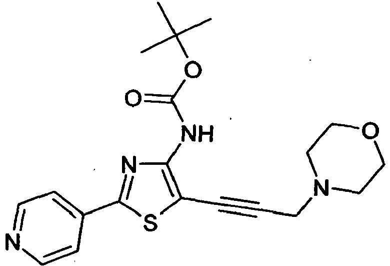 | |
| "A26" | {5-[3-(1,3-Dioxo-1,3-dihydro-isoindol-2-yl)-prop-1-inyl]-thiazol-4-yl}-carbaminsäure-tert.-butylester | 132-141; ESI-MS: Rₜ: 2.19 min; m/z (%): 328 (100, [M-tBu+2H]⁺), 384 (15, [M+H]⁺) |
| | 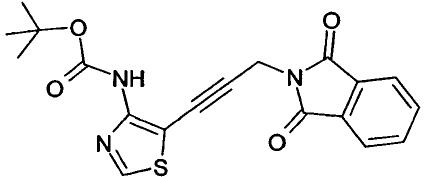 | |
| ¹H NMR (400 MHz, DMSO-d₆): δ [ppm] 1.33 (s, 9H), 4.65 (s, 2H), 7.84 - 7.96 (m, 4H), 8.91 (s, 1H), 9.44 (s, 1H); | | |
| ¹³C NMR (100 MHz, DMSO-d₆): δ [ppm] 27.16, 27.29, 71.37, 78.69, 92.47, 105.53, 122.81, 130.95, 134.18, 150.82, 151.78, 151.97, 166.11 | | |
| "A27" | 3-(4-Amino-thiazol-5-ylethinyl)-phenol | ESI-MS: Rₜ: 1.83 min; m/z (%): 217 (100, [M+H]⁺) |
| | 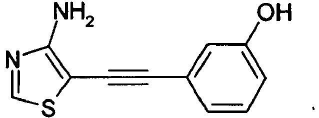 | |
| "A28" | [2-(2-Amino-pyridin-3-yl)-5-(4-fluor-phenylethinyl)-thiazol-4-yl]-carbaminsäure-tert.-butylester | ESI-MS: Rₜ: 3.32 min; m/z (%): 411 (100, [M+H]⁺) |
| | 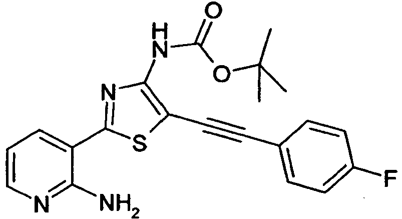 | |
| ¹H NMR (400 MHz, DMSO-d₆): δ [ppm] 1.48 (s, 9H), 6.67 (dd, *J*_{H,H}= 7.7 Hz, *J*_{H,H} = 4.7 Hz, 1H), 7.35 - 7.25 (m, 2H), 7.58 - 7.67 (m, 4H), 7.90 (dd, *J* = 7.7 Hz, *J*_{H,H} = 1.7 Hz, 1H), 8.13 (dd, *J*_{H,H} = 4.7 Hz, *J*_{H,H} = 1.7 Hz, 1H), 9.89 (s, 1H); | | |
| ¹³C NMR (100 MHz, DMSO-d₆): δ [ppm] 27.43, 78.41, 79.32, 97.25, 100.85, 108.17, 111.68, 115.55 (d, ²*J*_{C,F} = 23 Hz), 118.02, 132.90 (d, ³*J*_{C,F} = 8 Hz), 135.56, 149.33, 150.59, 151.41, 155.11, 161.62 (d, ¹*J*_{C,F} = 247 Hz), 162.98 | | |
| "A30" | 3-[5-(4-Fluor-phenyl)-4H-pyrrolo[2,3-d]thiazol-2-yl]-pyridin-2-ylamin | 300-301; ESI-MS: Rₜ: 1.95 min; m/z (%): 311 (100, [M+H]⁺) |
| | 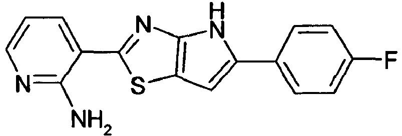 | |
| "A31" | 3-[5-(4-Fluor-phenyl)-4H-pyrrolo[2,3-d]thiazol-2-yl]-5-(1-piperidin-4-yl-1H-pyrazol-4-yl)-pyridin-2-ylamin | >230° (Zersetzung) ESI-MS: Rₜ: 1.70 min; ^{$} m/z (%): 460 (100, [M+H]⁺); |
| | 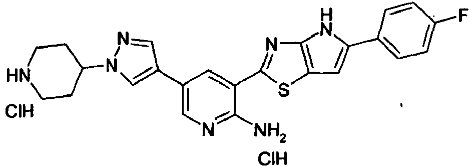 | ESI-HRMS: Rₜ: 4.55 min, m/z [M+H]⁺; berechnet für C₂₄H₂₃FN₇S: 460.1719; gefunden: 460.1704 |
| ¹H NMR (400 MHz, DMSO-d₆): δ [ppm] 2.30-2.13 (m, 4H), 3.17-3.08 (m, 2H), 3.45-3.40 (m, 2H), 4.57-4.50 (m, 1H), 7.02 (d, *J* = 1.7 Hz, 1H), 7.32 (d, *J* = 8.9 Hz, 2H),7.86 (d, *J* = 3.4 Hz, 2H), 8.06 (s, 1H), 8.33 (s, 1H), 8.39 (d, *J* = 1.9 Hz, 1H), 8.41 (s, 1H), 12.58 (s, 1H); | | |
| ¹³C NMR (100 MHz, DMSO-d₆): δ [ppm] = 28.03, 41.33, 54.87, 94.87, 96.83, 115.3 (d, *²J*_{C,F} = 22 Hz), 115.86, 115.98, 117.9, 125.6, 125.9 (d, ³*J*_{C,F} = 7 Hz), 128.0, 135.8, 135.6, 136.5, 148.6, 150.9, 157.9, 159.6, 160.9 (d, ¹*J*_{C,F} = 250 Hz) | | |
| "A32" | 6-(4,4-Dimethyl-pent-2-inyl)-4H-pyrrolo[2,3-d]thiazol | 116-118; ESI-MS: Rₜ: 2.40 min; ^{$} m/z (%): 219 (100, [M+H]⁺); |
| | 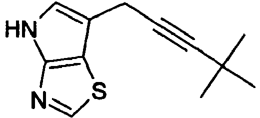 | ESI-HRMS: Rₜ: 6.19 min, m/z [M+H]⁺; m/z berechnet für C₁₂H₁₅N₂S: 219.0956; gefunden: 219.0951 |
| ¹H NMR (300 MHz, DMSO-d₆): δ [ppm] = 1.21 (d, *J* = 8.6 Hz, 9H), 2.51 | | |
| (dd, *J* = 3.6 Hz, 1.8 Hz, 1H), 3.52 (d, *J* = 0.7 Hz, 2H),), 6.94 (dd, *J* = 2.3 Hz, 1.2 Hz, 1H), 8.72 (d, *J* = 1.3 Hz, 1H), 11.58 (s, 1H); | | |
| ¹³C NMR (75 MHz, DMSO-d₆): δ [ppm] = 16.27, 26.99, 31.03, 76.00, 89.60, 109.98, 112.08, 119.57, 150.62, 151.57 | | |
| "A33" | 2-tert.-Butyl-3-(4H-pyrrolo[2,3-d]thiazol-6-ylmethyl)-1H-indol-5-carbonsäure-methylester | >167 (Zersetzung); ESI-MS: Rₜ: 2.42 min; ^{$} m/z (%): 368 (100, [M+H]⁺); |
| | 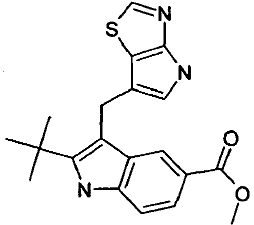 | ESI-HRMS: Rₜ: 6.08 min, m/z [M+H]⁺; m/z berechnet für C₂₀H₂₂N₃O₂S: 368.1432; gefunden: 368.1425 |
| ¹H NMR (300 MHz, DMSO-d₆): δ [ppm] = 1.46 (s, 9H), 3.83 (s, 3H), 4.27 (s, 2H), 6.93 (d, *J* = 1.0 Hz, 2H), 7.45 (d, *J* = 8.5 Hz, 1H), 7.72 (dd, *J* = 8.5 Hz, *J* = 1.6, 1H), 8.07 (d, *J* = 1.4 Hz, 1H), 8.54 (d, *J* = 1.3 Hz, 1H), 11.01 (s, 1H), 11.53 (s, 2H); | | |
| ¹³C NMR (75 MHz, DMSO-d₆): δ [ppm] = 21.72, 30.39, 33.26, 51.41, 107.98, 110.48, 114.55, 119.34, 119.44, 120.26, 120.79, 121.46, 121.62, 125.43, 129.12, 137.42, 144.72, 150.12 | | |
| "A34" | 2-tert.-Butyl-3-(4H-pyrrolo[2,3-d]thiazol-6-ylmethyl)-1H-pyrrolo[2,3-b]pyridin | 220-225; ESI-MS: Rₜ: 1.77 min; ^{$} m/z (%): 311 (100, [M⁺H]⁺); |
| | 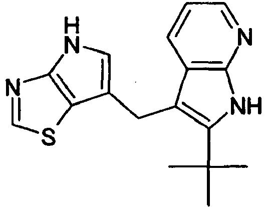 | |
| | | EI-HRMS: m/z berechnet für C₁₇H₁₈N₄S: 310.1252; gefunden: 310.1201 |
| ¹H NMR (300 MHz, DMSO-d₆): δ [ppm] = 1.41 (s, 9H), 4.16 (s, 2H), 6.97 - 6.84 (m, 2H), 7.67 (dd, *J* = 7.8 Hz, *J* = 1.2 Hz, 1H), | | |
| 8.10 (s, 1H), 8.50 (d, *J* = 1.3 Hz, 1H), 11.10 (s, 1H), 11.46 (s, 1H); | | |
| ¹³C NMR (75 MHz, DMSO-d₆): δ [ppm] = 21.79, 30.44, 33.55, 39.53, 105.35, 112.15, 114.57, 114.66, 119.39, 121.73, 125.50, 141.45, 143.57, 147.63, 150.03, 151.37 | | |
| "A35" | 6-(3,5-Difluor-benzyl)-4H-pyrrolo-[2,3-d]thiazol | ESI-MS: Rₜ: 4.49 min; ^{§} [M]⁺ 251 |
| | 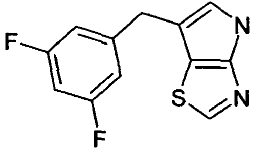 | |
| ¹H NMR (400 MHz, CDCl₃) δ [ppm] 8.61 (br. s, 1H), 8.48 (d, *J* = 1.3, 1H), 6.89 (d, *J* = 1.0, 1H), 6.83 - 6.74 (m, 2H), 6.67 (m, 1H), 3.96 (s, 2H) | | |
| "A36" | 6-(4-Methoxy-benzyl)-4H-pyrrolo-[2,3-d]thiazol | ESI-MS: Rₜ: 4.123 min; ^{§} [M]⁺ 245 |
| | 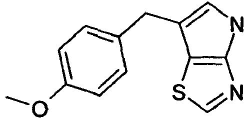 | |
| ¹H NMR (400 MHz, CDCl₃) δ [ppm] 8.66 (br. s, 1H), 8.50 (s, 1H), 7.19 (d, *J* = 8.5, 2H), 6.87 (d, *J* = 8.5, 2H), 3.91 (s, 2H), 3.81 (s, 3H) | | |
| "A37" | 6-(4-Brom-benzyl)-4H-pyrrolo[2,3-d]-thiazol | ESI-MS: Rₜ: 4.793 min; ^{§} [M]⁺ 293 |
| | 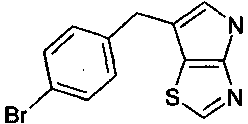 | |
| ¹H NMR (400 MHz, CDCl₃) δ [ppm] 8.61 (br. s, 1H), 8.47 (s, 1H), 7.46 - 7.41 (m, 2H), 7.15 (d, *J* = 8.4, 2H), 6.86 (s, 1H), 3.94 (s, 2H) | | |
| "A38" | 6-(4-Isopropyl-benzyl)-4H-pyrrolo-[2,3-d]thiazol | ESI-MS: Rₜ: 5.225 min; ^{§} [M]⁺ 257 |
| | 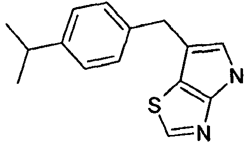 | |
| ¹H NMR (400 MHz, CDCl₃) δ [ppm] 9.10 (br. s, 1H), 8.52 (d, *J* = 1.2, 1H), 7.27 (s, 5H), 7.19 (s, 4H), 6.90 (d, *J* = 1.0, 1H), 3.95 (s, 2H), 2.90 (quint, *J* = 6.9, 1H), 1.25 (d, *J* = 6.9, 6H) | | |
| "A39" | 6-(4-Brom-2-fluor-benzyl)-4H-pyrrolo-[2,3-d]thiazol | ESI-MS: Rₜ: 4.837 min; ^{§} [M]⁺ 311 |
| | 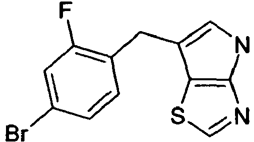 | |
| ¹H NMR (400 MHz, CDCl₃) δ [ppm] 8.56 (br. s, 1H), 8.47 (d, *J* = 1.3, 1H), 7.24 (t, *J* = 8.8, 2H), 7.11 (t, *J* = 7.9, 1H), 6.88 (s, 1H), 3.96 (s, 2H) | | |
| "A40" | 6-(4-tert.-Butyl-benzyl)-4H-pyrrolo-[2,3-d]thiazol | ESI-MS: Rₜ: 5.439 min; ^{§} [M]⁺ 271.3 |
| | 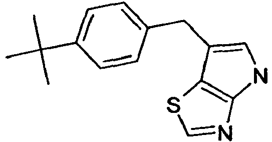 | |
| ¹H NMR (400 MHz, CDCl₃) δ [ppm] 8.59 (br. s, 1H), 8.46 (s, 1H), 7.39 - 7.31 (m, 2H), 7.21 (d, *J* = 8.3, 2H), 6.85 (d, *J* = 1.1, 1H), 3.95 (s, 2H), 1.32 (s, 9H) | | |
| "A41" | 6-(2-Chlor-benzyl)-4H-pyrrolo-[2,3-d]thiazol | ESI-MS: Rₜ: 4.581 min; ^{§} [M]⁺ 249 |
| | 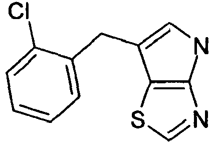 | |
| ¹H NMR (400 MHz, CDCl₃) δ [ppm] 9.11 (br. s, 1H), 8.51 (s, 1H), 7.40 (dd, *J* = 7.3, 2.0, 1H), 7.29 (dd, *J* = 7.3, 2.3, 1H), 7.25 - 7.21 (m, 1H), 7.21 - 7.17 (m, 1H), 6.92 (d, *J* = 1.1, 1H), 4.11 (s, 2H) | | |
| "A42" | 6-(4-Fluor-benzyl)-4H-pyrrolo[2,3-d]-thiazol | ESI-MS: Rₜ: 4.37 min; ^{§} [M]⁺ 233 |
| | 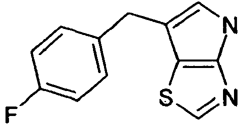 | |
| ¹H NMR (400 MHz, CDCl₃) δ [ppm] 8.65 (br. s, 1H), 8.46 (s, 1H), 7.25 - 7.20 (m, 2H), 7.04 - 6.96 (m, 2H), 6.85 (d, *J* = 1.0, 1H), 3.96 (s, 2H) | | |
| "A43" | 6-(3-Fluor-benzyl)-4H-pyrrolo-[2,3-d]thiazol | ESI-MS: Rₜ: 4.367 min; ^{§} [M]⁺ 233 |
| | 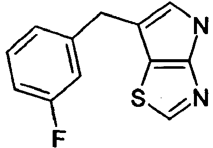 | |
| ¹H NMR (400 MHz, CDCl₃) δ [ppm] 8.80 (br. s, 1H), 8.49 (s, 1H), 7.29 (dd, *J* = 7.0, 5.2, 1H), 7.06 (d, *J* = 7.5, 1H), 6.99 - 6.90 (m, 2H), 6.89 (d, *J* = 1.0, 1H), 3.98 (s,2H) | | |
| "A44" | 6-(2,3-Difluor-benzyl)-4H-pyrrolo-[2,3-d]thiazol | ESI-MS: RT:4.454 min; ^{§} [M]⁺ 251 |
| | 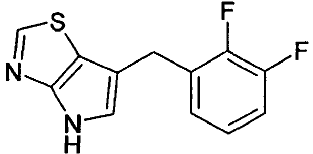 | |
| ¹H NMR (400 MHz, CDCl₃) δ [ppm] 8.95 (s, 1H), 8.51 (s, 1H), 7.04 (m, 3H), 6.93 (s, 1H), 4.04 (s, 2H) | | |
| "A45" | 6-(2,4-Difluor-benzyl)-4H-pyrrolo-[2,3-d]thiazol | ESI-MS: RT: 4.488 min;^{§} [M]⁺ 251 |
| | 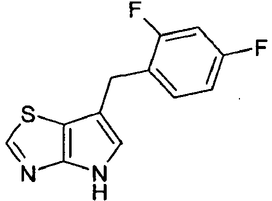 | |
| ¹H NMR (400 MHz, CDCl₃) δ [ppm] 8.92 (s, 1H), 8.49 (s, 1H), 7.24 - 7.16 (m, 1H), 6.87 (m, 1H), 6.82 (m, 1H), 3.96 (s, 2H) | | |
| "A46" | 6-(2,5-Difluor-benzyl)-4H-pyrrolo-[2,3-d]thiazol | ESI-MS: RT: 4.438 min; ^{§} [M]⁺ 251 |
| | 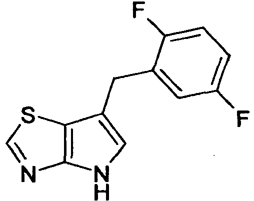 | |
| ¹H NMR (400 MHz, CDCl₃) δ [ppm] 9.54 (s, 1H), 8.63 (s, 1H), 7.07 - 7.00 (m, 1H), 6.99 (s, 1H), 6.95 - 6.88 (m, 2H), 3.99 (s, 2H) | | |
| "A47" | 6-(3,4-Difluor-benzyl)-4H-pyrrolo-[2,3-d]thiazol | ESI-MS: RT:4.508 min; ^{§} [M]⁺ 251 |
| | 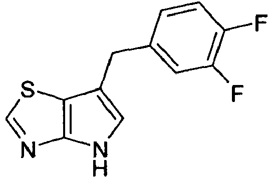 | |
| ¹H NMR (400 MHz, CDCl₃) δ [ppm] 8.93 (s, 1H), 8.52 (s, 1H), 7.13 - 7.06 (m, 1H), 7.07 - 7.02 (m, 1H), 6.99 (s, 1H), 6.91 (s, 1H), 3.95 (s, 2H) | | |
| "A48" | 6-(6-Chlor-pyridin-3-ylmethyl)-4H-pyrrolo-[2,3-d]thiazol | ESI-MS: RT: 3.549 min; ^{§} [M]⁺ 250 |
| | 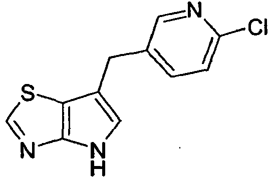 | |
| ¹H NMR (400 MHz, CDCl₃) δ [ppm] 9.17 (s, 1H), 8.55 (s, 1H), 8.36 (d, *J* = 2.3, 1H), 7.55 (dd, *J* = 8.2, 2.5, 1H), 7.29 (d, *J* = 8.4, 1H), 6.92 (s, 1H), 3.99 (s, 2H) | | |
| "A49" | 6-(3-Chlor-benzyl)-4H-pyrrolo-[2,3-d]thiazol | ESI-MS: RT: 4.679 min;^{§} [M]⁺ 249 |
| | 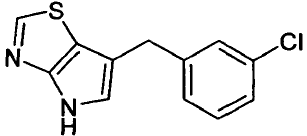 | |
| ¹H NMR (400 MHz, CDCl₃) δ [ppm] 8.56 (br. s, 1H), 8.46 (d, *J* = 1.4, 1H), 7.27 - 7.25 (m, 1H), 7.23 (m, 2H), 7.17 (m, 1H), 6.87 (m, 1H), 3.96 (s, 2H) | | |
| "A50" | 6-(3,4-Dichlor-benzyl)-4H-pyrrolo-[2,3-d]thiazol | ESI-MS: RT: 5.029 min; ^{§} [M]⁺ 283 |
| | 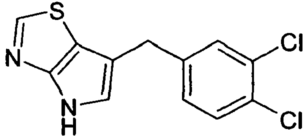 | |
| ¹H NMR (400 MHz, CDCl₃) δ [ppm] 9.06 (br. s, 1H), 8.52 (s, 1H), 7.37 (dd, *J* = 10.3, 5.1, 2H), 7.11 (dd, *J* = 8.2, 2.0, 1H), 6.89 (s, 1H), 3.94 (s, 2H) | | |

6-Benzyl-2-pyridin-3-yl-4H-pyrrolo[2,3-d]thiazol ("A51")
   ¹H NMR (400 MHz, DMSO-d₆) δ [ppm] 11.77 (s, 1 H), 9.02 (d, *J* = 2.2, 1H), 8.55 (dd, *J* = 4.8, 1.5, 1 H), 8.20 - 8.09 (m, 1 H), 7.46 (dd, *J* = 8.0, 4.8, 1 H), 7.31 (m, 1H), 7.27 (m, 3H), 7.20 (m, 1 H), 7.09 (d, *J* = 2.4, 1 H), 3.92 (s, 2H);
6-Benzyl-2-(1-methyl-1H-pyrazol-4-yl)-4H-pyrrolo[2,3-d]thiazol ("A52")
   ¹H NMR (400 MHz, DMSO-d₆) δ [ppm] 11.46 (s, 1H), 8.18 (s, 1H), 7.78 (d, *J* = 0.7, 1 H), 7.31 - 7.22 (m, 4H), 7.21 - 7.15 (m, 1 H), 6.90 (d, *J* = 2.4, 1 H), 3.87 (s, 2H), 3.84 (s, 3H);
5-(6-Benzyl-4H-pyrrolo[2,3-d]thiazol-2-yl)-pyridin-2-ylamin ("A53")
   ¹H NMR (400 MHz, DMSO-d₆) δ [ppm] 11.49 (d, *J* = 2.0, 1 H), 8.37 (d, *J* = 2.2, 1H), 7.75 (dd, *J* = 8.7, 2.5, 1 H), 7.28 (m, 4H), 7.18 (m, 1 H), 6.91 (d, J = 2.4, 1 H), 6.47 (dd, *J* = 8.8, 0.4, 1H), 6.41 (s, 2H), 3.87 (s, 2H);
6-Prop-2-inyl-4H-pyrrolo[2,3-d]thiazol ("A54")
   ¹H NMR (400 MHz, CDCl₃) δ [ppm] 8.63 (s, 1 H), 8.53 (d, *J* = 1.1, 1 H), 6.98 (d, *J* = 1.2, 1 H), 3.62 (d, *J* = 2.0, 3H), 2.19 (t, *J* = 2.6, 1 H);
3-(6-Benzyl-4H-pyrrolo[2,3-d]thiazol-2-yl)-pyridin-2-ylamin ("A55"),
6-Benzyl-2-(1 H-pyrazol-4-yl)-4H-pyrrolo[2,3-d]thiazol ("A56"),
6-Benzyl-2-(5-brom-pyridin-3-yl)-4H-pyrrolo[2,3-d]thiazol ("A57"),
6-Benzyl-2-pyrimidin-5-yl-4H-pyrrolo[2,3-d]thiazol ("A58"),
Morpholin-4-yl-[3-(4H-pyrrolo[2,3-d]thiazol-6-ylmethyl)-1 H-indol-5-yl]-methanon ("A59"),
3-(4H-pyrrolo[2,3-d]thiazol-6-ylmethyl)-1H-indol-5-carbonsäure-(2-methoxy-ethyl)-amid ("A60"),
3-(4H-pyrrolo[2,3-d]thiazol-6-ylmethyl)-1H-indol-5-carbonsäure-4-fluor-benzylamid ("A61"),
N-(2-Amino-ethyl)-2-[4-(4H-pyrrolo[2,3-d]thiazol-6-ylmethyl)-[1,2,3]triazol-1-yl]-acetamid ("A62"),
1-Piperazin-1-yl-2-[4-(4H-pyrrolo[2,3-d]thiazol-6-ylmethyl)-[1,2,3]triazol-1-yl]-ethanon ("A63"),
N-Piperidin-4-yl-2-[4-(4H-pyrrolo[2,3-d]thiazol-6-ylmethyl)-[1,2,3]triazol-1-yl]-acetamid ("A64"),
6-(1-Pyridin-3-ylmethyl-1H-[1,2,3]triazol-4-ylmethyl) 4H-pyrrolo[2,3-d]thiazol ("A65"),
   ¹H NMR (400 MHz, CDCl₃) δ [ppm] 8.61 (dd, *J* = 4.8, 1.5, 1 H), 8.58 (d, *J* = 1.9, 2H), 8.47 (d, *J* = 1.3, 1H), 7.58 (d, *J* = 8.4, 1 H), 7.31 (dd, *J* = 7.8, 4.8, 1H), 7.25 (s, 1 H), 6.93 (d, *J* = 1.1, 1 H), 5.53 (s, 2H), 4.11 (s, 2H);
6-(1-Pyridin-4-ylmethyl-1H-[1,2,3]triazol-4-ylmethyl) 4H-pyrrolo[2,3-d]thiazol ("A66")
   ¹H NMR (400 MHz, CDCl₃) δ [ppm] 8.61 (dd, *J* = 4.4, 1.6, 3H), 8.48 (d, *J* = 1.3, 1H), 7.09 (dd, *J* = 4.5, 1.5, 2H), 6.96 (d, *J* = 1.1, 1H), 5.53 (s, 2H), 4.14 (s, 2H);
6-Benzyl-2-(5-(1-methyl-1H-pyrazol-4-yl)-pyridin-3-yl)-4H-pyrrolo[2,3-d]thiazol ("A67"),
6-Benzyl-2-(5-(1 H-pyrazol-4-yl)-pyridin-3-yl)-4H-pyrrolo[2,3-d]thiazol ("A68"),
6-Benzyl-2-(5-(4-fluor-phenyl)-pyridin-3-yl)-4H-pyrrolo[2,3-d]thiazol ("A69"),
6-(5-Methansulfonyl-thiophen-2ylmethyl)-4H-pyrrolo[2,3-d]thiazol ("A70"),
6-[1-(2-Morpholin-4-yl-ethyl)-1H-[1,2,3]triazol-4-ylmethyl) 4H-pyrrolo[2,3-d]thiazol ("A71")
   ¹H NMR (400 MHz, CDCl₃) δ [ppm] 8.57 (s, 1 H), 8.49 (d, *J* = 1.3, 1 H), 7.42 (s, 1H), 6.96 (d, *J* = 1.1, 1H), 4.43 (t, *J* = 6.3, 2H), 4.12 (s, 2H), 3.71 - 3.60 (m, 5H), 2.80 (t, *J* = 6.3, 2H), 2.53 - 2.42 (m, 4H);
6-Benzyl-2-{5-[1-((E)-3-phenyl-allyl)-1H-pyrazol-4-yl]-pyridin-3-yl}-4H-pyrrolo[2,3-d]thiazol ("A72")
   ¹H NMR (400 MHz, DMSO-d₆) δ [ppm] 11.47 (s, 1H), 8.27 (s, 1H), 7.85 (d, *J* = 0.6, 1 H), 7.45 (d, *J* = 7.1, 2H), 7.36 - 7.22 (m, 7H), 7.21 - 7.15 (m, 1H), 6.91 (d, *J* = 2.4, 1 H), 6.59 (d, *J* = 16.0, 1 H), 6.51 (t, *J* = 6.1, 1 H), 4.92 (d, *J* = 5.9, 2H), 3.87 (s, 2H).

### Ergebnisse der Rezeptorbindungstests

Die hier gezeigten Verbindungen sind bei einer Konzentration von 1 µM gegen 102 Kinasen getestet worden. Wie die Gegenüberstellung zeigt, ist sowohl mit den Verbindungen der Formel la als auch denen der Formel Ib ein hohes Maß an Potenz und Selektivität verknüpft, so dass sie sich für den Einsatz in der targetierten Tumortherapie eignen.

Die angegebenen % Werte sind ein Maß für die verbliebene Restaktivität der Kinase, wenn diese unter den beschriebenen Bedingungen im Filterbindungsassay mit der Substanz behandelt wird. Man kann im Rahmen der Messgenauigkeit annehmen, dass ein %-Wert von etwa 50 einem IC₅₀ von 1µM entspricht. M.W. Karaman et al. beschreiben in Nature Biotechnology 2008; 26 (1): 127 - 32 die quantitative Analyse der Kinase-Inhibitor-Selektivität.

Nachfolgend sind beispielhaft einige erfindungsgemäße Verbindungen aufgeführt und dazu die Kinasen genannt, die die Substanz zu ca. 50% oder intensiver inhibiert:
- 6-Benzyl-4H-pyrrolo[2,3-d]thiazol ("A7")
   Aurora A (33%); Rock II (36%); CDK2 (41%); CK2 (53%); NUAK1 (27%);
- 3-(4-Amino-thiazol-5-ylethinyl)-phenol ("A27")
   Nek2A (59%); NUAK1 (49%);
- {5-[3-(1,3-Dioxo-1,3-dihydro-isoindol-2-yl)-prop-1-inyl]-thiazol-4-yl}-carbaminsäure-tert.-butylester ("A26")
   CDK2 (28%); NUAK1 (24%);
- [5-(3-Morpholin-4-yl-prop-1-inyl)-2-pyridin-4-yl-thiazol-4-yl]-carbaminsäure-tert.-butylester ("A25")
   HIPK1 (48%);
- 5-(1H-Pyrrolo[2,3-b]pyridin-5-ylethinyl)-thiazol-4-ylamin ("A22")
   Aurora A (39%); HIPK1 (36%); MARK2 (31%); MKK1 (43%); PDK1 (53%); GSK3b (9%); CHK1 (40%); CDK2 (52%); PRK2 (38%); MAPK8 (34%); MARK3 (15%); HIPK2 (44%); PAK4 (34%); VEGFR (11%); GCK 31%); NUAK1 (16%); MLK3 (23%).

Wenn man des Weiteren davon ausgeht, dass die Kinaseinhibitoren im betrachteten Konzentrationsbereich der Aktivitätsmessung sich normal verhalten, kann eine Regressionsgerade mit der Steigung 1 für diesen Betrachtungsbereich angenommen werden. Somit kann man unten angeführte IC₅₀-Werte für die Verbindungen für eine Vielzahl von Kinasen erhalten.
IC₅₀: 10 nM - 1 µM = A; 1 µM - 10 µM = B; 10 µM - 100 µM = C NA bedeutet hier, dass die Verbindung bei der Testkonzentration von 1 µM keinerlei inhibitorische Effekte auf die Kinase ausgeübt hat (no activity). Aus der Tabelle mit den aus den %-Inhibierungsdaten abgeleiteten IC₅₀-Werten lässt sich ersehen, dass mit den hier vorgestellten Verbindungen ein Höchstmaß an flexibler Kinase-Inhibierung gegeben ist.

| Kinase | "A26" | "A7" | "A27" | "A22" | "A25" | "A31" |
|---|---|---|---|---|---|---|
| MAP2K1 | B | B | NA | A | B | |
| SRC | C | B | B | C | B | |
| MAPK1 | C | NA | B | C | NA | |
| MAPK8 | C | B | B | B | NA | |
| MAPK14 | C | B | B | C | C | |
| MAPK11 | NA | B | B | NA | NA | |
| MAPK12 | C | NA | B | B | C | |
| MAPK13 | C | B | B | C | NA | |
| RPS6KA1 | NA | C | NA | NA | C | |
| MAPKAPK2 | C | NA | C | NA | NA | |
| RPS6KA5 | NA | B | C | C | C | |
| MAPKAPK5 | B | NA | C | C | NA | |
| PRKCA | B | C | NA | B | C | |
| PDPK1 | B | NA | NA | B | NA | |
| AKT1 | NA | NA | NA | C | NA | |
| SGK | B | NA | NA | B | C | |
| RPS6KB1 | NA | B | B | B | NA | |
| GSK3B | B | B | B | A | NA | |
| ROCK2 | NA | A | C | NA | C | |
| PRKAA1 | C | C | B | C | C | |
| CHEK1 | C | NA | C | A | C | |
| CSNK2A1 | C | NA | NA | NA | NA | |
| CSK | C | B | C | C | NA | |
| CDK2 | A | A | C | B | C | |
| CSNK1D | NA | B | B | C | NA | |
| NEK6 | C | C | C | NA | B | |
| NEK2 | B | NA | B | NA | B | |
| LCK | NA | C | B | NA | NA | |
| PRKACA | C | NA | B | B | NA | |
| PBK | C | NA | NA | B | NA | |
| RPS6KA3 | NA | B | B | C | NA | |
| IKBKB | NA | B | B | C | NA | |
| MYLK | NA | NA | B | NA | NA | |
| PKN2 | B | B | C | A | NA | |
| MKNK2 | C | NA | NA | NA | NA | |
| CAMK1 | NA | B | B | C | B | |
| PIM2 | NA | NA | B | C | B | |
| MAPK10 | NA | NA | B | NA | NA | |
| MAPKAPK3 | NA | NA | C | NA | C | |
| MAPK8 | B | NA | NA | A | NA | |
| MKNK1 | NA | NA | C | C | NA | |
| SRPK1 | NA | C | B | NA | NA | |
| AKT2 | NA | NA | B | NA | B | |
| AURKB | NA | B | C | C | NA | |
| CHEK2 | NA | NA | C | NA | NA | |
| EEF2K | C | C | B | C | NA | |
| MARK3 | B | B | C | A | NA | |
| STK3 | NA | B | B | C | NA | |
| PRKD2 | NA | B | B | NA | NA | |
| MAPK9 | NA | C | B | NA | NA | |
| DYRK3 | B | C | C | C | C | |
| HIPK2 | B | NA | NA | A | NA | |
| HIPK3 | C | B | B | B | C | |
| PAK4 | B | NA | NA | A | NA | |
| DYRK2 | NA | NA | B | NA | NA | |
| CAMKK2 | C | C | C | NA | NA | |
| PIM1 | C | C | B | B | NA | |
| PIM3 | NA | B | B | C | NA | |
| PAK6 | NA | NA | C | C | C | |
| PLK1 | NA | NA | C | B | NA | |
| BRSK2 | NA | NA | C | NA | NA | |
| MELK | NA | B | B | NA | NA | |
| PRKCZ | C | NA | NA | NA | C | |
| MAPK1 | C | C | NA | B | C | |
| FGFR1 | B | B | NA | B | NA | |
| INSRR | B | NA | NA | B | NA | |
| EPHA2 | NA | C | NA | NA | NA | |
| MST4 | C | NA | NA | B | NA | |
| SYK | NA | NA | C | C | NA | |
| YES1 | NA | NA | NA | B | NA | |
| IGF1R | NA | C | NA | B | NA | |
| KDR | NA | B | B | A | NA | |
| BTK | NA | C | C | C | C | |
| INSR | B | NA | C | C | NA | |
| EPHB3 | B | NA | B | C | NA | |
| TBK1 | NA | NA | NA | NA | C | A |
| IKBKE | C | C | NA | NA | B | |
| **NUAK1** | **A** | **A** | **A** | **A** | **C** | |
| MAP3K9 | B | B | B | B | NA | |
| MINK1 | C | B | B | B | C | |
| MAP4K2 | B | B | B | A | NA | |
| IRAK4 | C | NA | C | NA | C | |
| TTK | C | C | C | C | C | |
| DYRK1A | C | NA | C | NA | NA | |
| MAP3K11 | B | C | C | A | NA | |
| STK11 | C | C | NA | NA | NA | |
| ERBB4 | NA | C | B | NA | NA | |
| PAK2 | NA | B | NA | NA | NA | |
| BRSK1 | NA | B | B | B | NA | |
| AURKA | B | A | B | A | NA | |
| RIPK2 | C | C | B | B | NA | |
| HIPK1 | NA | NA | B | A | A | |
| MARK4 | NA | NA | B | C | C | |
| MARK2 | B | C | C | A | NA | |
| MET | NA | B | C | NA | NA | |
| TGFBR1 | C | C | B | C | B | |
| PDPK1 | C | NA | NA | B | NA | |

Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel 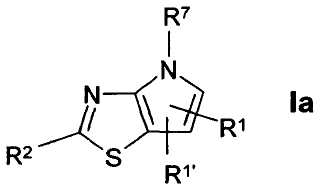 worin
R¹ H, (CH₂)ₙAr oder (CH₂)ₙHet,
R^{1'} H oder A,
R² H, A, Hal, (CH₂)ₙHet¹, (CH₂)ₙHet³, -C≡C-Ar, (CH₂)ₙAr², NHCONHAr, COA, COOH, COOA, COHet¹, COAr², CONH₂, CONHA, CONA₂, SO₂NH₂, SO₂NHA, S(O)ₘA, NHCOA, SO₂NHA, SO₂NA₂, SO₂NHHet¹, SO₂NHAr² oder 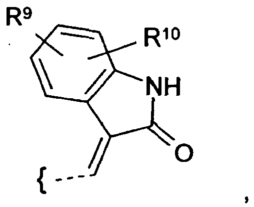
R⁷ H, A, Ar oder Het¹,
Ar unsubstituiertes oder ein-, zwei- oder dreifach durch Hal, A, OH, OA, CN, NO₂, SO₂A, COOH, COOA, NH₂, NHA, NA₂, NHCH₂Ar¹, CHO, COA, CHO, CONH₂, CONHA, CONA₂, Het, SO₂NH₂, SO₂NHA, S(O)ₘA und/oder NHCOA substituiertes Phenyl,
Het einen ein- oder zweikernigen aromatischen Heterocyclus mit 1 bis 4 N-, O- und/oder S-Atomen, der unsubstituiert oder ein-, zwei- oder dreifach durch Hal, A, NH₂, NHA, NA₂, COOH, COOA, CONH₂, CONHA, CONA₂, CONHAr, CONHHet⁴, S(O)ₘA und/oder NHCH₂Ar¹ substituiert sein kann,
Ar¹ unsubstituiertes oder ein-, zwei- oder dreifach durch Hal, A, OH und/oder OA substituiertes Phenyl,
Ar² einfach durch NHCONHHet⁴ substituiertes Phenyl,
Het¹ einen ein- oder zweikernigen aromatischen, ungesättigten oder gesättigten Heterocyclus mit 1 bis 4 N-, O- und/oder S-Atomen, der unsubstituiert oder ein-, zwei- oder dreifach durch Hal, A, COOA, NH₂, NHR⁸ und/oder (CH₂)ₙHet² substituiert sein kann,
R⁸ H, A, Ar¹ oder Het⁴,
R⁹, R¹⁰ jeweils unabhängig voneinander H oder Hal,
R¹¹ H oder A',
Het² einen ein- oder zweikernigen aromatischen Heterocyclus mit 1 bis 4 N-, O- und/oder S-Atomen, der unsubstituiert oder ein-, zwei- oder dreifach durch A, COOA und/oder NH₂ substituiert sein kann,
Het³ einen ein- oder zweikernigen aromatischen Heterocyclus mit 1 bis 4 N-, O- und/oder S-Atomen, der unsubstituiert oder ein-, zwei- oder dreifach durch NH₂ substituiert sein kann,
Het⁴ einen ein- oder zweikernigen aromatischen, ungesättigten oder gesättigten Heterocyclus mit 1 bis 4 N-, O- und/oder S-Atomen, der unsubstituiert oder ein-, zwei- oder dreifach durch Hal, A, Ar¹, NH₂, NHCH₂Ar¹ und/oder =O substituiert sein kann,
A unverzweigtes oder verzweigtes Alkyl mit 1-10 C-Atomen, worin 1-7 H-Atome durch F ersetzt sein können und/oder worin eine oder zwei nicht-benachbarte CH- und/oder CH₂-Gruppen durch O, NH, NA', S, SO, SO₂ und/oder durch CH=CH-Gruppen ersetzt sein können,
oder
cyclisches Alkyl mit 3-7 C-Atomen,
A' unverzweigtes oder verzweigtes Alkyl mit 1-4 C-Atomen,
Hal F, Cl, Br oder I,
m 0, 1 oder 2,
n 0, 1, 2, 3 oder 4,
bedeuten,
sowie ihre pharmazeutisch verwendbaren Salze, Tautomere und Stereoisomere, einschließlich deren Mischungen in allen Verhältnissen,
wobei 4*H*-Pyrrolo[2,3-d]thiazol, 2,5-dimethyl-4H-pyrrolo[2,3-d]thiazole, 6-methyl-4H-pyrrolo[2,3-d]thiazole, 2-methyl-4H-pyrrolo[2,3-d]thiazole, 5-methyl-4H-pyrrolo[2,3-d]thiazole, 4-methyl-4H-pyrrolo[2,3-d]thiazole, 2,4,5-trimethyl-4H-pyrrolo[2,3-d]thiazole, 2,4-dimethyl-4H-pyrrolo[2,3-d]thiazole, 4,5-dimethyl-4H-pyrrolo[2,3-d]thiazole, 4,6-dimethyl-4H-pyrrolo[2,3-d]thiazole, ausgeschlossen sind.

2. Verbindungen nach Anspruch 1, worin
R¹ H, (CH₂)ₙAr oder (CH₂)ₙHet,
R^{1'} H,
R² H, Hal, (CH₂)ₙHet¹, (CH₂)ₙHet³ oder -C=C-Ar,
R⁷ H,
Ar unsubstituiertes oder ein-, zwei- oder dreifach durch Hal, A, OH, OA, CN, NO₂, SO₂A, COOH, COOA, NH₂, NHA, NA₂, NHCH₂Ar¹, CHO, COA, CHO, CONH₂, CONHA, CONA₂, SO₂NH₂, SO₂NHA und/oder NHCOA substituiertes Phenyl,
Het einen ein- oder zweikernigen aromatischen Heterocyclus mit 1 bis 4 N-, O- und/oder S-Atomen, der unsubstituiert oder ein-, zwei- oder dreifach durch Hal, A, NH₂ und/oder NHCH₂Ar¹ substituiert sein kann,
Ar¹ unsubstituiertes oder ein-, zwei- oder dreifach durch Hal, A, OH und/oder OA substituiertes Phenyl,
Het¹ einen ein- oder zweikernigen aromatischen, ungesättigten oder gesättigten Heterocyclus mit 1 bis 4 N-, O- und/oder S-Atomen, der unsubstituiert oder ein-, zwei- oder dreifach durch Hal, A, NH₂ und/oder CH₂Het² substituiert sein kann,
Het² einen ein- oder zweikernigen aromatischen Heterocyclus mit 1 bis 4 N-, O- und/oder S-Atomen, der unsubstituiert oder ein-, zwei- oder dreifach durch A und/oder NH₂ substituiert sein kann,
Het³ einen ein- oder zweikernigen aromatischen Heterocyclus mit 1 bis 4 N-, O- und/oder S-Atomen, der unsubstituiert oder ein-, zwei- oder dreifach durch NH₂ substituiert sein kann,
Het⁴ einen ein- oder zweikernigen aromatischen, ungesättigten oder gesättigten Heterocyclus mit 1 bis 4 N-, O- und/oder S-Atomen, der unsubstituiert oder ein-, zwei- oder dreifach durch Hal, A, NH₂, NHCH₂Ar¹ und/oder =O substituiert sein kann,
A unverzweigtes oder verzweigtes Alkyl mit 1-10 C-Atomen, worin 1-7 H-Atome durch OH, F, Cl und/oder Br ersetzt sein können,
A' unverzweigtes oder verzweigtes Alkyl mit 1-4 C-Atomen,
Hal F, Cl, Br oder I,
n 0, 1, 2, 3 oder 4,
bedeuten,
sowie ihre pharmazeutisch verwendbaren Salze, Tautomere und Stereoisomere, einschließlich deren Mischungen in allen Verhältnissen.

3. Verbindungen nach Anspruch 1 oder 2, worin
Ar unsubstituiertes oder ein-, zwei- oder dreifach durch Hal, OH, OA, NH₂, NHA, NA₂ und/oder NHCH₂Ar¹ substituiertes Phenyl,
bedeutet,
sowie ihre pharmazeutisch verwendbaren Salze, Tautomere und Stereoisomere, einschließlich deren Mischungen in allen Verhältnissen.

4. Verbindungen nach Anspruch 1, 2 oder 3, worin
Het unsubstituiertes oder ein- oder zweifach durch NH₂ und/oder NHCH₂Ar¹ substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl,
bedeutet,
sowie ihre pharmazeutisch verwendbaren Salze, Tautomere und Stereoisomere, einschließlich deren Mischungen in allen Verhältnissen.

5. Verbindungen nach einem oder mehreren der Ansprüche 1-4,
worin
Ar¹ Phenyl bedeutet,
sowie ihre pharmazeutisch verwendbaren Salze, Tautomere und Stereoisomere, einschließlich deren Mischungen in allen Verhältnissen.

6. Verbindungen nach einem oder mehreren der Ansprüche 1-5,
worin
Het¹ unsubstituiertes oder ein- oder zweifach durch NH₂ und/oder CH₂Het² substituiertes Piperidinyl, Pyrrolidinyl, Morpholinyl, Piperazinyl, Imidazolidinyl, Oxazolidinyl, Tetrahydropyranyl, Thiazolyl, Thiophenyl, Furanyl, Pyrrolyl, Oxazolyl, Isoxazolyl, Oxadiazolyl, Pyrazolyl, Imidazolyl, Triazolyl, Thiadiazolyl, Pyridazinyl, Pyrazinyl, Pyridinyl oder Pyrimidinyl,
bedeutet,
sowie ihre pharmazeutisch verwendbaren Salze, Tautomere und Stereoisomere, einschließlich deren Mischungen in allen Verhältnissen.

7. Verbindungen nach einem oder mehreren der Ansprüche 1-6,
worin
Het² unsubstituiertes oder ein- oder zweifach durch A und/oder NH₂ substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl,
bedeutet,
sowie ihre pharmazeutisch verwendbaren Salze, Tautomere und Stereoisomere, einschließlich deren Mischungen in allen Verhältnissen.

8. Verbindungen nach einem oder mehreren der Ansprüche 1-7,
worin
Het³ unsubstituiertes oder ein- oder zweifach durch A und/oder NH₂ substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl,
bedeutet,
sowie ihre pharmazeutisch verwendbaren Salze, Tautomere und Stereoisomere, einschließlich deren Mischungen in allen Verhältnissen.

9. Verbindungen nach einem oder mehreren der Ansprüche 1-8,
worin
Het⁴ unsubstituiertes oder ein- oder zweifach durch NH₂, NHCH₂Ar¹ und/oder =O substituiertes Piperidinyl, Pyrrolidinyl, Morpholinyl, Piperazinyl, Imidazolidinyl, Oxazolidinyl, Tetrahydropyranyl, Dihydroisoindolyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl,
bedeutet,
sowie ihre pharmazeutisch verwendbaren Salze, Tautomere und Stereoisomere, einschließlich deren Mischungen in allen Verhältnissen.

10. Verbindungen nach Anspruch 1, ausgewählt aus der Gruppe
| Nr. | Name und/oder Struktur |
|---|---|
| "A1" | 5-(4-Fluorphenyl)-4*H*-pyrrolo[2,3-*d*][1,3]thiazol |
| | 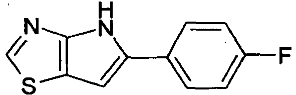 |
| "A2" | 5-Benzyl-4*H*-pyrrolo[2,3-*d*]thiazol |
| "A4" | 3-(4*H*-Pyrrolo[2,3-*d*]thiazol-5-yl)-phenylamin |
| | 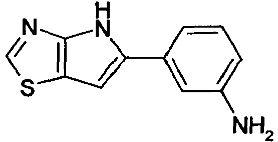 |
| "A5" | Benzyl-[4-(4*H*-pyrrolo[2,3-*d*]thiazol-5-yl)-pyridin-2-yl]-amin |
| | 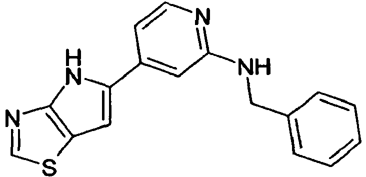 |
| "A6" | Benzyl-[3-(4*H*-pyrrolo[2,3-*d*]thiazol-5-yl)-phenyl]-amin |
| | 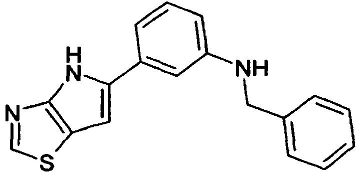 |
| "A7" | 6-Benzyl-4*H*-pyrrolo[2,3-*d*]thiazol |
| | 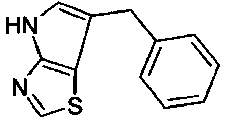 |
| "A8" | 6-Benzyl-2-pyridin-4-yl-4*H*-pyrrolo[2,3-*d*]thiazol |
| | 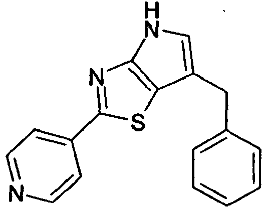 |
| "A9" | 4-(4*H*-Pyrrolo[2,3-*d*]thiazol-5-yl)-pyridin-2-ylamin |
| | 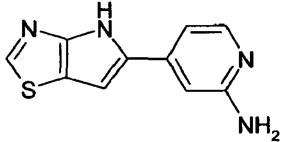 |
| "A10" | 2-Chlor-5-(4-fluor-phenyl)-4*H*-pyrrolo[2,3-*d*]thiazol |
| | 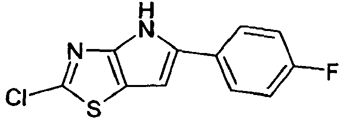 |
| "A11" | 5-(4-Fluor-phenyl)-2-(4-fluor-phenylethinyl)-4*H*-pyrrolo[2,3-*d*]thiazol |
| | 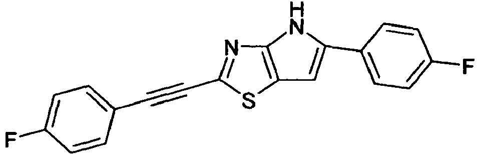 |
| "A12" | 5-(4-Fluor-phenyl)-2-morpholin-4-yl-4*H-*pyrrolo[2,3-*d*]thiazol |
| | 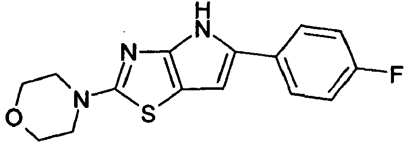 |
| "A13" | 5-(4-Fluor-phenyl)-2-(4-pyridin-4-ylmethyl-piperazin-1-yl)-4*H*-pyrrolo[2,3-*d*]thiazol |
| | 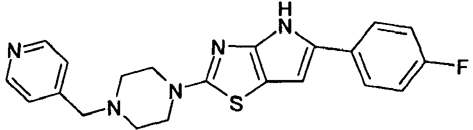 |
| "A14" | 5-[5-(4-Fluor-phenyl)-4*H*-pyrrolo[2,3-*d*]thiazol-2-yl]-pyrimidin-2-ylamin |
| | 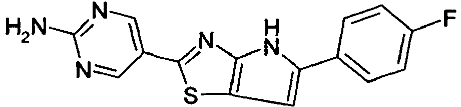 |
| "A30" | 3-[5-(4-Fluor-phenyl)-4*H*-pyrrolo[2,3-*d*]thiazol-2-yl]-pyridin-2-ylamin |
| | 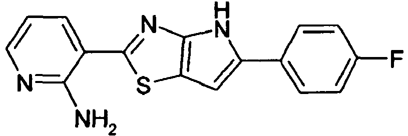 |
| "A31" | 3-[5-(4-Fluor-phenyl)-4H-pyrrolo[2,3-d]thiazol-2-yl]-5-(1-piperidin-4-yl-1H-pyrazol-4-yl)-pyridin-2-ylamin |
| "A32" | 6-(4,4-Dimethyl-pent-2-inyl)-4H-pyrrolo[2,3-d]thiazol |
| "A33" | 2-tert.-Butyl-3-(4H-pyrrolo[2,3-d]thiazol-6-ylmethyl)-1 H-indol-5-carbonsäure-methylester |
| "A34" | 2-tert.-Butyl-3-(4H-pyrrolo[2,3-d]thiazol-6-ylmethyl)-1H-pyrrolo[2,3-b]pyridin |
| | 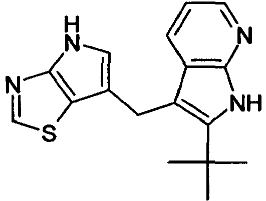 |
| "A35" | 6-(3,5-Difluor-benzyl)-4H-pyrrolo[2,3-d]thiazol |
| "A36" | 6-(4-Methoxy-benzyl)-4H-pyrrolo[2,3-d]thiazol |
| "A37" | 6-(4-Brom-benzyl)-4H-pyrrolo[2,3-d]thiazol |
| "A38" | 6-(4-Isopropyl-benzyl)-4H-pyrrolo[2,3-d]thiazol |
| "A39" | 6-(4-Brom-2-fluor-benzyl)-4H-pyrrolo[2,3-d]thiazol |
| "A40" | 6-(4-tert.-Butyl-benzyl)-4H-pyrrolo[2,3-d]thiazol |
| "A41" | 6-(2-Chlor-benzyl)-4H-pyrrolo[2,3-d]thiazol |
| "A42" | 6-(4-Fluor-benzyl)-4H-pyrrolo[2,3-d]thiazol |
| "A43" | 6-(3-Fluor-benzyl)-4H-pyrrolo[2,3-d]thiazol |
| "A44" | 6-(2,3-Difluor-benzyl)-4H-pyrrolo[2,3-d]thiazol |
| "A45" | 6-(2,4-Difluor-benzyl)-4H-pyrrolo[2,3-d]thiazol |
| "A46" | 6-(2,5-Difluor-benzyl)-4H-pyrrolo[2,3-d]thiazol |
| "A47" | 6-(3,4-Difluor-benzyl)-4H-pyrrolo[2,3-d]thiazol |
| "A48" | 6-(6-Chlor-pyridin-3-ylmethyl)-4H-pyrrolo[2,3-d]thiazol |
| "A49" | 6-(3-Chlor-benzyl)-4H-pyrrolo[2,3-d]thiazol |
| "A50" | 6-(3,4-Dichlor-benzyl)-4H-pyrrolo[2,3-d]thiazol |
| "A51" | 6-Benzyl-2-pyridin-3-yl-4H-pyrrolo[2,3-d]thiazol |
| "A52" | 6-Benzyl,-2-(1-methyl-1H-pyrazol-4-yl)-4H-pyrrolo-[2,3-d]thiazol |
| "A53" | 5-(6-Benzyl-4H-pyrrolo[2,3-d]thiazol-2-yl)-pyridin-2-ylamin |
| "A54" | 6-Prop-2-inyl-4H-pyrrolo[2,3-d]thiazol |
| "A55" | 3-(6-Benzyl-4H-pyrrolo[2,3-d]thiazol-2-yl)-pyridin-2-ylamin |
| "A56" | 6-Benzyl-2-(1H-pyrazol-4-yl)-4H-pyrrolo[2,3-d]thiazol |
| "A57" | 6-Benzyl-2-(5-brom-pyridin-3-yl)-4H-pyrrolo[2,3-d]thiazol |
| "A58" | 6-Benzyl-2-pyrimidin-5-yl-4H-pyrrolo[2,3-d]thiazol |
| "A59" | Morpholin-4-yl-[3-(4H-pyrrolo[2,3-d]thiazol-6-ylmethyl)-1H-indol-5-yl]-methanon |
| "A60" | 3-(4H-pyrrolo[2,3-d]thiazol-6-ylmethyl)-1H-indol-5-carbonsäure-(2-methoxy-ethyl)-amid |
| "A61" | 3-(4H-pyrrolo[2,3-d]thiazol-6-ylmethyl)-1H-indol-5-carbonsäure-4-fluor-benzylamid |
| "A62" | N-(2-Amino-ethyl)-2-[4-(4H-pyrrolo[2,3-d]thiazol-6-ylmethyl)-[1,2,3]triazol-1-yl]-acetamid |
| "A63" | 1-Piperazin-1-yl-2-[4-(4H-pyrrolo[2,3-d]thiazol-6-ylmethyl)-[1,2,3]triazol-1-yl]-ethanon |
| "A64" | N-Piperidin-4-yl-2-[4-(4H-pyrrolo[2,3-d]thiazol-6-ylmethyl)-[1,2,3]triazol-1-yl]-acetamid |
| "A65" | 6-(1-Pyridin-3-ylmethyl-1 H-[1,2,3]triazol-4-ylmethyl) 4H-pyrrolo[2,3-d]thiazol |
| "A66" | 6-(1-Pyridin-4-ylmethyl-1H-[1,2,3]triazol-4-ylmethyl) 4H-pyrrolo[2,3-d]thiazol |
| "A67" | 6-Benzyl-2-(5-(1-methyl-1H-pyrazol-4-yl)-pyridin-3-yl)-4H-pyrrolo[2,3-d]thiazol |
| "A68" | 6-Benzyl-2-(5-(1H-pyrazol-4-yl)-pyridin-3-yl)-4H-pyrrolo[2,3-d]thiazol |
| "A69" | 6-Benzyl-2-(5-(4-fluor-phenyl)-pyridin-3-yl)-4H-pyrrolo[2,3-d]thiazol |
| "A70" | 6-(5-Methansulfonyl-thiophen-2ylmethyl)-4H-pyrrolo[2,3-d]thiazol |
| "A71" | 6-[1-(2-Morpholin-4-yl-ethyl)-1H-[1,2,3]triazol-4-ylmethyl) 4H-pyrrolo[2,3-d]thiazol |
| "A72" | 6-Benzyl-2-{5-[1-((E)-3-phenyl-allyl)-1H-pyrazol-4-yl]-pyridin-3-yl}-4H-pyrrolo[2,3-d]thiazol |
sowie ihre pharmazeutisch verwendbaren Salze, Tautomere und Stereoisomere, einschließlich deren Mischungen in allen Verhältnissen.

11. Verfahren zur Herstellung von Verbindungen der Formel la nach den Ansprüchen 1-10 sowie ihrer pharmazeutisch verwendbaren Salze, Tautomeren und Stereoisomeren, **dadurch gekennzeichnet, daß** man
a) zur Herstellung einer Verbindung der Formel Ia', 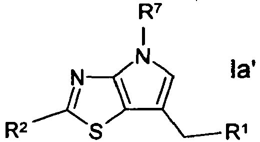 worin
R¹ Ar oder Het,
R² außer Hal, die in Anspruch 1 angegebenen Bedeutungen hat,
R⁷ tert.-Butyloxycarbonyl
bedeuten,
und Ar und Het die in Anspruch 1 angegebenen Bedeutungen haben,
eine Verbindung der Formel IIc 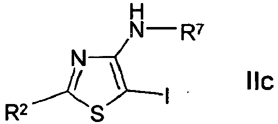 worin
R² außer Hal, die in Anspruch 1 angegebene Bedeutung hat,
R⁷ tert.-Butyloxycarbonyl
bedeuten,
mit einer Verbindung der Formel IIIc
R¹-CH=CH-CH₂Br IIIc
worin R¹ Ar oder Het bedeutet,
und Ar und Het die in Anspruch 1 angegebenen Bedeutungen haben,
umsetzt,
und anschließend oder gleichzeitig die tert.-Butyloxycarbonyl-Gruppe abspaltet,
oder
b) zur Herstellung einer Verbindung der Formel la" 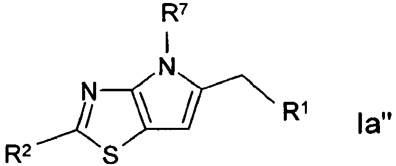 worin
R¹ Ar oder Het,
R² H, Cl, (CH₂)ₙHet³
R⁷ H
bedeuten,
und n, Ar, Het und Het³ die in Anspruch 1 angegebenen Bedeutungen haben,
eine Verbindung der Formel Ib' 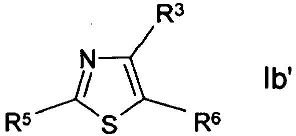 worin
R³ NH-COO-tert.-butyl,
R⁵ H, Cl, (CH₂)ₙHet³,
R⁶ -C≡C-R⁴
R⁴ Ar oder Het,
bedeuten,
und Ar, Het, Het³ und R⁴ die in Anspruch 1 angegebenen bedeutungen haben,
in Gegenwart einer Base erhitzt,
oder
c) zur Herstellung einer Verbindung der Formel la" 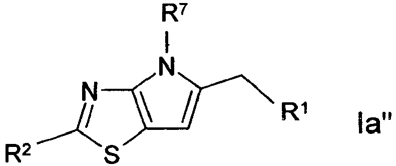 worin
R¹ Ar oder Het,
R² Het¹
R⁷ H
bedeuten,
eine Verbindung der Formel la" 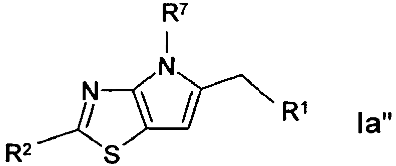 worin
R¹ Ar oder Het,
R² Hal
R⁷ H
bedeuten,
Ar und Het die in Anspruch 1 angegebenen Bedeutungen haben,
mit einer Verbindung der Formel Va
Het¹-H Va
worin Het¹ die in Anspruch 1 angegebene Bedeutung hat,
umsetzt,
und/oder
eine Base oder Säure der Formel la in eines ihrer Salze umwandelt.

12. Arzneimittel, enthaltend mindestens eine Verbindung der Formel la nach Anspruch 1-10 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

13. Verbindungen nach Anspruch 1-10 sowie ihrer pharmazeutisch verwendbaren Salze, Tautomeren und Stereoisomeren, einschließlich deren Mischungen in allen Verhältnissen, zur Verwendung zur Herstellung eines Arzneimittels zur Behandlung von Tumoren, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, Autoimmunerkrankungen, Zirrhose, Diabetes und Erkrankungen der Blutgefässe.

14. Verbindungen ausgewählt aus der Gruppe
| Nr. | Name und/oder Struktur |
|---|---|
| "2" | *tert*.-Butyl-*N*-(5-iodo-1,3-thiazol-4-yl)carbamat |
| "A15" | *tert*.-Butyl-*N*-{5-[2-(4-fluorphenyl)ethinyl]-1,3-thiazol-4-yl}carbamat |
| | 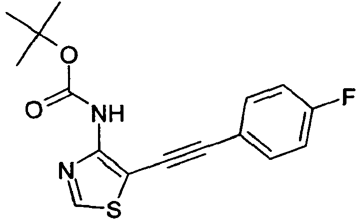 |
| "A16" | [5-(3-Phenyl-prop-1-inyl)-thiazol-4-yl]-carbaminsäure-tert.-butylester |
| | 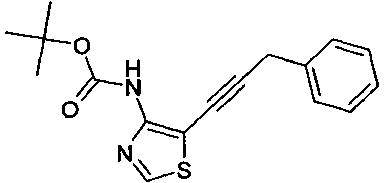 |
| "A17" | (5-Triethylsilanylethinyl-thiazol-4-yl)-carbaminsäure-tert.-butylester |
| | 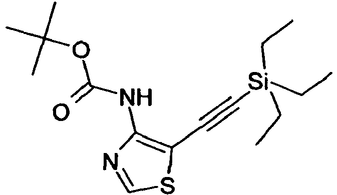 |
| "A18" | [5-(3-Amino-phenylethinyl)-thiazol-4-yl]-carbaminsäure-tert.-butylester |
| | 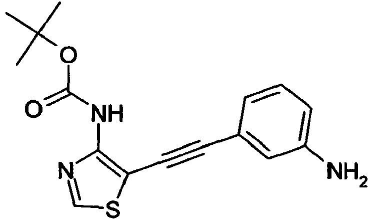 |
| "A19" | [5-(3-Benzylamino-phenylethinyl)-thiazol-4-yl]-carbaminsäure-tert.-butylester |
| | 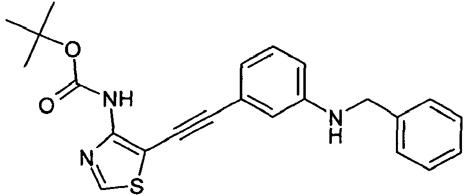 |
| "A20" | [5-(2-Benzylamino-pyridin-4-ylethinyl)-thiazol-4-yl]-carbaminsäure-tert.-butyl ester |
| | 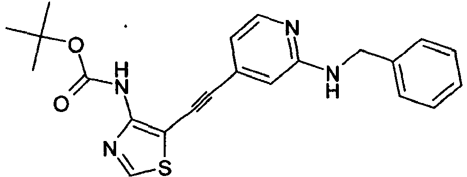 |
| "A21" | [5-(1H-Pyrrolo[2,3-b]pyridin-5-ylethinyl)-thiazol-4-yl]-carbaminsäure-tert.-butyl ester |
| | 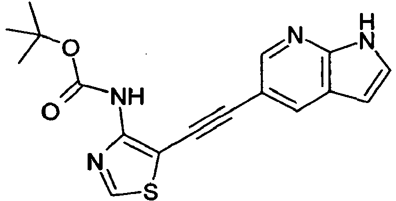 |
| "A22" | 5-(1H-Pyrrolo[2,3-b]pyridin-5-ylethinyl)-thiazol-4-ylamin |
| | 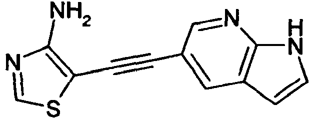 |
| "A24" | *tert*.-Butyl-*N*-(2-pyridin-4-yl-5-iodo-1,3-thiazol-4-yl)carbamat |
| | 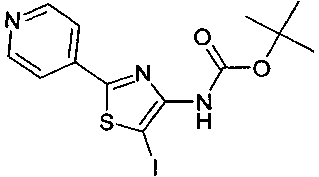 |
| "A25" | [5-(3-Morpholin-4-yl-prop-1-inyl)-2-pyridin-4-yl-thiazol-4-yl]-carbaminsäure-tert.-butylester |
| | 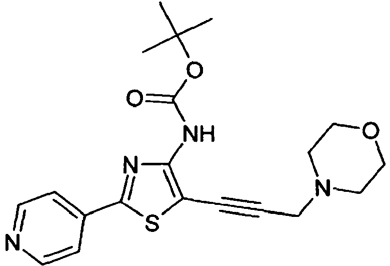 |
| "A26" | {5-[3-(1,3-Dioxo-1,3-dihydro-isoindol-2-yl)-prop-1-inyl]-thiazol-4-yl}-carbaminsäure-tert.-butylester |
| | 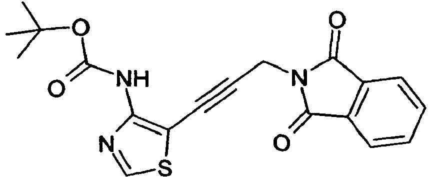 |
| "A27" | 3-(4-Amino-thiazol-5-ylethinyl)-phenol |
| | 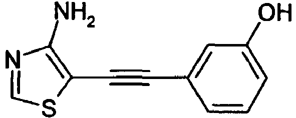 |
| "A28" | [2-(2-Amino-pyridin-3-yl)-5-(4-fluor-phenylethinyl)-thiazol-4-yl]-carbaminsäure-tert.-butylester |
| | 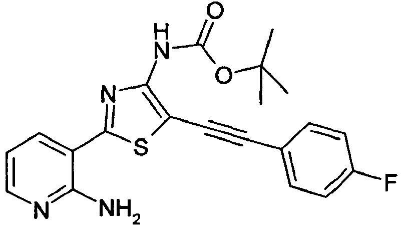 |
| "A29a" | *tert*.-Butyl-*N*-{2-chlor-5-[2-(4-fluorphenyl)ethinyl]-1,3-thiazol-4-yl}carbamat |
| | 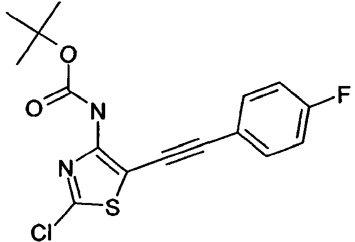 |
| "A29" | *tert*.-Butyl-*N*-[2-(2-aminopyrimidin-5-yl)-5-[2-(4-fluorphenyl)ethinyl]-1,3-thiazol-4-yl]carbamat |
| | 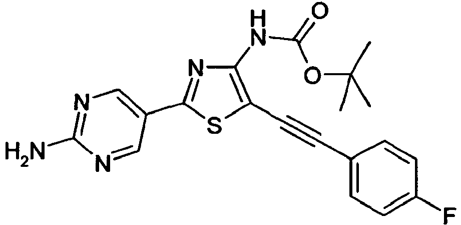 |
sowie ihre pharmazeutisch verwendbaren Salze, Tautomere und Stereoisomere, einschließlich deren Mischungen in allen Verhältnissen.

15. Arzneimittel, enthaltend mindestens eine Verbindung nach Anspruch 14 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

## Claims

1. Compounds of the formula 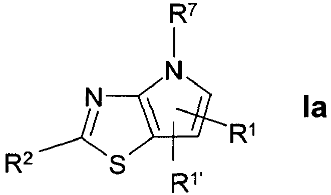 in which
R¹ denotes H, (CH₂)ₙAr or (CH₂)ₙHet,
R^{1'} denotes H or A,
R² denotes H, A, Hal, (CH₂)ₙHet¹, (CH₂)ₙHet³, -C≡C-Ar, (CH₂)ₙAr², NHCONHAr, COA, COOH, COOA, COHet¹, COAr², CONH₂, CONHA, CONA₂, SO₂NH₂, SO₂NHA, S(O)ₘA, NHCOA, SO₂NHA, SO₂NA₂, SO₂NHHet¹, SO₂NHAr² or 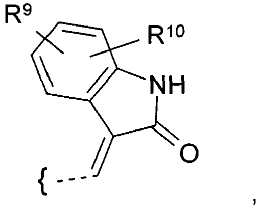
R⁷ denotes H, A, Ar or Het¹,
Ar denotes phenyl which is unsubstituted or mono-, di- or trisubstituted by Hal, A, OH, OA, CN, NO₂, SO₂A, COOH, COOA, NH₂, NHA, NA₂, NHCH₂Ar¹, CHO, COA, CHO, CONH₂, CONHA, CONA₂, Het, SO₂NH₂, SO₂NHA S(O)ₘA and/or NHCOA,
Het denotes a mono- or bicyclic aromatic heterocycle having 1 to 4 N, O and/or S atoms, which may be unsubstituted or mono-, di- or trisubstituted by Hal, A, NH₂, NHA, NA₂, COOH, COOA, CONH₂, CONHA, CONA₂, CONHAr, CONHHet⁴, S(O)ₘA and/or NHCH₂Ar¹,
Ar¹ denotes phenyl which is unsubstituted or mono-, di- or trisubstituted by Hal, A, OH and/or OA,
Ar² denotes phenyl which is monosubstituted by NHCONHHet⁴,
Het¹ denotes a mono- or bicyclic aromatic, unsaturated or saturated heterocycle having 1 to 4 N, O and/or S atoms, which may be unsubstituted or mono-, di- or trisubstituted by Hal, A, COOA, NH₂, NHR⁸ and/or (CH₂)ₙHet²,
R⁸ denotes H, A, Ar¹ or Het⁴,
R⁹, R¹⁰ each, independently of one another, denote H or Hal,
R¹¹ denotes H or A',
Het² denotes a mono- or bicyclic aromatic heterocycle having 1 to 4 N, O and/or S atoms, which may be unsubstituted or mono-, di- or trisubstituted by A, COOA and/or NH₂,
Het³ denotes a mono- or bicyclic aromatic heterocycle having 1 to 4 N, O and/or S atoms, which may be unsubstituted or mono-, di- or trisubstituted by NH₂,
Het⁴ denotes a mono- or bicyclic aromatic, unsaturated or saturated heterocycle having 1 to 4 N, O and/or S atoms, which may be unsubstituted or mono-, di- or trisubstituted by Hal, A, Ar¹, NH₂, NHCH₂Ar¹ and/or =O,
A denotes unbranched or branched alkyl having 1-10 C atoms, in which 1-7 H atoms may be replaced by F and/or in which one or two non-adjacent CH and/or CH₂ groups may be replaced by O, NH, NA', S, SO, SO₂ and/or by CH=CH groups,
or
cyclic alkyl having 3-7 C atoms,
A' denotes unbranched or branched alkyl having 1-4 C atoms,
Hal denotes F, Cl, Br or I,
m denotes 0, 1 or 2,
n denotes 0, 1, 2, 3 or 4,
and pharmaceutically usable salts, tautomers and stereoisomers thereof, including mixtures thereof in all ratios,
where 4H-pyrrolo[2,3-d]thiazole, 2,5-dimethyl-4H-pyrrolo[2,3-d]thiazole, 6-methyl-4H-pyrrolo[2,3-d]thiazole, 2-methyl-4H-Pyrrolo[2,3-d]thiazole, 5-methyl-4H-pyrrolo[2,3-d]thiazole, 4-methyl-4H-pyrrolo[2,3-d]thiazole, 2,4,5-trimethyl-4H-pyrrolo[2,3-d]thiazole, 2,4-dimethyl-4H-pyrrolo[2,3-d]-thiazole, 4,5-dimethyl-4H-pyrrolo[2,3-d]thiazole, 4,6-dimethyl-4H-pyrrolo-[2,3-d]thiazole are excluded.

2. Compounds according to Claim 1 in which
R¹ denotes H, (CH₂)ₙAr or (CH₂)ₙHet,
R^{1'} denotes H,
R² denotes H, Hal, (CH₂)ₙHet¹, (CH₂)ₙHet³ or -C=C-Ar,
R⁷ denotes H,
Ar denotes phenyl which is unsubstituted or mono-, di- or trisubstituted by Hal, A, OH, OA, CN, NO₂, SO₂A, COOH, COOA, NH₂, NHA, NA₂, NHCH₂Ar¹, CHO, COA, CHO, CONH₂, CONHA, CONA₂, SO₂NH₂, SO₂NHA and/or NHCOA,
Het denotes a mono- or bicyclic aromatic heterocycle having 1 to 4 N, O and/or S atoms, which may be unsubstituted or mono-, di- or trisubstituted by Hal, A, NH₂ and/or NHCH₂Ar¹,
Ar¹ denotes phenyl which is unsubstituted or mono-, di- or trisubstituted by Hal, A, OH and/or OA,
Het¹ denotes a mono- or bicyclic aromatic, unsaturated or saturated heterocycle having 1 to 4 N, O and/or S atoms, which may be unsubstituted or mono-, di- or trisubstituted by Hal, A, NH₂ and/or CH₂Het²,
Het² denotes a mono- or bicyclic aromatic heterocycle having 1 to 4 N, O and/or S atoms, which may be unsubstituted or mono-, di- or trisubstituted by A and/or NH₂,
Het³ denotes a mono- or bicyclic aromatic heterocycle having 1 to 4 N, O and/or S atoms, which may be unsubstituted or mono-, di- or trisubstituted by NH₂,
Het⁴ denotes a mono- or bicyclic aromatic, unsaturated or saturated heterocycle having 1 to 4 N, O and/or S atoms, which may be unsubstituted or mono-, di- or trisubstituted by Hal, A, NH₂, NHCH₂Ar¹ and/or =O,
A denotes unbranched or branched alkyl having 1-10 C atoms, in which 1-7 H atoms may be replaced by OH, F, Cl and/or Br,
A' denotes unbranched or branched alkyl having 1-4 C atoms,
Hal denotes F, Cl, Br or I,
n denotes 0, 1, 2, 3 or 4,
and pharmaceutically usable salts, tautomers and stereoisomers thereof, including mixtures thereof in all ratios.

3. Compounds according to Claim 1 or 2 in which
Ar denotes phenyl which is unsubstituted or mono-, di- or trisubstituted by Hal, OH, OA, NH₂, NHA, NA₂ and/or NHCH₂Ar¹,
and pharmaceutically usable salts, tautomers and stereoisomers thereof, including mixtures thereof in all ratios.

4. Compounds according to Claim 1, 2 or 3 in which
Het denotes furyl, thienyl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazolyl, pyridazinyl, pyrazinyl, benzimidazolyl, benzotriazolyl, quinolinyl, quinoxalinyl, quinazolinyl, pyrrolopyridinyl, purinyl, indolyl or indazolyl, each of which is unsubstituted or mono- or disubstituted by NH₂ and/or NHCH₂Ar¹,
and pharmaceutically usable salts, tautomers and stereoisomers thereof, including mixtures thereof in all ratios.

5. Compounds according to one or more of Claims 1-4
in which
Ar¹ denotes phenyl,
and pharmaceutically usable salts, tautomers and stereoisomers thereof,
including mixtures thereof in all ratios.

6. Compounds according to one or more of Claims 1-5
in which
Het¹ denotes piperidinyl, pyrrolidinyl, morpholinyl, piperazinyl, imidazolidinyl, oxazolidinyl, tetrahydropyranyl, thiazolyl, thiophenyl, furanyl, pyrrolyl, oxazolyl, isoxazolyl, oxadiazolyl, pyrazolyl, imidazolyl, triazolyl, thiadiazolyl, pyridazinyl, pyrazinyl, pyridinyl or pyrimidinyl, each of which is unsubstituted or mono- or disubstituted by NH₂ and/or CH₂Het²,
and pharmaceutically usable salts, tautomers and stereoisomers thereof,
including mixtures thereof in all ratios.

7. Compounds according to one or more of Claims 1-6
in which
Het² denotes furyl, thienyl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazolyl, pyridazinyl, pyrazinyl, benzimidazolyl, benzotriazolyl, quinolinyl, quinoxalinyl, quinazolinyl, pyrrolopyridinyl, purinyl, indolyl or indazolyl, each of which is unsubstituted or mono- or disubstituted by A and/or NH₂,
and pharmaceutically usable salts, tautomers and stereoisomers thereof,
including mixtures thereof in all ratios.

8. Compounds according to one or more of Claims 1-7
in which
Het³ denotes furyl, thienyl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazolyl, pyridazinyl, pyrazinyl, benzimidazolyl, benzotriazolyl, quinolinyl, quinoxalinyl, quinazolinyl, pyrrolopyridinyl, purinyl, indolyl or indazolyl, each of which is unsubstituted or mono- or disubstituted by A and/or NH₂,
and pharmaceutically usable salts, tautomers and stereoisomers thereof,
including mixtures thereof in all ratios.

9. Compounds according to one or more of Claims 1-8
in which
Het⁴ denotes piperidinyl, pyrrolidinyl, morpholinyl, piperazinyl, imidazolidinyl, oxazolidinyl, tetrahydropyranyl, dihydroisoindolyl, furyl, thienyl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazolyl, pyridazinyl, pyrazinyl, benzimidazolyl, benzotriazolyl, quinolinyl, quinoxalinyl, quinazolinyl, pyrrolopyridinyl, purinyl, indolyl or indazolyl, each of which is unsubstituted or mono- or disubstituted by NH₂, NHCH₂Ar¹ and/or =O,
and pharmaceutically usable salts, tautomers and stereoisomers thereof,
including mixtures thereof in all ratios.

10. Compounds according to Claim 1, selected from the group
| No. | Name and/or structure |
|---|---|
| "A1" | 5-(4-Fluorophenyl)-4*H*-pyrrolo[2,3*-d*]-1,3-thiazole |
| | 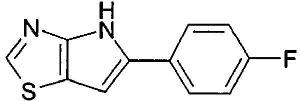 |
| "A2" | 5-Benzyl-4*H*-pyrrolo[2,3-*d*]thiazole |
| "A4" | 3-(4*H*-Pyrrolo[2,3-d]thiazol-5-yl)phenylamine |
| | 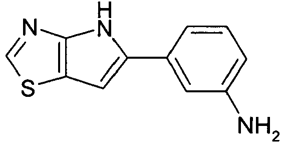 |
| "A5" | Benzyl-[4-(4*H*-pyrrolo[2,3-*d*]thiazol-5-yl)pyridin-2-yl]amine |
| | 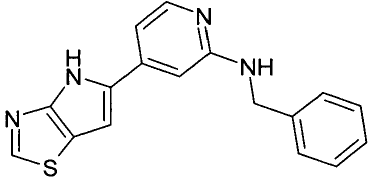 |
| "A6" | Benzyl-[3-(4*H*-pyrrolo[2,3-*d*]thiazol-5-yl)phenyl]amine |
| | 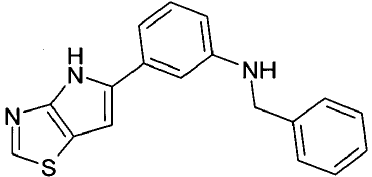 |
| "A7" | 6-Benzyl-4*H*-pyrrolo[2,3-*d*]thiazole |
| | 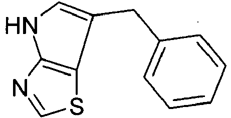 |
| "A8" | 6-Benzyl-2-pyridin-4-yl-4*H*-pyrrolo[2,3-*d*]thiazole |
| | 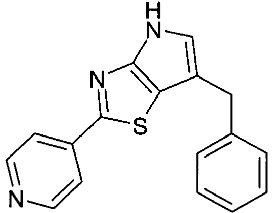 |
| "A9" | 4-(4*H*-Pyrrolo[2,3-*d*]thiazol-5-yl)pyridin-2-ylamine |
| | 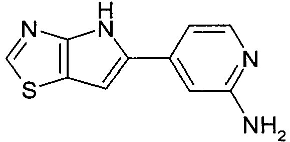 |
| "A10" | 2-Chloro-5-(4-fluorophenyl)-4*H*-pyrrolo[2,3-*d*]thiazole |
| | 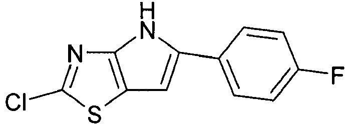 |
| "A11" | 5-(4-Fluorophenyl)-2-(4-fluorophenylethynyl)-4*H*-pyrrolo-[2,3-*d*]thiazole |
| | 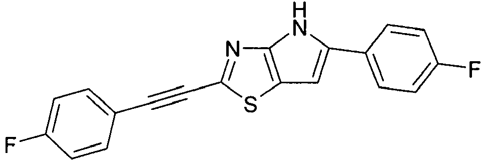 |
| "A12" | 5-(4-Fluorophenyl)-2-morpholin-4-yl-4*H*-pyrrolo-[2,3-*d*]thiazole |
| | 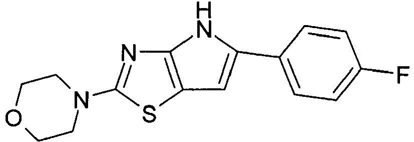 |
| "A13" | 5-(4-Fluorophenyl)-2-(4-pyridin-4-ylmethylpiperazin-1-yl)-4*H*-pyrrolo[2,3-*d*]thiazole |
| | 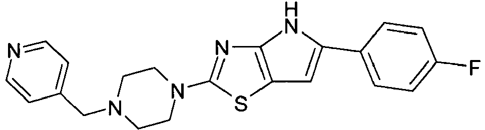 |
| "A14" | 5-[5-(4-Fluorophenyl)-4*H*-pyrrolo[2,3-*d*]thiazol-2-yl]-pyrimidin-2-ylamine |
| | 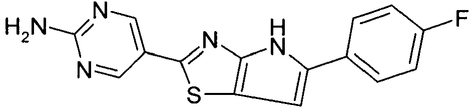 |
| "A30" | 3-[5-(4-Fluorophenyl)-4H-pyrrolo[2,3-d]thiazol-2-yl]-pyridin-2-ylamine |
| | 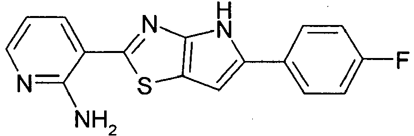 |
| "A31" | 3-[5-(4-Fluorophenyl)-4H-pyrrolo[2,3-d]thiazol-2-yl]-5-(1-piperidin-4-yl-1H-pyrazol-4-yl)pyridin-2-ylamine |
| "A32" | 6-(4,4-Dimethylpent-2-ynyl)-4H-pyrrolo[2,3-d]thiazole |
| "A33" | Methyl 2-tert-butyl-3-(4H-pyrrolo[2,3-d]thiazol-6-ylmethyl)-1H-indole-5-carboxylate |
| "A34" | 2-tert-Butyl-3-(4H-pyrrolo[2,3-d]thiazol-6-ylmethyl)-1H-pyrrolo[2,3-b]pyridine |
| | 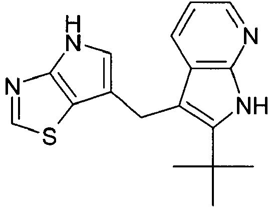 |
| "A35" | 6-(3,5-Difluorobenzyl)-4H-pyrrolo[2,3-d]thiazole |
| "A36" | 6-(4-Methoxybenzyl)-4H-pyrrolo[2,3-d]thiazole |
| "A37" | 6-(4-Bromobenzyl)-4H-pyrrolo[2,3-d]thiazole |
| "A38" | 6-(4-Isopropylbenzyl)-4H-pyrrolo[2,3-d]thiazole |
| "A39" | 6-(4-Bromo-2-fluorobenzyl)-4H-pyrrolo[2,3-d]thiazole |
| "A40" | 6-(4-tert-Butylbenzyl)-4H-pyrrolo[2,3-d]thiazole |
| "A41" | 6-(2-Chlorobenzyl)-4H-pyrrolo[2,3-d]thiazole |
| "A42" | 6-(4-Fluorobenzyl)-4H-pyrrolo[2,3-d]thiazole |
| "A43" | 6-(3-Fluorobenzyl)-4H-pyrrolo[2,3-d]thiazole |
| "A44" | 6-(2,3-Difluorobenzyl)-4H-pyrrolo[2,3-d]thiazole |
| "A45" | 6-(2,4-Difluorobenzyl)-4H-pyrrolo[2,3-d]thiazole |
| "A46" | 6-(2,5-Difluorobenzyl)-4H-pyrrolo[2,3-d]thiazole |
| "A47" | 6-(3,4-Difluorobenzyl)-4H-pyrrolo[2,3-d]thiazole |
| "A48" | 6-(6-Chloropyridin-3-ylmethyl)-4H-pyrrolo[2,3-d]thiazole |
| "A49" | 6-(3-Chlorobenzyl)-4H-pyrrolo[2,3-d]thiazole |
| "A50" | 6-(3,4-Dichlorobenzyl)-4H-pyrrolo[2,3-d]thiazole |
| "A51" | 6-Benzyl-2-pyridin-3-yl-4H-pyrrolo[2,3-d]thiazole |
| "A52" | 6-Benzyl-2-(1-methyl-1H-pyrazol-4-yl)-4H-pyrrolo-[2,3-d]thiazole |
| "A53" | 5-(6-Benzyl-4H-pyrrolo[2,3-d]thiazol-2-yl)pyridin-2-yl-amine |
| "A54" | 6-Prop-2-ynyl-4H-pyrrolo[2,3-d]thiazole |
| "A55" | 3-(6-Benzyl-4H-pyrrolo[2,3-d]thiazol-2-yl)pyridin-2-yl-amine |
| "A56" | 6-Benzyl-2-(1H-pyrazol-4-yl)-4H-pyrrolo[2,3-d]thiazole |
| "A57" | 6-Benzyl-2-(5-bromopyridin-3-yl)-4H-pyrrolo[2,3-d]-thiazole |
| "A58" | 6-Benzyl-2-pyrimidin-5-yl-4H-pyrrolo[2,3-d]thiazole |
| "A59" | Morpholin-4-yl-[3-(4H-pyrrolo[2,3-d]thiazol-6-ylmethyl)-1H-indol-5-yl]methanone |
| "A60" | N-(2-Methoxyethyl)-3-(4H-pyrrolo[2,3-d]thiazol-6-yl-methyl)-1H-indole-5-carboxamide |
| "A61" | N-(4-Fluorobenzyl)-3-(4H-pyrrolo[2,3-d]thiazol-6-yl-methyl)-1H-indole-5-carboxamide |
| "A62" | N-(2-Aminoethyl)-2-[4-(4H-pyrrolo[2,3-d]thiazol-6-yl-methyl)-1,2,3-triazol-1-yl]acetamide |
| "A63" | 1-Piperazin-1-yl-2-[4-(4H-pyrrolo[2,3-d]thiazol-6-yl-methyl)-1,2, 3-triazol-1-yl]ethanone |
| "A64" | N-(Piperidin-4-yl)-2-[4-(4H-pyrrolo[2,3-d]thiazol-6-yl-methyl)-1,2,3-triazol-1-yl]acetamide |
| "A65" | 6-(1-Pyridin-3-ylmethyl-1H-1,2,3-triazol-4-ylmethyl)-4H-pyrrolo[2,3-d]thiazole |
| "A66" | 6-(1-Pyridin-4-ylmethyl-1H-1,2,3-triazol-4-ylmethyl)-4H-pyrrolo[2,3-d]thiazole |
| "A67" | 6-Benzyl-2-(5-(1-methyl-1H-pyrazol-4-yl)pyridin-3-yl)-4H-pyrrolo[2,3-d]thiazole |
| "A68" | 6-Benzyl-2-(5-(1H-pyrazol-4-yl)pyridin-3-yl)-4H-pyrrolo-[2,3-d]thiazole |
| "A69" | 6-Benzyl-2-(5-(4-fluorophenyl)pyridin-3-yl)-4H-pyrrolo-[2,3-d]thiazole |
| "A70" | 6-(5-Methanesulfonylthiophen-2-ylmethyl)-4H-pyrrolo-[2,3-d]thiazole |
| "A71" | 6-[1-(2-Morpholin-4-ylethyl)-1H-1,2,3-triazol-4-ylmethyl]-4H-pyrrolo[2,3-d]thiazole |
| "A72" | 6-Benzyl-2-{5-[1-((E)-3-phenylallyl)-1H-pyrazol-4-yl]-pyridin-3-yl}-4H-pyrrolo[2,3-d]thiazole |
and pharmaceutically usable salts, tautomers and stereoisomers thereof, including mixtures thereof in all ratios.

11. Process for the preparation of compounds of the formula la according to Claims 1-10 and pharmaceutically usable salts, tautomers and stereoisomers thereof, **characterised in that**
a) for the preparation of a compound of the formula la', 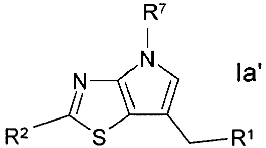 in which
R¹ denotes Ar or Het,
R² has the meanings indicated in Claim 1, apart from Hal,
R⁷ denotes tert-butyloxycarbonyl,
and Ar and Het have the meanings indicated in Claim 1,
a compound of the formula IIc 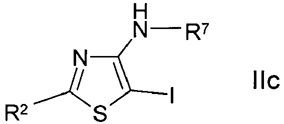 in which
R² has the meaning indicated in Claim 1, apart from Hal,
R⁷ denotes tert-butyloxycarbonyl,
is reacted with a compound of the formula IIIc
R¹-CH=CH-CH₂Br IIIc
in which R¹ denotes Ar or Het,
and Ar and Het have the meanings indicated in Claim 1,
and subsequently or simultaneously the tert-butyloxycarbonyl group is cleaved off,
or
b) for the preparation of a compound of the formula la" 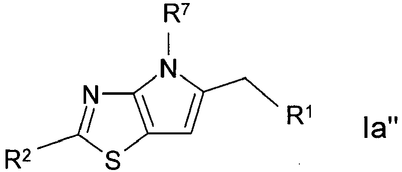 in which
R¹ denotes Ar or Het,
R² denotes H, Cl, (CH₂)ₙHet³,
R⁷ denotes H,
and n, Ar, Het and Het³ have the meanings indicated in Claim 1,
a compound of the formula Ib' 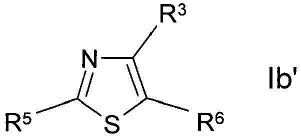 in which
R³ denotes NH-COO-tert-butyl,
R⁵ denotes H, Cl, (CH₂)ₙHet³,
R⁶ denotes -C≡C-R⁴,
R⁴ denotes Ar or Het,
and Ar, Het, Het³ and R⁴ have the meanings indicated in Claim 1,
is heated in the presence of a base,
or
c) for the preparation of a compound of the formula la" 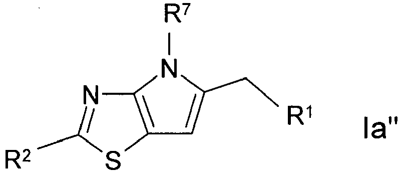 in which
R¹ denotes Ar or Het,
R² denotes Het¹,
R⁷ denotes H,
a compound of the formula la" 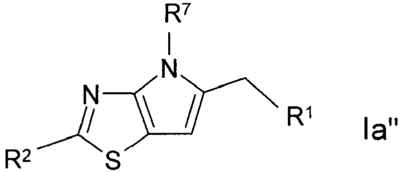 in which
R¹ denotes Ar or Het,
R² denotes Hal,
R⁷ denotes H,
Ar and Het have the meanings indicated in Claim 1,
is reacted with a compound of the formula Va
Het¹-H Va
in which Het¹ has the meaning indicated in Claim 1,
and/or
a base or acid of the formula la is converted into one of its salts.

12. Medicaments comprising at least one compound of the formula la according to Claims 1-10 and/or pharmaceutically usable salts, tautomers and stereoisomers thereof, including mixtures thereof in all ratios, and optionally excipients and/or adjuvants.

13. Compounds according to Claims 1-10 and pharmaceutically usable salts, tautomers and stereoisomers thereof, including mixtures thereof in all ratios, for use for the preparation of a medicament for the treatment of tumours, cancer, tumour formation, growth and propagation, arteriosclerosis, ocular diseases, such as age-induced macular degeneration, choroidal neovascularisation and diabetic retinopathy, inflammatory diseases, arthritis, thrombosis, fibrosis, glomerulonephritis, neurodegeneration, psoriasis, restenosis, wound healing, transplant rejection, metabolic and diseases of the immune system, autoimmune diseases, cirrhosis, diabetes and diseases of the blood vessels.

14. Compounds selected from the group
| No. | Name and/or structure |
|---|---|
| "2" | *tert*-Butyl *N*-(5-iodo-1,3-thiazol-4-yl)carbamate |
| "A15" | *tert*-Butyl *N*-{5-[2-(4-fluorophenyl)ethynyl]-1,3-thiazol-4-yl}carbamate |
| | 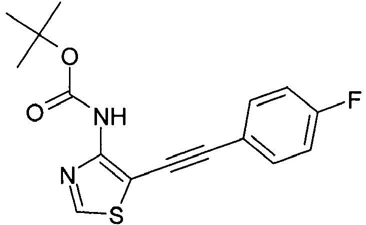 |
| "A16" | tert-Butyl [5-(3-phenylprop-1-ynyl)thiazol-4-yl]carbamate |
| | 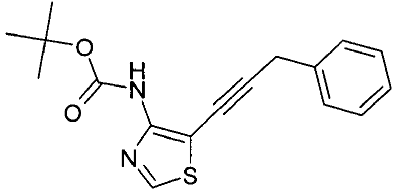 |
| "A17" | tert-Butyl (5-triethylsilanylethynylthiazol-4-yl)carbamate |
| | 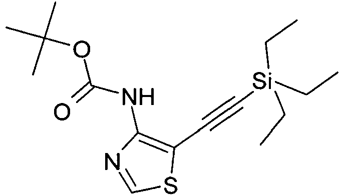 |
| "A18" | tert-Butyl [5-(3-aminophenylethynyl)thiazol-4-yl]-carbamate |
| | 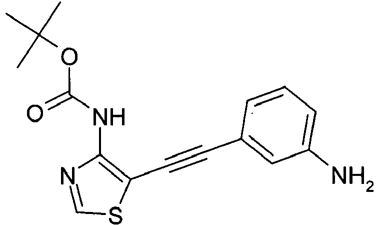 |
| "A19" | tert-Butyl [5-(3-benzylaminophenylethynyl)thiazol-4-yl]-carbamate |
| | 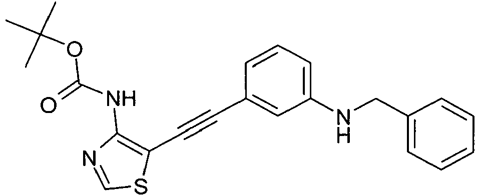 |
| "A20" | tert-Butyl [5-(2-benzylaminopyridin-4-ylethynyl)thiazol-4-yl]carbamate |
| | 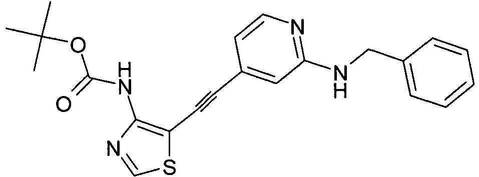 |
| "A21" | tert-Butyl [5-(1H-pyrrolo[2,3-b]pyridin-5-ylethynyl)thiazol-4-yl]carbamate |
| | 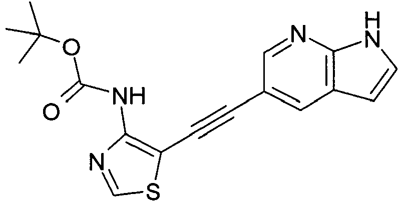 |
| "A22" | 5-(1H-Pyrrolo[2,3-b]pyridin-5-ylethynyl)thiazol-4-ylamine |
| | 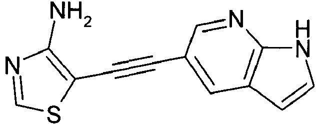 |
| "A24" | *tert*-Butyl *N*-(2-pyridin-4-yl-5-iodo-1,3-thiazol-4-yl)-carbamate |
| | 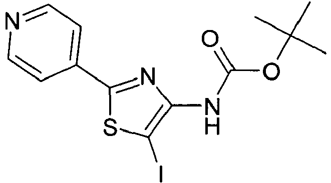 |
| "A25" | tert-Butyl [5-(3-morpholin-4-ylprop-1-ynyl)-2-pyridin-4-yl-thiazol-4-yl]carbamate |
| | 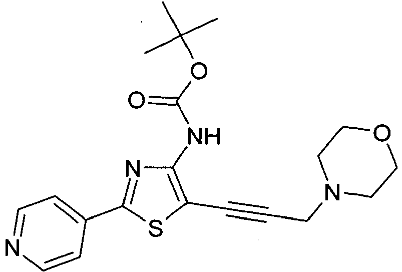 |
| "A26" | tert-Butyl {5-[3-(1,3-dioxo-1,3-dihydroisoindol-2-yl)prop-1-ynyl]thiazol-4-yl}carbamate |
| | 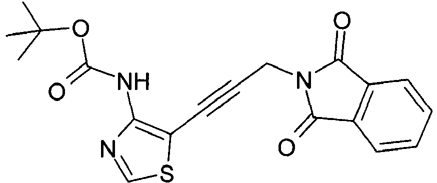 |
| "A27" | 3-(4-Aminothiazol-5-ylethynyl)phenol |
| | 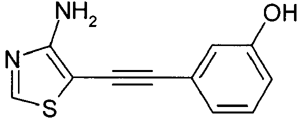 |
| "A28" | tert-Butyl [2-(2-aminopyridin-3-yl)-5-(4-fluorophenyl-ethynyl)thiazol-4-yl]carbamate |
| | 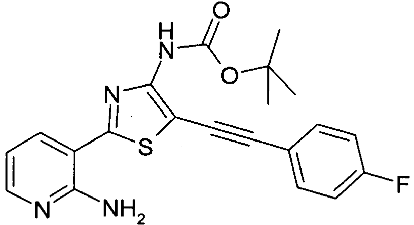 |
| "A29a" | *tert*-Butyl *N*-{2-chloro-5-[2-(4-fluorophenyl)ethynyl]-1,3-thiazol-4-yl}carbamate |
| | 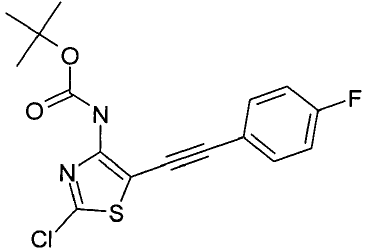 |
| "A29" | *tert*-Butyl *N*-[2-(2-aminopyrimidin-5-yl)-5-[2-(4-fluorophenyl)ethynyl]-1,3-thiazol-4-yl]carbamate |
| | 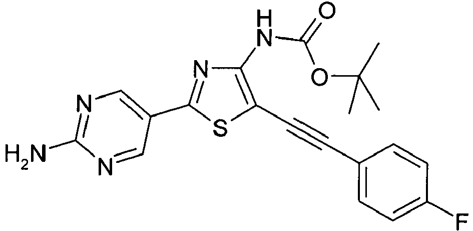 |
and pharmaceutically usable salts, tautomers and stereoisomers thereof, including mixtures thereof in all ratios.

15. Medicaments comprising at least one compound according to Claim 14 and/or pharmaceutically usable salts, tautomers and stereoisomers thereof, including mixtures thereof in all ratios, and optionally excipients and/or adjuvants.

## Revendications

1. Composés de formule 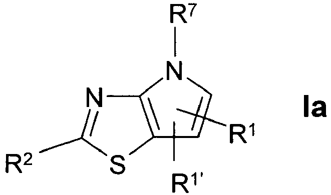 dans laquelle
R¹ désigne H, (CH₂)ₙAr ou (CH₂)ₙHét,
R^{1'} désigne H ou A,
R² désigne H, A, Hal, (CH₂)ₙHét¹, (CH₂)ₙHét³, -C≡C-Ar, (CH₂)ₙAr², NHCONHAr, COA, COOH, COOA, COHét¹, COAr², CONH₂, CONHA, CONA₂, SO₂NH₂, SO₂NHA, S(O)ₘA, NHCOA, SO₂NHA, SO₂NA₂, SO₂NHHét¹, SO₂NHAr² ou 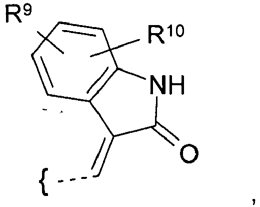
R⁷ désigne H, A, Ar ou Hét¹,
Ar désigne phényle qui est non substitué ou mono-, di- ou trisubstitué par Hal, A, OH, OA, CN, NO₂, SO₂A, COOH, COOA, NH₂, NHA, NA₂, NHCH₂Ar¹, CHO, COA, CHO, CONH₂, CONHA, CONA₂, Hét, SO₂NH₂ SO₂NHA, S(O)ₘA et/ou NHCOA,
Hét désigne un hétérocycle mono- ou bicyclique aromatique ayant de 1 à 4 atomes de N, O et/ou S, qui peut être non substitué ou mono-, di- ou trisubstitué par Hal, A, NH₂, NHA, NA₂, COOH, COOA, CONH₂, CONHA, CONA₂, CONHAr, CONHHét⁴, S(O)ₘA et/ou NHCH₂Ar¹,
Ar¹ désigne phényle qui est non substitué ou mono-, di- ou trisubstitué par Hal, A, OH et/ou OA,
Ar² désigne phényle qui est monosubstitué par NHCONHHét⁴,
Hét¹ désigne un hétérocycle mono- ou bicyclique aromatique, insaturé ou saturé ayant de 1 à 4 atomes de N, O et/ou S, qui peut être non substitué ou mono-, di- ou trisubstitué par Hal, A, COOA, NH₂, NHR⁸ et/ou (CH₂)ₙHét²,
R⁸ désigne H, A, Ar¹ ou Hét⁴,
R⁹, R¹⁰ désignent chacun, indépendamment l'un de l'autre, H ou Hal,
R¹¹ désigne H ou A',
Hét² désigne un hétérocycle mono- ou bicyclique aromatique ayant de 1 à 4 atomes de N, O et/ou S, qui peut être non substitué ou mono-, di- ou trisubstitué par A, COOA et/ou NH₂,
Hét³ désigne un hétérocycle mono- ou bicyclique aromatique ayant de 1 à 4 atomes de N, O et/ou S, qui peut être non substitué ou mono-, di- ou trisubstitué par NH₂,
Hét⁴ désigne un hétérocycle mono- ou bicyclique aromatique, insaturé ou saturé ayant de 1 à 4 atomes de N, O et/ou S, qui peut être non substitué ou mono-, di- ou trisubstitué par Hal, A, Ar¹, NH₂, NHCH₂Ar¹ et/ou =O,
A désigne alkyle non ramifié ou ramifié ayant 1-10 atomes de C, où 1-7 atomes de H peuvent être remplacés par F et/ou où un ou deux groupements CH et/ou CH₂ non adjacents peuvent être remplacés par O, NH, NA', S, SO, SO₂ et/ou par des groupements CH=CH,
ou
alkyle cyclique ayant 3-7 atomes de C,
A' désigne alkyle non ramifié ou ramifié ayant 1-4 atomes de C,
Hal désigne F, Cl, Br ou I,
m désigne 0, 1 ou 2,
n désigne 0, 1, 2, 3 ou 4,
et les sels, tautomères et stéréoisomères pharmaceutiquement utilisables de ceux-ci, y compris des mélanges de ceux-ci en toutes proportions,
où le 4H-pyrrolo[2,3-d]thiazole, le 2,5-diméthyl-4H-pyrrolo[2,3-d]thiazole, le 6-méthyl-4H-pyrrolo[2,3-d]thiazole, le 2-méthyl-4H-pyrrolo[2,3-d]-thiazole, le 5-méthyl-4H-pyrrolo[2,3-d]thiazole, le 4-méthyl-4H-pyrrolo-[2,3-d]thiazole, le 2,4,5-triméthyl-4H-pyrrolo[2,3-d]thiazole, le 2,4-diméthyl-4H-pyrrolo[2,3-d]thiazole, le 4,5-diméthyl-4H-pyrrolo[2,3-d]-thiazole, le 4,6-diméthyl-4H-pyrrolo[2,3-d]thiazole, sont exclus.

2. Composés selon la revendication 1, dans lesquels
R¹ désigne H, (CH₂)ₙAr ou (CH₂)ₙHét,
R^{1'} désigne H,
R² désigne H, Hal, (CH₂)ₙHét¹, (CH₂)ₙHét³ ou -C=C-Ar,
R⁷ désigne H,
Ar désigne phényle qui est non substitué ou mono-, di- ou trisubstitué par Hal, A, OH, OA, CN, NO₂, SO₂A, COOH, COOA, NH₂, NHA, NA₂, NHCH₂Ar¹, CHO, COA, CHO, CONH₂, CONHA, CONA₂, SO₂NH₂, SO₂NHA et/ou NHCOA,
Hét désigne un hétérocycle mono- ou bicyclique aromatique ayant de 1 à 4 atomes de N, O et/ou S, qui peut être non substitué ou mono-, di- ou trisubstitué par Hal, A, NH₂ et/ou NHCH₂Ar¹,
Ar¹ désigne phényle qui est non substitué ou mono-, di- ou trisubstitué par Hal, A, OH et/ou OA,
Hét¹ désigne un hétérocycle mono- ou bicyclique aromatique, insaturé ou saturé ayant de 1 à 4 atomes de N, O et/ou S, qui peut être non substitué ou mono-, di- ou trisubstitué par Hal, A, NH₂ et/ou CH₂Hét²,
Hét² désigne un hétérocycle mono- ou bicyclique aromatique ayant de 1 à 4 atomes de N, O et/ou S, qui peut être non substitué ou mono-, di- ou trisubstitué par A et/ou NH₂,
Hét³ désigne un hétérocycle mono- ou bicyclique aromatique ayant de 1 à 4 atomes de N, O et/ou S, qui peut être non substitué ou mono-, di- ou trisubstitué par NH₂,
Hét⁴ désigne un hétérocycle mono- ou bicyclique aromatique, insaturé ou saturé ayant de 1 à 4 atomes de N, O et/ou S, qui peut être non substitué ou mono-, di- ou trisubstitué par Hal, A, NH₂, NHCH₂Ar¹ et/ou =O,
A désigne alkyle non ramifié ou ramifié ayant 1-10 atomes de C, où 1-7 atomes de H peuvent être remplacés par OH, F, Cl et/ou Br,
A' désigne alkyle non ramifié ou ramifié ayant 1-4 atomes de C,
Hal désigne F, Cl, Br ou I,
n désigne 0, 1, 2, 3 ou 4,
et les sels, tautomères et stéréoisomères pharmaceutiquement utilisables de ceux-ci, y compris des mélanges de ceux-ci en toutes proportions.

3. Composés selon la revendication 1 ou 2, dans lesquels
Ar désigne phényle qui est non substitué ou mono-, di- ou trisubstitué par Hal, OH, OA, NH₂, NHA, NA₂ et/ou NHCH₂Ar¹,
et les sels, tautomères et stéréoisomères pharmaceutiquement utilisables de ceux-ci, y compris des mélanges de ceux-ci en toutes proportions.

4. Composés selon la revendication 1, 2 ou 3, dans lesquels
Hét désigne furyle, thiényle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle, benzimidazolyle, benzotriazolyle, quinoléinyle, quinoxalinyle, quinazolinyle, pyrrolopyridinyle, purinyle, indolyle ou indazolyle, chacun d'entre eux étant non substitué ou mono- ou disubstitué par NH₂ et/ou NHCH₂Ar¹,
et les sels, tautomères et stéréoisomères pharmaceutiquement utilisables de ceux-ci, y compris des mélanges de ceux-ci en toutes proportions.

5. Composés selon l'une ou plusieurs parmi les revendications 1-4, dans lesquels
Ar¹ désigne phényle,
et les sels, tautomères et stéréoisomères pharmaceutiquement utilisables de ceux-ci, y compris des mélanges de ceux-ci en toutes proportions.

6. Composés selon l'une ou plusieurs parmi les revendications 1-5, dans lesquels
Hét¹ désigne pipéridinyle, pyrrolidinyle, morpholinyle, pipérazinyle, imidazolidinyle, oxazolidinyle, tétrahydropyranyle, thiazolyle, thiophényle, furanyle, pyrrolyle, oxazolyle, isoxazolyle, oxadiazolyle, pyrazolyle, imidazolyle, triazolyle, thiadiazolyle, pyridazinyle, pyrazinyle, pyridinyle ou pyrimidinyle, chacun d'entre eux étant non substitué ou mono- ou disubstitué par NH₂ et/ou CH₂Hét²,
et les sels, tautomères et stéréoisomères pharmaceutiquement utilisables de ceux-ci, y compris des mélanges de ceux-ci en toutes proportions.

7. Composés selon l'une ou plusieurs parmi les revendications 1-6, dans lesquels
Hét² désigne furyle, thiényle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle, benzimidazolyle, benzotriazolyle, quinoléinyle, quinoxalinyle, quinazolinyle, pyrrolopyridinyle, purinyle, indolyle ou indazolyle, chacun d'entre eux étant non substitué ou mono- ou disubstitué par A et/ou NH₂,
et les sels, tautomères et stéréoisomères pharmaceutiquement utilisables de ceux-ci, y compris des mélanges de ceux-ci en toutes proportions.

8. Composés selon l'une ou plusieurs parmi les revendications 1-7, dans lesquels
Hét³ désigne furyle, thiényle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle, benzimidazolyle, benzotriazolyle, quinoléinyle, quinoxalinyle, quinazolinyle, pyrrolopyridinyle, purinyle, indolyle ou indazolyle, chacun d'entre eux étant non substitué ou mono- ou disubstitué par A et/ou NH₂,
et les sels, tautomères et stéréoisomères pharmaceutiquement utilisables de ceux-ci, y compris des mélanges de ceux-ci en toutes proportions.

9. Composés selon l'une ou plusieurs parmi les revendications 1-8, dans lesquels
Hét⁴ désigne pipéridinyle, pyrrolidinyle, morpholinyle, pipérazinyle, imidazolidinyle, oxazolidinyle, tétrahydropyranyle, dihydroisoindolyle, furyle, thiényle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle, benzimidazolyle, benzotriazolyle, quinoléinyle, quinoxalinyle, quinazolinyle, pyrrolopyridinyle, purinyle, indolyle ou indazolyle, chacun d'entre eux étant non substitué ou mono- ou disubstitué par NH₂, NHCH₂Ar¹ et/ou =O,
et les sels, tautomères et stéréoisomères pharmaceutiquement utilisables de ceux-ci, y compris des mélanges de ceux-ci en toutes proportions.

10. Composés selon la revendication 1, choisis dans le groupe constitué par
| n° | Nom et/ou structure |
|---|---|
| "A1" | 5-(4-Fluorophényl)-4*H*-pyrrolo[2,3-*d*]-1,3-thiazole |
| | 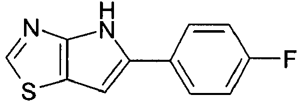 |
| "A2" | 5-Benzyl-4*H*-pyrrolo[2,3-*d*]thiazole |
| "A4" | 3-(4*H*-Pyrrolo[2,3-*d*]thiazol-5-yl)phénylamine |
| | 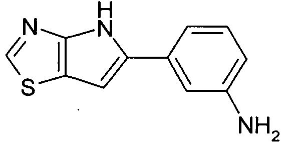 |
| "A5" | Benzyl-[4-(4*H*-pyrrolo[2,3-*d*]thiazol-5-yl)pyridin-2-yl]amine |
| | 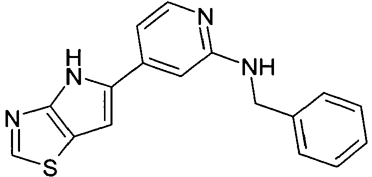 |
| "A6" | Benzyl-[3-(4*H*-pyrrolo[2,3-*d*]thiazol-5-yl)phényl]amine |
| | 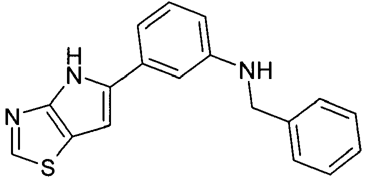 |
| "A7" | 6-Benzyl-4*H*-pyrrolo[2,3-*d*]thiazole |
| | 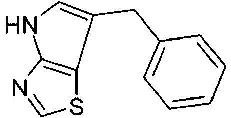 |
| "A8" | 6-Benzyl-2-pyridin-4-yl-4*H*-pyrrolo[2,3-*d*]thiazole |
| | 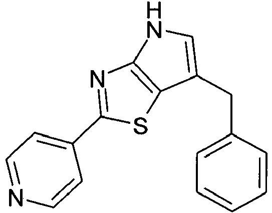 |
| "A9" | 4-(4*H*-Pyrrolo[2,3-*d*]thiazol-5-yl)pyridin-2-ylamine |
| | 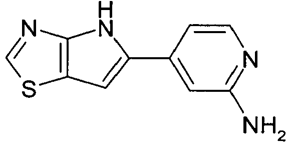 |
| "A10" | 2-Chloro-5-(4-fluorophényl)-4*H*-pyrrolo[2,3-*d*]thiazole |
| | 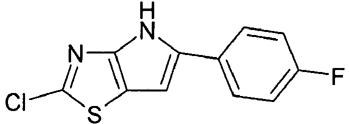 |
| "A11" | 5-(4-Fluorophényl)-2-(4-fluorophényléthynyl)-4*H-*pyrrolo[2,3-*d*]thiazole |
| | 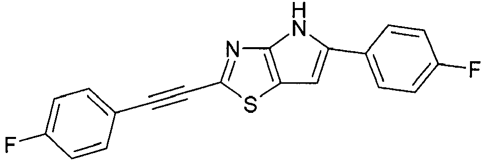 |
| "A12" | 5-(4-Fluorophényl)-2-morpholin-4-yl-4*H-*pyrrolo[2,3-*d*]thiazole |
| | 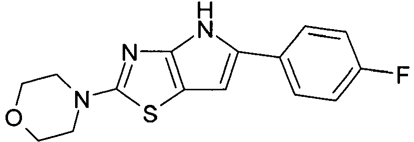 |
| "A13" | 5-(4-Fluorophényl)-2-(4-pyridin-4-ylméthylpipérazin-1-yl)-4*H*-pyrrolo[2,3-*d*]thiazole |
| | 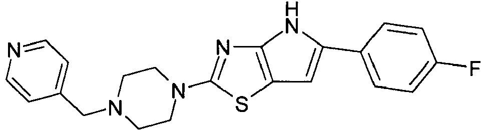 |
| "A14" | 5-[5-(4-Fluorophényl)-4*H*-pyrrolo[2,3-*d*]thiazol-2-yl]-pyrimidin-2-ylamine |
| | 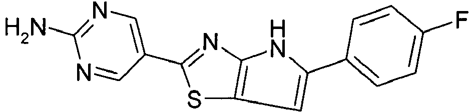 |
| "A30" | 3-[5-(4-Fluorophényl)-4*H*-pyrrolo[2,3-*d*]thiazol-2-yl]-pyridin-2-ylamine |
| | 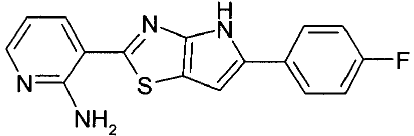 |
| "A31" | 3-[5-(4-Fluorophényl)-4H-pyrrolo[2,3-d]thiazol-2-yl]-5-(1-pipéridin-4-yl-1H-pyrazol-4-yl)pyridin-2-ylamine |
| "A32" | 6-(4,4-Diméthylpent-2-ynyl)-4H-pyrrolo[2,3-d]thiazole |
| "A33" | 2-tertio-Butyl-3-(4H-pyrrolo[2,3-d]thiazol-6-ylméthyl)-1H-indole-5-carboxylate de méthyle |
| "A34" | 2-tertio-butyl-3-(4H-pyrrolo[2,3-d]thiazol-6-ylméthyl)-1H-pyrrolo[2,3-b]pyridine |
| | 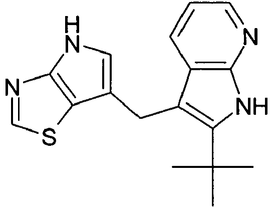 |
| "A35" | 6-(3,5-Difluorobenzyl)-4H-pyrrolo[2,3-d]thiazole |
| "A36" | 6-(4-Méthoxybenzyl)-4H-pyrrolo[2,3-d]thiazole |
| "A37" | 6-(4-Bromobenzyl)-4H-pyrrolo[2,3-d]thiazole |
| "A38" | 6-(4-Isopropylbenzyl)-4H-pyrrolo[2,3-d]thiazole |
| "A39" | 6-(4-Bromo-2-fluorobenzyl)-4H-pyrrolo[2,3-d]thiazole |
| "A40" | 6-(4-tertio-Butylbenzyl)-4H-pyrrolo[2,3-d]thiazole |
| "A41" | 6-(2-Chlorobenzyl)-4H-pyrrolo[2,3-d]thiazole |
| "A42" | 6-(4-Fluorobenzyl)-4H-pyrrolo[2,3-d]thiazole |
| "A43" | 6-(3-Fluorobenzyl)-4H-pyrrolo[2,3-d]thiazole |
| "A44" | 6-(2,3-Difluorobenzyl)-4H-pyrrolo[2,3-d]thiazole |
| "A45" | 6-(2,4-Difluorobenzyl)-4H-pyrrolo[2,3-d]thiazole |
| "A46" | 6-(2,5-Difluorobenzyl)-4H-pyrrolo[2,3-d]thiazole |
| "A47" | 6-(3,4-Difluorobenzyl)-4H-pyrrolo[2,3-d]thiazole |
| "A48" | 6-(6-Chloropyridin-3-ylméthyl)-4H-pyrrolo[2,3-d]thiazole |
| "A49" | 6-(3-Chlorobenzyl)-4H-pyrrolo[2,3-d]thiazole |
| "A50" | 6-(3,4-Dichlorobenzyl)-4H-pyrrolo[2,3-d]thiazole |
| "A51" | 6-Benzyl-2-pyridin-3-yl-4H-pyrrolo[2,3-d]thiazole |
| "A52" | 6-Benzyl-2-(1-méthyl-1H-pyrazol-4-yl)-4H-pyrrolo-[2,3-d]thiazole |
| "A53" | 5-(6-Benzyl-4H-pyrrolo[2,3-d]thiazol-2-yl)pyridin-2-ylamine |
| "A54" | 6-Prop-2-ynyl-4H-pyrrolo[2,3-d]thiazole |
| "A55" | 3-(6-Benzyl-4H-pyrrolo[2,3-d]thiazol-2-yl)pyridin-2-ylamine |
| "A56" | 6-Benzyl-2-(1H-pyrazol-4-yl)-4H-pyrrolo[2,3-d]thiazole |
| "A57" | 6-Benzyl-2-(5-bromopyridin-3-yl)-4H-pyrrolo[2,3-d]thiazole |
| "A58" | 6-Benzyl-2-pyrimidin-5-yl-4H-pyrrolo[2,3-d]thiazole |
| "A59" | Morpholin-4-yl-[3-(4H-pyrrolo[2,3-d]thiazol-6-ylméthyl)-1H-indol-5-yl]méthanone |
| "A60" | N-(2-Méthoxyéthyl)-3-(4H-pyrrolo[2,3-d]thiazol-6-ylméthyl)-1H-indole-5-carboxamide |
| "A61" | N-(4-Fluorobenzyl)-3-(4H-pyrrolo[2,3-d]thiazol-6-ylméthyl)-1H-indole-5-carboxamide |
| "A62" | N-(2-Aminoéthyl)-2-[4-(4H-pyrrolo[2,3-d]thiazol-6-ylméthyl)-1,2,3-triazol-1-yl]acétamide |
| "A63" | 1-Pipérazin-1-yl-2-[4-(4H-pyrrolo[2,3-d]thiazol-6-ylméthyl)-1,2,3-triazol-1-yl]éthanone |
| "A64" | N-(Pipéridin-4-yl)-2-[4-(4H-pyrrolo[2,3-d]thiazol-6-ylméthyl)-1,2,3-triazol-1-yl]acétamide |
| "A65" | 6-(1-Pyridin-3-ylméthyl-1H-1,2,3-triazol-4-ylméthyl)-4H-pyrrolo[2,3-d]thiazole |
| "A66" | 6-(1-Pyridin-4-ylméthyl-1H-1,2,3-triazol-4-ylméthyl)-4H-pyrrolo[2,3-d]thiazole |
| "A67" | 6-Benzyl-2-(5-(1-méthyl-1H-pyrazol-4-yl)pyridin-3-yl)-4H-pyrrolo[2,3-d]thiazole |
| "A68" | 6-Benzyl-2-(5-(1H-pyrazol-4-yl)pyridin-3-yl)-4H-pyrrolo[2,3-d]thiazole |
| "A69" | 6-Benzyl-2-(5-(4-fluorophényl)pyridin-3-yl)-4H-pyrrolo[2,3-d]thiazole |
| "A70" | 6-(5-Méthanesulfonylthiophén-2-ylméthyl)-4H-pyrrolo[2,3-d]thiazole |
| "A71" | 6-[1-(2-Morpholin-4-yléthyl)-1H-1,2,3-triazol-4-ylméthyl]-4H-pyrrolo[2,3-d]thiazole |
| "A72" | 6-Benzyl-2-{5-[1-((E)-3-phénylallyl)-1H-pyrazol-4-yl]pyridin-3-yl}-4H-pyrrolo[2,3-d]thiazole |
et les sels, tautomères et stéréoisomères pharmaceutiquement utilisables de ceux-ci, y compris des mélanges de ceux-ci en toutes proportions.

11. Procédé de préparation de composés de formule la selon les revendications 1-10 et de sels, tautomères et stéréoisomères pharmaceutiquement utilisables de ceux-ci, **caractérisé en ce que**
a) pour la préparation d'un composé de formule la', 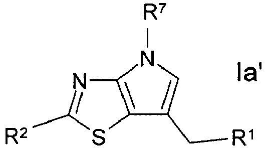 dans laquelle
R¹ désigne Ar ou Hét,
R² revêt les significations indiquées selon la revendication 1, excepté Hal,
R⁷ désigne tertio-butyloxycarbonyle,
et Ar et Hét revêtent les significations indiquées selon la revendication 1,
un composé de formule IIc 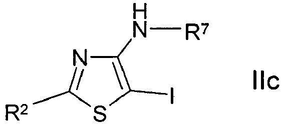 dans laquelle
R² revêt la signification indiquée selon la revendication 1, excepté Hal,
R⁷ désigne tertio-butyloxycarbonyle,
est réagi avec un composé de formule IIIc
R¹-CH=CH-CH₂Br IIIc
dans laquelle R¹ désigne Ar ou Hét,
et Ar et Hét revêtent les significations indiquées selon la revendication 1,
et ensuite ou simultanément le groupement tertio-butyloxycarbonyle est éliminé par clivage,
ou
b) pour la préparation d'un composé de formule la" 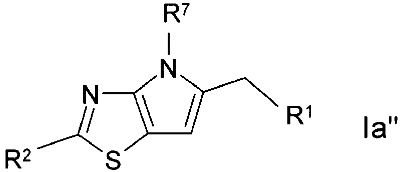 dans laquelle
R¹ désigne Ar ou Hét,
R² désigne H, Cl, (CH₂)ₙHét³,
R⁷ désigne H ,
et n, Ar, Hét et Hét³ revêtent les significations indiquées selon la revendication 1,
un composé de formule Ib' 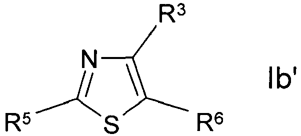 dans laquelle
R³ désigne NH-COO-tertio-butyle,
R⁵ désigne H, Cl, (CH₂)ₙHét³,
R⁶ désigne -C≡C-R⁴,
R⁴ désigne Ar ou Hét,
et Ar, Hét, Hét³ et R⁴ revêtent les significations indiquées selon la revendication 1,
est chauffé en présence d'une base,
ou
c) pour la préparation d'un composé de formule la" 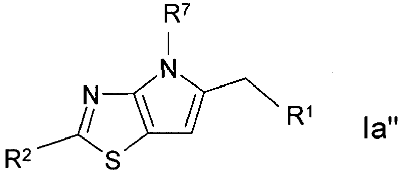 dans laquelle
R¹ désigne Ar ou Hét,
R² désigne Hét¹,
R⁷ désigne H ,
un composé de formule la" 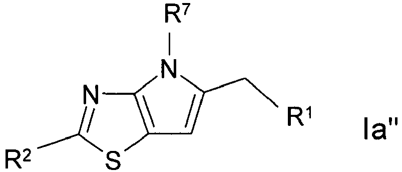 dans laquelle
R¹ désigne Ar ou Hét,
R² désigne Hal,
R⁷ désigne H,
Ar et Hét revêtent les significations indiquées selon la revendication 1,
est réagi avec un composé de formule Va
Hét¹-H Va
dans laquelle Hét¹ revêt la signification indiquée selon la revendication 1,
et/ou
une base ou un acide de formule la est converti(e) en l'un de ses sels.

12. Médicaments comprenant au moins un composé de formule la selon les revendications 1-10 et/ou des sels, tautomères et stéréoisomères pharmaceutiquement utilisables de celui-ci, y compris des mélanges de ceux-ci en toutes proportions, et éventuellement des excipients et/ou des adjuvants.

13. Composés selon les revendications 1-10 et les sels, tautomères et stéréoisomères pharmaceutiquement utilisables de ceux-ci, y compris des mélanges de ceux-ci en toutes proportions, pour une utilisation dans la préparation d'un médicament destiné au traitement de tumeurs, du cancer, de la formation, de la croissance et de la propagation de tumeurs, de l'artériosclérose, de maladies oculaires, telles que la dégénérescence maculaire liée à l'âge, de la néovascularisation choroïdienne et de la rétinopathie diabétique, des maladies inflammatoires, de l'arthrite, de la thrombose, de la fibrose, de la glomérulonéphrite, de la neurodégénérescence, du psoriasis, de la resténose, de la guérison de plaies, du rejet de greffe, des maladies métaboliques et des maladies du système immunitaire, des maladies auto-immunes, de la cirrhose, du diabète et des maladies des vaisseaux sanguins.

14. Composés choisis dans le groupe constitué par
| N° | Nom et/ou structure |
|---|---|
| "2" | *N*-(5-iodo-1,3-thiazol-4-yl)carbamate de *tertio-bu*tyle |
| "A15" | *N*-{5-[2-(4-fluorophényl)éthynyl]-1,3-thiazol-4-yl}-carbamate de *tertio-bu*tyle |
| | 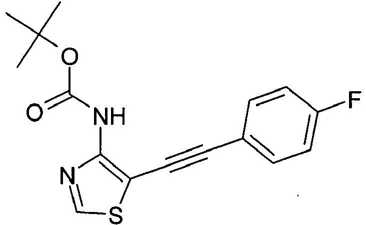 |
| "A16" | [5-(3-phénylprop-1-ynyl)thiazol-4-yl]carbamate de *tertio-bu*tyle |
| | 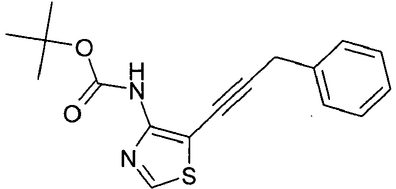 |
| "A17" | (5-triéthylsilanyléthynylthiazol-4-yl)carbamate de *tertio-*butyle |
| | 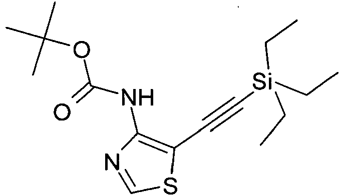 |
| "A18" | [5-(3-aminophényléthynyl)thiazol-4-yl]carbamate de *tertio-bu*tyle |
| | 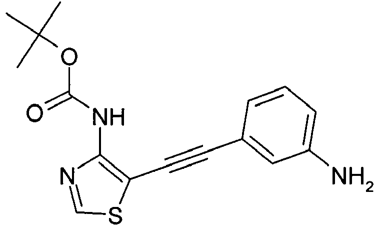 |
| "A19" | [5-(3-benzylaminophényléthynyl)thiazol-4-yl]carbamate de *tertio-bu*tyle |
| | 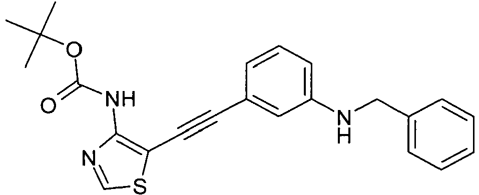 |
| "A20" | [5-(2-benzylaminopyridin-4-yléthynyl)thiazol-4-yl]-carbamate de *tertio-bu*tyle |
| | 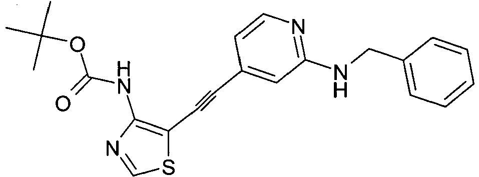 |
| "A21" | [5-(1H-pyrrolo[2,3-b]pyridin-5-yléthynyl)thiazol-4-yl]carbamate de *tertio-bu*tyle |
| | 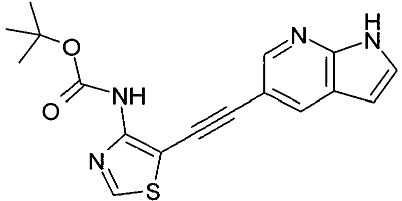 |
| "A22" | 5-(1H-Pyrrolo[2,3-b]pyridin-5-yléthynyl)thiazol-4-ylamine |
| | 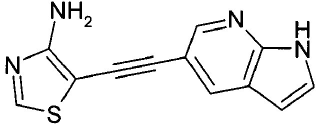 |
| "A24" | *N*-(2-pyridin-4-yl-5-iodo-1,3-thiazol-4-yl)carbamate de *tertio-bu*tyle |
| | 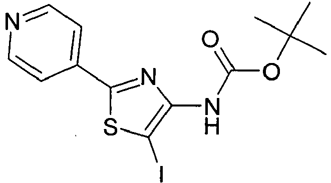 |
| "A25" | [5-(3-morpholin-4-ylprop-1-ynyl)-2-pyridin-4-ylthiazol-4-yl]carbamate de *tertio-bu*tyle |
| | 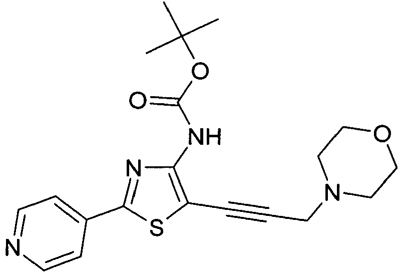 |
| "A26" | {5-[3-(1,3-dioxo-1,3-dihydroisoindol-2-yl)prop-1-ynyl]-thiazol-4-yl}carbamate de *tertio-bu*tyle |
| | 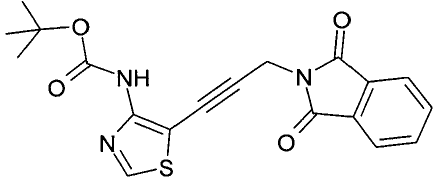 |
| "A27" | 3-(4-Aminothiazol-5-yléthynyl)phénol |
| | 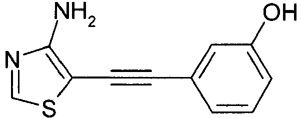 |
| "A28" | [2-(2-aminopyridin-3-yl)-5-(4-fluorophényléthynyl)thiazol-4-yl]carbamate de *tertio-bu*tyle |
| | 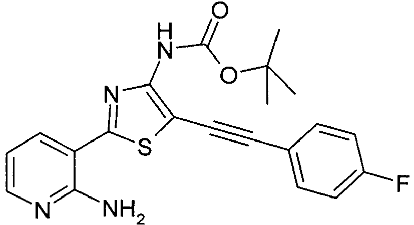 |
| "A29a" | *N*-{2-chloro-5-[2-(4-fluorophényl)éthynyl]-1,3-thiazol-4-yl}-carbamate de *tertio-bu*tyle |
| | 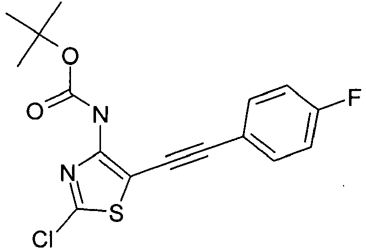 |
| "A29" | *N*-[2-(2-aminopyrimidin-5-yl)-5-[2-(4-fluorophényl)éthynyl]-1,3-thiazol-4-yl]carbamate de *tertio-bu*tyle |
| | 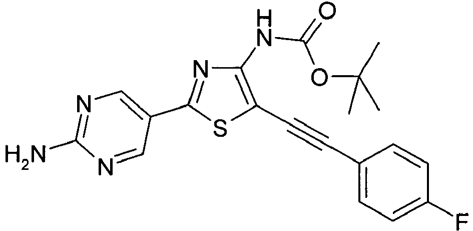 |
et les sels, tautomères et stéréoisomères pharmaceutiquement utilisables de ceux-ci, y compris des mélanges de ceux-ci en toutes proportions.

15. Médicaments comprenant au moins un composé selon la revendication 14 et/ou des sels, tautomères et stéréoisomères pharmaceutiquement utilisables de celui-ci, y compris des mélanges de ceux-ci en toutes proportions, et éventuellement des excipients et/ou des adjuvants.
